# EUROPEAN PATENT SPECIFICATION

(11) **EP 0 600 018 B1**
(45) Date of publication and mention of the grant of the patent: **11.07.2001**
(21) Application number: 92919024.7
(22) Date of filing: 21.08.1992
(51) Int. Cl.: C12N 15/62, C07K 16/08, C12Q 1/70

(54) **HEPATITIS C ASSAY UTILIZING RECOMBINANT ANTIGENS TO C-100 REGION**
HEPATITIS C-NACHWEISTEST UNTER BENUTZUNG REKOMBINANTER ANTIGENE DER C-100-REGION
DEPISTAGE DE L'HEPATITE C A L'AIDE D'ANTIGENES RECOMBINES DIRIGES CONTRE LA REGION C-100

(30) Priority: 21.08.1991 US 748566
(43) Date of publication of application: 08.06.1994
(73) Proprietor: ABBOTT LABORATORIES, Abbott Park, Illinois 60064-3500 (US)
(72) Inventor: DESAI, Suresh, M., Libertyville, IL 60048 (US); CASEY, James, M., Waukegan, IL 60085 (US); RUPPRECHT, Kevin, R., Grayslake, IL 60030 (US); DEVARE, Sushil, G., Northbrook, IL 60062 (US)
(74) Representative: Modiano, Guido, Dr.-Ing.
(86) International application number: US9207187
(87) International publication number: WO9304087

(56) References cited:
- EP-A- 0 388 232
- WO-A-90/11089
- US-A- 4 808 536
- US-A- 4 925 784
- Biotechniques, Volume 8, Number 5, issued 1990, BOLLING et al., "An Escherichia coli expression vector for high-level production of heterologous proteins in fusion with CMP-KDO synthetase", pages 488-492, see entire document.

## Description

This invention relates generally to an assay for identifying the presence in a sample of an antibody which is immunologically reactive with a hepatitis C virus antigen and specifically to an assay for detecting a complex of an antibody and recombinant antigens representing distinct regions of the HCV genome. Recombinant antigens derived from the molecular cloning and expression in a heterologous expression system of the synthetic DNA sequences representing distinct antigenic regions of the HCV genome can be used as reagents for the detection of antibodies and antigen in body fluids from individuals exposed to hepatitis C virus (HCV).

### BACKGROUND OF THE INVENTION

Acute viral hepatitis is clinically diagnosed by a well-defined set of patient symptoms, including jaundice, hepatic tenderness, and an increase in the serum levels of alanine aminotransferase (ALT) and aspartate aminotransferase. Additional serologic immunoassays are generally performed to diagnose the specific type of viral causative agent. Historically, patients presenting clinical hepatitis symptoms and not otherwise infected by hepatitis A, hepatitis B, Epstein-Barr or cytomegalovirus were clinically diagnosed as having non-A non-B hepatitis (NANBH) by default. The disease may result in chronic liver damage.

Each of the well-known, immunologically characterized hepatitis-inducing viruses, hepatitis A virus (HAV), hepatitis B virus (HBV), and hepatitis D virus (HDV) belongs to a separate family of viruses and has a distinctive viral organization, protein structure, and mode of replication.

Attempts to identify the NANBH virus by virtue of genomic similarity to one of the known hepatitis viruses have failed, suggesting that NANBH has a distinct organization and structure. [Fowler, et al., J. Med. Virol., 12:205-213 (1983) and Weiner, et al., J. Med. Virol., 21:239-247 (1987)].

Progress in developing assays to detect antibodies specific for NANBH has been particularly hampered by difficulties in correctly identifying antigens associated with NANBH. See, for example, Wands, J., et al., U.S. Patent 4,870,076, Wands, et al., Proc. Nat'l, Acad. Sci., 83:6608-6612 (1986), Ohori, et al., J. Med. Virol., 12:161-178 (1983), Bradley, et al., Proc. Nat'l. Acad. Sci., 84:6277-6281, (1987), Akatsuka, T., et al., J. Med. Virol, 20:43-56 (1986), Seto, B., et al., U.S. Patent Application Number 07/234,641 (available from U.S. Department of Commerce National Technical Information Service, Springfield, Virginia, No. 89138168), Takahashi, K., et al., European Patent Application publication No. 0 293 274, published November 30, 1988, and Seelig, R., et al., in PCT Application PCT/EP88/00123.

Recently, another hepatitis-inducing virus has been unequivocally identified as hepatitis C virus (HCV) by Houghton, M., et al., European Patent Application publication number 0 318 216, May 31, 1989. Related papers describing this virus include Kuo, G., et al., Science, 244:359-361 (1989) and Choo, Q., et. al, Science, 244:362-364 (1989). Houghton, M., et al. reported isolating cDNA sequences from HCV which encode antigens which react immunologically with antibodies present in patients infected with NANBH, thus establishing that HCV is one of the viral agents causing NANBH. The cDNA sequences associated with HCV were isolated from a cDNA library prepared from the RNA obtained from pooled serum from a chimpanzee with chronic HCV infection. The cDNA library contained cDNA sequences of approximate mean size of about 200 base pairs. The cDNA library was screened for encoded epitopes expressed in clones that could bind to antibodies in sera from patients who had previously experienced NANBH.

In the European Patent Application, Houghton, M., et al. also described the preparation of several superoxide dismutase fusion polypeptides (SOD) and the use of these SOD fusion polypeptides to develop an HCV screening assay. The most complex SOD fusion polypeptide described in the European Patent Application, designated c100-3, was described as containing 154 amino acids of human SOD at the aminoterminus, 5 amino acid residues derived from the expression of a synthetic DNA adapter containing a restriction site, EcoRl, 363 amino acids derived from the expression of a cloned HCV cDNA fragment, and 5 carboxyl terminal amino acids derived from an MS2 cloning vector nucleotide sequence. The DNA sequence encoding this polypeptide was transformed into yeast cells using a plasmid. The transformed cells were cultured and expressed a 54,000 molecular weight polypeptide which was purified to about 80% purity by differential extraction.

Other SOD fusion polypeptides designated SOD-NANB₅₋₁₋₁ and SOD-NANB₈₁ were expressed in recombinant bacteria. The E. coli fusion polypeptides were purified by differential extraction and by chromatography using anion and cation exchange columns. The purification procedures were able to produce SOD-NANB₅₋₁₋₁ as about 80% pure and SOD-NAN38, as about 50% pure.

The recombinant SOD fusion polypeptides described by Houghton, M., et al. were coated on microtiter wells or polystyrene beads and used to assay serum samples. Briefly, coated microtiter wells were incubated with a sample in a diluent. After incubation, the microtiter wells were washed and then developed using either a radioactively labelled sheep anti-human antibody or a mouse antihuman IgG-HRP (horseradish peroxidase) conjugate. These assays were used to detect both post acute phase and chronic phase HCV infection.

Due to the preparative methods, assay specificity required adding yeast or E. coli extracts to the samples in order to prevent undesired immunological reactions with any yeast or E. coli antibodies present in samples.

In European Patent Application publication No. 0 388 232, Houghton, M. et al. disclose further HCV cDNA sequences and polypeptides. Also disclosed is the application of these sequences and polypeptides in immunoassays, probe diagnostics, anti-HCV antibody production, PCR technology and recombinant DNA technology.

Ortho Diagnostic Systems Inc. have developed a immunoenzyme assay to detect antibodies to HCV antigens. The Ortho assay procedure is a three-stage test for serum/plasma carried out in a microwell coated with the recombinant yeast/hepatitis C virus SOD fusion polypeptide c100-3.

In the first stage, a test specimen is diluted directly in the test well and incubated for a specified length of time. If antibodies to HCV antigens are present in the specimen, antigen-antibody complexes will be formed on the microwell surface. If no antibodies are present, complexes will not be formed and the unbound serum or plasma proteins will be removed in a washing step.

In the second stage, anti-human lgG murine monoclonal antibody horseradish peroxidase conjugate is added to the microwell. The conjugate binds specifically to the antibody portion of the antigen-antibody complexes. If antigen-antibody complexes are not present, the unbound conjugate will also be removed by a washing step.

In the third stage, an enzyme detection system composed of o-phenylenediamine 2HCl (OPD) and hydrogen peroxide is added to the test well. If bound conjugate is present, the OPD will be oxidized, resulting in a colored end product. After formation of the colored end product, dilute sulfuric acid is added to the microwell to stop the color-forming detection reaction.

The intensity of the colored end product is measured with a microwell reader. The assay may be used to screen patient serum and plasma.

It is established that HCV may be transmitted by contaminated blood and blood products. In transfused patients, as many as 10% will suffer from posttransfusion hepatitis. Of these, approximately 90% are the result of infections diagnosed as HCV. The prevention of transmission of HCV by blood and blood requires reliable, sensitive and specific diagnosis and prognostic tools to identify HCV carriers as well as contaminated blood and blood products. Thus, there exists a need for an HCV assay which uses reliable and efficient reagents and methods to accurately detect the presence of HCV antibodies in samples.

### SUMMARY OF THE INVENTION

The present invention provides recombinant fusion proteins and assays for detecting the presence of an antibody to an HCV antigen in a sample by contacting the sample with at least one of said recombinant proteins representing a distinct antigenic region of the HCV genome.

Recombinant antigens which are derived from the molecular cloning and expression of synthetic DNA sequences in heterologous hosts are provided. Briefly, synthetic DNA sequences which encode the desired proteins representing distinct antigenic regions of the HCV genome are optimized for expression in E. coli by specific codon selection. Specifically, recombinant proteins representing antigenic regions of the HCV genome, are described. The proteins are expressed as chimeric fusions with E. coli CMP-KDO synthetase (CKS) gene.

The first protein is expressed by plasmid pHCV-63 and is identified by SEQ. ID. NO. 12. The fusion protein itself will also be referred to as pHCV-63 and pHCV-63' shall be the designation for the polypeptide containing an HCV sequence corresponding to the HCV sequence contained in the fusion protein identified by SEQ. ID. NO. 12, said HCV sequence in said polypeptide not forming part of a longer HCV sequence, said polypeptide being prepared using other recombinant methodologies other than through expression as fusions with CKS, which would include the preparation of pHCV-63', utilizing different expression systems. The next protein is expressed by plasmid pHCV-204 and is identified by SEQ. ID. NO. 13. The fusion protein itself will also be referred to as pHCV-204 and pHCV-204' shall be the designation for the polypeptide containing an HCV sequence corresponding to the HCV sequence contained in the fusion protein identified by SEQ. ID. NO. 13, said HCV sequence in said polypeptide not forming part of a longer HCV sequence, said polypeptide being prepared using other recombinant methodologies other than through expression as fusions with CKS. The next protein is expressed by plasmid pHCV-112 and is identified by SEQ. ID. NO. 14. The fusion protein itself will also be referred to as pHCV-112 and pHCV-112' shall be the designation for the polypeptide containing an HCV sequence corresponding to the HCV sequence contained in the fusion protein identified by SEQ. ID. NO. 14, said HCV sequence in said polypeptide not forming part of a longer HCV sequence, said polypeptide being prepared using other recombinant methodologies other than through expression as fusions with CKS. The next protein is expressed by plasmid pHCV-73 and is identified by SEQ. ID. NO. 17. The fusion protein itself will also be referred to as pHCV-73 and pHCV-73' shall be the designation for the polypeptide containing an HCV sequence corresponding to the HCV sequence contained in the fusion protein identified by SEQ: ID. NO. 17, said HCV sequence in said polypeptide not forming part of a longer HCV sequence, said polypeptide being prepared using the other recombinant methodologies other than through expression as fusions with CKS. The next protein is expressed by plasmid pHCV-205 and is identified by SEQ. ID. NO. 18. The fusion protein itself will also be referred to as pHCV-205 and pHCV-205' shall be the designation for the polypeptide containing an HCV sequence corresponding to the HCV sequence contained in the fusion protein identified by SEQ. ID. NO. 18, said HCV sequence in said polypeptide not forming part of a longer HCV sequence, said polypeptide being prepared using other recombinant methodologies other than through expression as fusions with CKS. These antigens are used in the inventive immunoassays to detect the presence of HCV antibodies in samples.

One assay format according to the invention provides a screening assay for identifying the presence of an antibody that is immunologically reactive with an HCV antigen. Briefly, a fluid sample is incubated with a solid support containing the two commonly bound recombinant proteins selected from the group consisting of pHCV-63, pHCV-204, pHCV-112, pHCV-73, pHCV-205, pHCV-63', pHCV-204', pHCV-112', pHCV-73' and pHCV-205'. Finally, the antibody-antigen complex is detected.

Another assay format provides a confirmatory assay for unequivocally identifying the- presence of an antibody that is immunologically reactive with an HCV antigen. The confirmatory assay includes recombinant antigens representing an epitope contained within the C100 region of the HCV genome, which are the same regions represented by the recombinant proteins described in the screening assay. Recombinant proteins used in the confirmatory assay should have a heterologous source of antigen to that used in the primary screening assay (i.e. should not be an E. coli-derived recombinant antigen or a recombinant antigen composed in part, of CKS sequences). Briefly, specimens repeatedly reactive in the primary screening assay are retested in the confirmatory assay. Aliquots containing identical amounts of specimen are contacted with a recombinant antigen individually coated onto a solid support. Finally, the antibody-antigen complex is detected.

Another assay format provides a competition assay or neutralization assay directed to the confirmation that positive results are not false by identifying the presence of an antibody that is immunologically reactive with an HCV antigen in a fluid sample where the sample is used to prepare first and second immunologically equivalent aliquois. The first aliquot is contacted with solid support containing a bound polypeptide which contains at least one epitope of an HCV antigen under conditions suitable for complexing with the antibody to form a detectable antibody-polypeptide complex and the second aliquot is first contacted with the same solid support containing bound polypeptide.

Another assay format provides an immunodot assay for identifying the presence of an antibody that is immunologically reactive with an HCV antigen by concurrently contacting a sample with recombinant polypeptides each containing distinct epitopes of an HCV antigen under conditions suitable for complexing the antibody with at least one of the polypeptides and detecting the antibody-polypeptide complex by reacting the complex with color producing reagents. The preferred recombinant polypeptides employed include those recombinant polypeptides derived from pHCV-63, pHCV-73, pHCV-112, pHCV-204 and pHCV-205.

A preferred immunodot assay system uses a panel of purified recombinant polypeptides placed in an array on a nitrocellulose solid support. The prepared solid support is contacted with a sample and captures specific antibodies to HCV antigens. The captured antibodies are detected by a conjugate-specific reaction. Preferably, the conjugate specific reaction is quantified using a reflectance optics assembly within an instrument which has been described in EP-A-0 353 591. The related EP-A-0 353 589, EP-A-0 353 592 and U.S. Patent No. 5,075,077 further describe specific methods and apparatus useful to perform an immunodot assay. The assay has also been described in EP-A-0 461 462.
Briefly, a nitrocellulose-base test cartridge is treated with multiple antigenic polypeptides. Each polypeptide is contained within a specific reaction zone on the test cartridge. After all the antigenic polypeptides have been placed on the nitrocellulose, excess binding sites on the nitrocellulose are blocked. The test cartridge is then contacted with a sample such that each antigenic polypeptide in each reaction zone will react if the sample contains the appropriate antibody. After reaction, the test cartridge is washed and any antigen-antibody reactions are identified using suitable well known reagents.

As described in the patent applications listed above, the entire process is amenable to automation.

In all of the assays, the sample is preferably diluted before contacting the polypeptide absorbed on a solid support. Samples may be obtained from different biological samples such as whole blood, serum, plasma, cerebral spinal fluid, and lymphocyte or cell culture supernatants. Solid support materials may include cellulose materials, such as paper and nitrocellulose, natural and synthetic polymeric materials, such as polyacrylamide, polystyrene, and cotton, porous gels such as silica gel, agarose, dextran and gelatin, and inorganic materials such as deactivated alumina, magnesium sulfate and glass. Suitable solid support materials may be used in assays in a variety of well known physical configurations, including microtiter wells, test tubes, beads, strips, membranes, and microparticles. A preferred solid support for a non-immunodot assay is a polystyrene bead. A preferred solid support for an immunodot assay is nitrocellulose.

Suitable methods and reagents for detecting an antibody-antigen complex in an assay of the present invention are commercially available or known in the relevant art. Representative methods may employ detection reagents such as enzymatic, radioisotopic, fluorescent, luminescent, or chemiluminescent reagents. These reagents may be used to prepare hapten-labelled anti-hapten detection systems according to known procedures, for example, a biotin-labelled anti-biotin system may be used to detect an antibody-antigen complex.

The present invention also encompasses assay kits including the above-described polypeptides which contain at least one epitope of an HCV antigen bound to a solid support as well as needed sample preparation reagents, wash reagents, detection reagents and signal producing reagents.

Other aspects and advantages of the invention will be apparent to those skilled in the art upon consideration of the following detailed description which provides illustrations of the invention in its presently preferred embodiments.

E. coli strains containing plasmids useful for constructs of the invention have been deposited at the American Type Culture Collection, Rockville, Maryland on September 26, 1991 under deposit number ATCC 68687 (pHCV-63), ATCC 68684 (pHCV-73), ATCC 68694 (pHCV-204), ATCC 68693 (pHCV-205) and ATCC 68686 (pHCV-112).

Further E. coli strains containing plasmids to which reference is made hereinbelow have been deposited at the American Type Culture Collection, Rockville, Maryland on November 6, 1990 under the accession Nos. ATCC 68459 (pHCV-57) and ATCC 68469 (pHCV-58), on September 26, 1991 under deposit numbers ATCC 68692 (pHCV-62) and ATCC 68685 (pHCV-72) and on August 10, 1990 under the accession No. ATCC 68381 (pHCV-29).

### BRIEF DESCRIPTION OF THE DRAWINGS

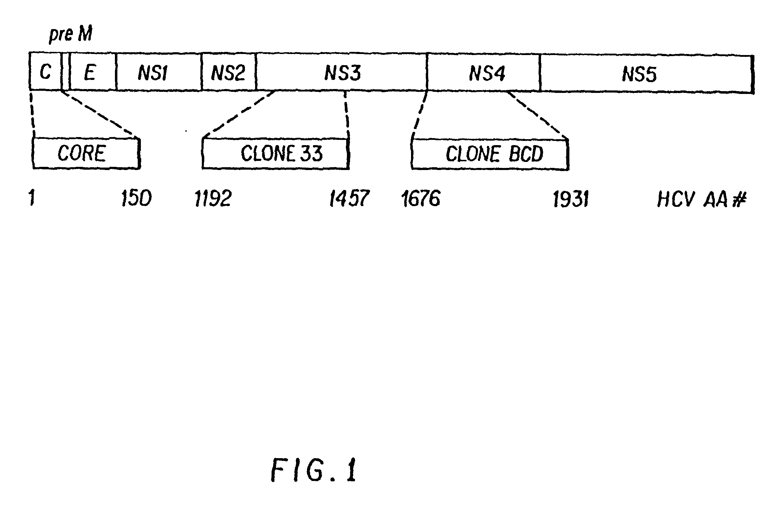

FIGURE 1 illustrates the HCV genome.

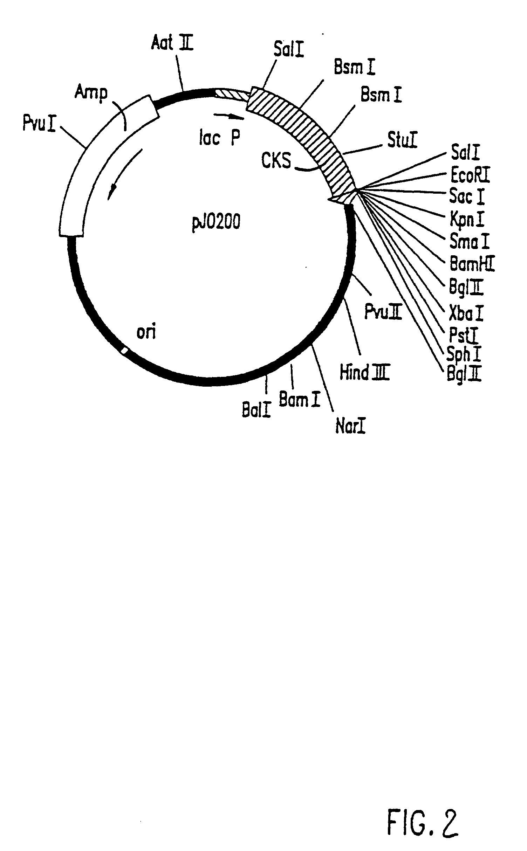

FIGURE 2 illustrates the plasmid pJ0200.
FIGURE 3 illustrates the fusion proteins pHCV-24, pHCV-57, pHCV-58.
FIGURE 4 illustrates the expression of pHCV-24, pHCV-57, and pHCV-58 in E. coli.
FIGURE 5A illustrates the expression of pHCV-19 (lane 1), pHCV-54 (lane 2), pHCV-55 (lane 3), pHCV-94 (lane 4), pHCV-95 (lane 6), pHCV-96 (lane 7) and pHCV-97 (lane 8) in E. coli, and
FIGURE 5B illustrates an immunoblot of pHCV-19 (lane 1), pHCV-54 (lane 2), pHCV-55 (lane 3), pHCV-94 (lane 4), pHCV-95 (lane 6), pHCV-96 (lane 7) and pHCV-97 (lane 8) in E. coli.
FIGURE 6A illustrates the expression of pHCV-202 (lanes 1, 2 and 3) and pHCV-203 (lanes 4, 5 and 6) in E. coli and
FIGURE 6B illustrates an immunoblot of pHCV-202 (lanes 1, 2 and 3) and pHCV-203 (lanes 4, 5 and 6) in E. coli.
FIGURE 7A illustrates the amino acid sequence of the recombinant antigen expressed by pHCV-62 (lanes 1 and 2) and pHCV-63 (lanes 3 and 4) and
FIGURE 7B illustrates an immunoblot of pHCV-62 (lanes 1 and 2) and pHCV-63 (lanes 3 and 4).
FIGURE 8A illustrates the expression of pHCV-204 in E. coli and
FIGURE 8B illustrates an immunoblot of pHCV-204 in E. coli.
FIGURE 9 illustrates the construction of plasmid pHCV-29.
FIGURE 10A illustrates the expression of pHCV-72 (lanes 1, 2 and 3) and pHCV-73 (lanes 4, 5 and 6) in E. coli and
FIGURE 10B illustrates an immunoblot of pHCV-72 (lanes 1, 2 and 3) and pHCV-73 (lanes 4, 5 and 6) in E. coli.
FIGURE 11A illustrates the expression of pHCV-205 in E. coli and
FIGURE 11B illustrates an immunoblot of pHCV-205 in E. coli.

### DETAILED DESCRIPTION OF THE INVENTION

The present invention is directed to recombinant fusion proteins and assays to detect an antibody to an HCV antigen in a sample. Human serum or plasma is preferably diluted in a sample diluent and incubated with a polystyrene bead coated with a recombinant polypeptide that represents a distinct antigenic region of the HCV genome. If antibodies are present in the sample they will form a complex with the antigenic polypeptide and become affixed to the polystyrene bead. After the complex has formed, unbound materials and reagents are removed by washing the bead and the bead-antigen-antibody complex is reacted with a solution containing horseradish peroxidase labeled goat antibodies directed against human antibodies. This peroxidase enzyme then binds to the antigen-antibody complex already fixed to the bead. In a final reaction the horseradish peroxidase is contacted with o-phenylenediamine and hydrogen peroxide which results in a yellow-orange color. The intensity of the color is proportional to the amount of antibody which initially binds to the antigen fixed to the bead.

The recombinant polypeptides having HCV antigenic epitopes were selected from portions of the HCV genome which encoded polypeptides which possessed amino acid sequences similar to other known immunologically reactive agents and which were identified as having some immunological reactivity. (The immunological reactivity of a polypeptide was initially identified by reacting the cellular extract of E. coli clones which had been transformed with cDNA fragments of the HCV genome with HCV infected serum. Polypeptides expressed by clone containing the incorporated cDNA were immunologically reactive with serum known to contain antibody to HCV antigens.) An analysis of a given amino acid sequence, however, only provides rough guides to predicting immunological reactivity. There is no invariably predictable way to ensure immunological activity short of preparing a given amino acid sequence and testing the suspected sequence in an assay.

The polypeptides useful in the practice of this invention are produced using recombinant technologies. The DNA sequences which encode the desired polypeptides are preferably assembled from fragments of the total desired sequence. Synthetic DNA fragments of the HCV genome can be synthesized based on their corresponding amino acid sequences. Once the amino acid sequence is chosen, this is then reverse translated to determine the complementary DNA sequence using codons optimized to facilitate expression in the chosen system. The fragments are generally prepared using well known automated processes and apparatus. After the complete sequence has been prepared the desired sequence is incorporated into an expression vector which is transformed into a host cell. The DNA sequence is then expressed by the host cell to give the desired polypeptide which is harvested from the host cell or from the medium in which the host cell is cultured. When smaller peptides are to be made using recombinant technologies it may be advantageous to prepare a single DNA sequence which encodes several copies of the desired polypeptide in a connected chain. The long chain is then isolated and the chain is cleaved into the shorter, desired sequences.

The methodology of polymerase chain reaction (PCR) may also be employed to develop PCR amplified genes from any portion of the HCV genome, which in turn may then be cloned and expressed in a manner similar to the synthetic genes.

Vector systems which can be used include plant, bacterial, yeast. insect, and mammalian expression systems. It is preferred that the codons are optimized for expression in the system used.

A preferred expression system utilizes a carrier gene for a fusion system where the recombinant HCV proteins are expressed as a fusion protein of an E. coli enzyme, CKS (CTP:CMP-3-deoxy-manno-octulosonate cytidylyl transferase or CMP-KDO synthetase). The CKS method of protein synthesis is disclosed by Bolling in European Patent Application No. 891029282 which enjoys common ownership.

Other expression systems may be utilized including the lambda PL vector system whose features include a strong lambda pL promoter, a strong three-frame translation terminator rrnBtl, and translation starting at an ATG codon.

In the present invention, the amino acid sequences encoding for the recombinant HCV antigens of interest were reverse translated using codons optimized to facilitate high level expression in E. coli. Individual oligonucleotides were synthesized by the method of oligonucleotide directed double-stranded break repair disclosed in by Mandecki in European Patent Application No. 87109357.1 which enjoys common ownership. Alternatively, the individual oligonucleotides may be synthesized on the Applied Biosystem 380A DNA synthesizer using methods and reagents recommended by the manufacturer. The DNA sequences of the individual oligonucleotides were confirmed using the Sanger dideoxy chain termination method (Sanger et al., J. Mole. Biol., 162:729 (1982)). These individual gene fragments were then annealed and ligated together and cloned as EcoRl-BamHl subfragments in the CKS fusion vector pJO200. After subsequent DNA sequence confirmation by the Sanger dideoxy chain termination method, the subfragments were digested with appropriate restriction enzymes, gel purified, ligated and cloned again as an EcoRl-BamHl fragment in the CKS fusion vector pJO200. The resulting clones were mapped to identify a hybrid gene consisting of the EcoRl-BamHl HCV fragment inserted at the 3' end of the CKS (CMP-KDO synthetase) gene. The resultant fusion proteins, under control of the lac promoter, consist of 239 amino acids of the CKS protein fused to the various regions of HCV.

The synthesis, cloning, and characterization of the recombinant polypeptides are provided in the following examples. Example 1 describes the construction of the plasmid pJ0200.

Example 2 describes the preparation of HCV CKS-C100 vectors. Example 3 describes the preparation of HCV PCR derived expression vectors. Example 4 describes the synthesis scheme, deletion analysis and production and characterization of recombinant antigen HCV pHCV-202, pHCV-204 and pHCV-205.

### REAGENTS AND ENZYMES

Media such as Luria-Bertani (LB) and Superbroth II (Dri Form) were obtained from Gibco Laboratories Life Technologies, Inc., Madison Wisconsin. Restriction enzymes, Klenow fragment of DNA polymerase I, T4 DNA ligase, T4 polynucleotide kinase, nucleic acid molecular weight standards, M13 sequencing system, X-gal (5-bromo-4-chloro-3-indonyl-β-D-galactoside), IPTG (isopropyl-β-D-thiogalactoside), glycerol, Dithiothreitol, 4-chloro-1-naphthol were purchased from Boehringer Mannheim Biochemicals, Indianapolis, Indiana; or New England Biolabs, Inc., Beverly, Massachusetts; or Bethesda Research Laboratories Life Technologies, Inc., Gaithersburg, Maryland. Prestained protein molecular weight standards, acrylamide (crystallized, electrophoretic grade >99%); N-N'-Methylene-bis-acrylamide (BIS); N,N,N',N',-Tetramethylethylenediamine (TEMED) and sodium dodecylsulfate (SDS) were purchased from BioRad Laboratories, Richmond, California. Lysozyme and ampicillin were obtained from Sigma Chemical Co., St. Louis, Missouri. Horseradish peroxidase (HRPO) labeled secondary antibodies were obtained from Kirkegaard & Perry Laboratories, Inc., Gaithersburg, Maryland. Seaplaque® agarose (low melting agarose) was purchased from FMC Bioproducts, Rockland, Maine.

T50E10 contained 50mM Tris, pH 8.0, 10mM EDTA; 1X TG contained 100mM Tris, pH 7.5 and 10% glycerol; 2X SDS/PAGE loading buffer consisted of 15% glycerol, 5% SDS, 100mM Tris base, 1M β-mercaptoethanol and 0.8% Bromophenol blue dye; TBS container 50 mM Tris, pH 8.0, and 150 mM sodium chloride; Blocking solution consisted of 5% Carnation nonfat dry milk in TBS.

### HOST CELL CULTURES. DNA SOURCES AND VECTORS

E. coli JM103 cells, pUC8, pUC18, pUC19 and M13 cloning vectors were purchased from Pharmacia LKB Biotechnology, Inc., Piscataway, New Jersey; Competent Epicurean™ coli stains XL1-Blue and JM109 were purchased from Stratagene Cloning Systems, LaJolla, California. RR1 cells were obtained from Coli Genetic Stock Center, Yale University, New Haven, Connecticut; and E. coli CAG456 cells from Dr. Carol Gross, University of Wisconsin, Madison, Wisconsin. Vector pRK248.clts was obtained from Dr. Donald R. Helinski, University of California, San Diego, California.

### GENERAL METHODS

All restriction enzyme digestion were performed according to suppliers' instructions. At least 5 units of enzyme were used per microgram of DNA, and sufficient incubation was allowed to complete digestion of DNA. Standard procedures were used for minicell lysate DNA preparation, phenol-chloroform extraction, ethanol precipitation of DNA, restriction analysis of DNA on agarose, and low melting agarose gel purification of DNA fragments (Maniatis et al., Molecular Cloning. A Laboratory Manual [New York: Cold Spring Harbor, 1982]). Plasmid isolations from E. coli strains used the alkali lysis procedure and cesium chloride-ethidium bromide density gradient method (Maniatis et al., supra). Standard buffers were used for T4 DNA ligase and T4 polynucleotide kinase (Maniatis et al., supra).

### EXAMPLE 1 Construction of the Plasmid pJ0200

The cloning vector pJO200 allows the fusion of recombinant proteins to the CKS protein. The plasmid consists of the plasmid pBR322 with a modified lac promoter fused to a KdsB gene fragment (encoding the first 239 of the entire 248 amino acids of the E. coli CMP-KDO synthetase of CKS protein), and a synthetic linker fused to the end of the KdsB gene fragment. The cloning vector pJO200 is a modification of vector pTB210. The synthetic linker includes: multiple restriction sites for insertion of genes; translational stop signals, and the trpA rho-independent transcriptional terminator. The CKS method of protein synthesis as well as CKS vectors including pTB210 are disclosed by Bolling in European Patent Application No. 891029282 which enjoys common ownership.

### EXAMPLE 2 HCV CKS-C100

### A. Preparation of HCV CKS-C100 Vectors

Eighteen individual oligonucleotides representing amino acids 1569-1931 of the HCV genome were ligated together and cloned as four separate EcoRl-BamHl subfragments into the CKS fusion vector pJ0200. After subsequent DNA sequences confirmation, the four subfragments were digested with the appropriate restriction enzymes, gel purified, ligated together, and cloned as an 1102 base pair EcoRl-BamHl fragment in the CKS fusion vector pJ0200. The resulting plasmid, designated pHCV-24, expresses the HCV CKS-C100 antigen under control of the lac promoter. The HCV CKS-c100 antigen consists of 239 amino acids of CKS, eight amino acids contributed by linker DNA sequences, 363 amino acids from the HCV NS4 region (amino acids 1569-1931) and 10 additional amino acids contributed by linker DNA sequences. The HCV CKS-c100 antigen was expressed at very low levels by pHCV-24.

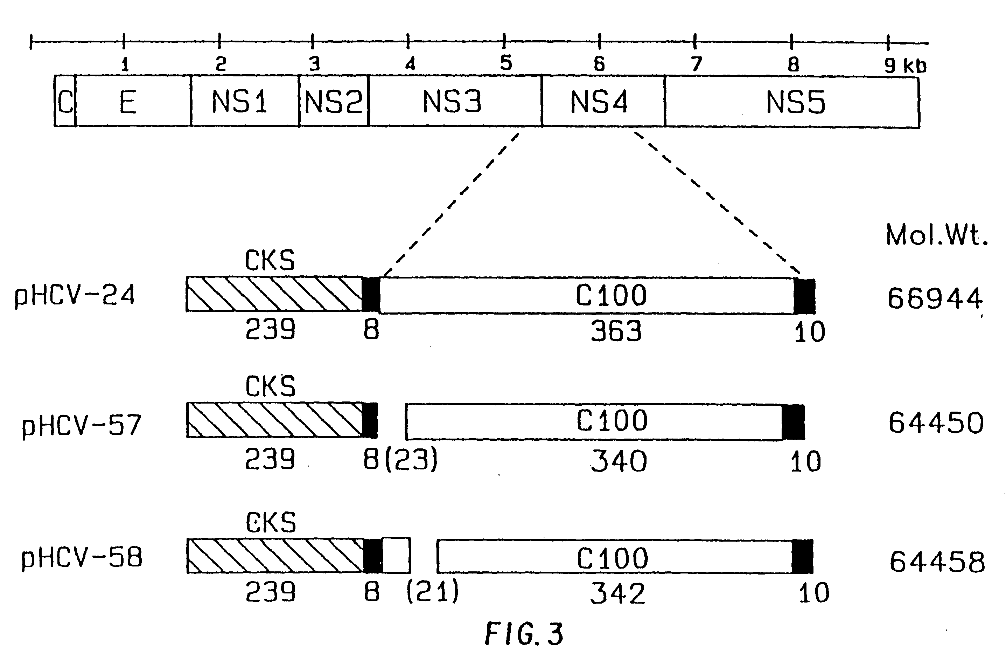

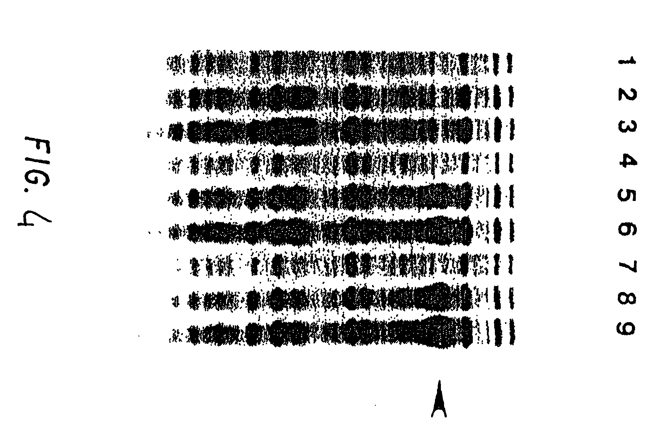

Poor expression levels of this HCV CKS-c100 recombinant antigen were overcome by constructing two additional clones containing deletions in the extreme amino terminal portion of the HCV c100 region. The first of these clones, designated pHCV-57, contains a 23 amino acid deletion (HCV amino acids 1575-1597) and was constructed by deleting a.69 base pair Ddel restriction fragment. The second of these clones, designated pHCV-58, contains a 21 amino acid deletion (HCV amino acids 1600-1620) and was constructed by deleting a 63 base pair NlalV-Haelll restriction fragment. Figure 3 presents a schematic representation of the recombinant antigens expressed by pHCV-24, pHCV-57, and pHCV-58. SEQ.ID.NO. 22 and 23 presents the DNA and amino acid sequence of the HCV-C100D1 recombinant antigen produced by pHCV-57. SEQ.ID.NO. 24 and 25 presents the DNA and amino acid sequence of the HCV-C100D2 recombinant antigen produced by pHCV-58. Figure 4 presents the expression of pHCV-24, pHCV-57, and pHCV-58 proteins in E. coli. Lane 1 contained the E. coli lysate containing pHCV-24 expressing the HCV CKS-c100 antigen (amino acids 1569-1931) prior to induction and lanes 2 and 3 after 2 and 4 hours post induction, respectively. Lane 4 contained the E. coli lysate containing pHCV-57 expressing the HCV-CKS-C100D1 antigen (amino acids 1569-1574 and 1598-1931) prior to induction and lanes 5 and 6 after 2 and 4 hours induction, respectively. Lane 7 contained the E. coli lysate containing pHCV-58 expressing the HCV CKS-C100D2 antigen'(amino acids 1569-1599 and 1621-1931) prior to induction, and lanes 8 and 9 after 2 and 4 hours induction, respectively. These results show that both the pHCV-57 and pHCV-58 fusion proteins express at significantly higher levels than the pHCV-24 fusion protein and that both the pHCV-57 and pHCV-58 fusion proteins have an apparent mobility corresponding to a molecular size of 65,000 daltons. This compares acceptably to the predicted molecular mass of 64,450 daltons for pHCV-57 and 64,458 daltons for pHCV-58.

### EXAMPLE 3 HCV CKS-C100A

### A. Construction of HCV CKS-C100A Deletion Clones

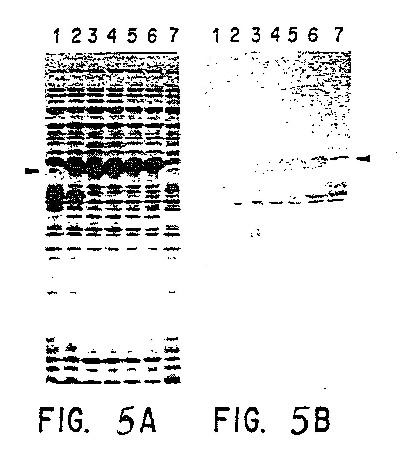

Example 2 described the construction of a synthetic gene encoding HCV a.a. 1569-1931. Expression of this synthetic gene as a CKS fusion protein in E. coli was at very low levels. In order to define the region(s) that were deleterious to the expression of the HCV CKS-C100 antigen in E. coli. the expression levels of four separate subfragments of HCV C100 were examined as fusions to CKS. One such clone, designated pHCV-19, contained HCV a.a. 1569-1677 and did not express the HCV CKS-C100A protein for which it was designed. Two internal deletions located in the amino terminal portion of C100 (HCV a.a. 1575-1620) were constructed. The first of these, pHCV-54, was an internal deletion of 23 amino acids (HCV a.a.1575-1597) using the restriction site Ddel. This deletion expressed well as a CKS fusion in E. coli. SEQ.ID.NO. 1 presents the amino acid sequence of the antigen produced by pHCV-54 . Figure 5A presents the expression of this HCV CKS-C100A protein in E. coli. Figure 5B presents an immunoblot of the antigen produced by pHCV-54. Lane 2 contained the E. coli lysate from pHCV-54 expressing the HCV CKS-C100A antigen four hours post induction. The second of these deletion clones, pHCV-55, deleted 21 amino acids (HCV a.a. 1600-1620) and utilized the restriction sites NlalV/Haelll. This internal deletion also expressed well in E. coli as a CKS fusion. SEQ.ID.NO. 2 presents the amino acid sequence of the antigen produced by pHCV-55. Figure 5A presents the expression of this HCV CKS-C100A protein in E. coli. Figure 5B presents an immunoblot of the antigen produced by pHCV-55. Lane 3 contained the E. coli lysate from pHCV-55 expressing the HCV CKS-C100A antigen four hours post induction.

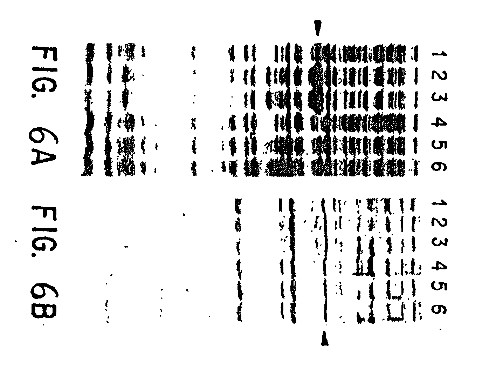

The HCV amino acids deleted in pHCV-55 (HCV a.a. 1600-1620) were sequentially replaced from the carboxy-terminal end using a fragment replacement method. The DNA fragments that were inserted were synthesized as complimentary pairs of single-stranded oligonucleotides. The oligonucieotide pairs were kinased, annealed, and ligated to the remainder of the C100A fragment from pHCV-19 using the restriction enzymes Bsp1286l/Sau96l. The resulting new C-100A fragments were cloned into the CKS fusion expression vector pJO200 and expressed in E. coli. Table 1 summarizes both the HCV amino acids that were manipulated as well as the expression levels of the various HCV CKS-C100A antigens in E. coli. SEQ.ID.NO.3 presents the amino acid sequence of the antigen produced by pHCV-94. Figure 5A presents the expression of this HCV CKS-C100A protein in E. coli. Figure 5B presents an immunoblot of the antigen produced by pHCV-94. Lane 4 contained the E. coli lysate from pHCV-94 expressing the HCV CKS-C100A antigen four hours post induction. SEQ.ID.NO. 4 presents the amino acid sequence of the antigen produced by pHCV-95. Figure 5A presents the expression of the HCV CKS-C100A protein in E. coli. Figure 5B presents an immunoblot of the antigen produced by pHCV-95. Lane 5 contained the E. coli lysate from pHCV-95 expressing the HCV CKS-C100A antigen four hours post induction. SEQ.ID.NO. 5 presents the amino acid sequence of the antigen produced by pHCV-96. Figure 5A presents the expression of this HCV CKS-C100A protein in E. coli. Figure 5B presents an immunoblot of the antigen produced by pHCV-96. Lane 6 contained the E. coli lysate from pHCV-96 expressing the HCV CKS-C100A antigen four hours post induction. SEQ.ID.NO. 6 presents the amino acid sequence of the antigen produced by pHCV-97. Figure 5A presents the expression of this HCV CKS-C100A protein in E. coli. Figure 5B presents an immunoblot of the antigen produced by pHCV-97. Lane 7 contained the E. coli lysate from pHCV-97 expressing the HCV CKS-C100A antigen four hours post induction. SEQ.ID.NO. 7 presents the amino acid sequence of the antigen produced by pHCV-202. Figure 6A presents the expression of this HCV CKS-C100A in E. coli. Figure 6B presents an immunoblot of the antigen produced by pHCV-202. Lanes 1, 2, and 3 contained the E. coli lysate from pHCV-202 expressing the HCV CKS-C100A antigen before induction and two and four hours post induction, respectively. SEQ.ID.NO 8 presents the amino acid sequence of the antigen produced by pHCV-203. Figure 6A presents the expression of this HCV CKS-C100A protein in E. coli. Figure 6B presents an immunoblot of the antigen produced by pHCV-203. Lanes 1, 2, and 3 contained the E. coli lysate from pHCV-203 expressing the HCV CKS-C100A antigen before induction and two and four hours post induction, respectively. SEQ.ID.NO. 19 delineates the amino acids that were manipulated in the fragment replacements described above. The results summarized in Table 1 indicated that the deletion of three proline residues at HCV a.a. 1600-1602 (pHCV-96) (SEQ.ID.NO. 5) permitted expresion of the HCV CKS-C100A antigen at high levels in E. coli. Further analysis showed that the deletion of two proline residues at HCV a.a. 1600-1601 (pHCV-202) also maintained expression of this antigen at high levels. However, when only one of these proline residues was deleted (pHCV-203)(SEQ.ID.NO. 8), the expression level of this antigen was reduced to that of pHCV-19, the original HCV CKS-C100A clone. Therefore, the deletion of two prolines at HCV a.a. 1600-1601 contributes to high level expression of the HCV CKS-C100A antigen in E. coli as determined by SDS-PAGE analysis. Western blot analysis of these deletion clones indicated a high degree of immunoreactivity when probed with human sera containing antibodies to HCV C100.

**TABLE 1**

| Plasmid | HCV a.a. Deleted | Expression Level |
|---|---|---|
| pHCV-19 | | +/- |
| pHCV-55 | 1600-1620 | +++ |
| pHCV-94 | 1600-1612 | +++ |
| pHCV-95 | 1600-1607 | +++ |
| pHCV-96 | 1600-1602 | +++ |
| pHCV-97 | * | +/- |
| pHCV-202 | 1600-1601 | +++ |
| pHCV-203 | 1600 | +/- |
| +/- = expression detectable by Western blot only. | | |
| +++ = expression level > 10% of total cell protein by coomassie stained gel | | |
| * indicates the use of alternate codons optimized for E. coli expression | | |

### B. Construction of HCV CKS-C100 Expression Clones

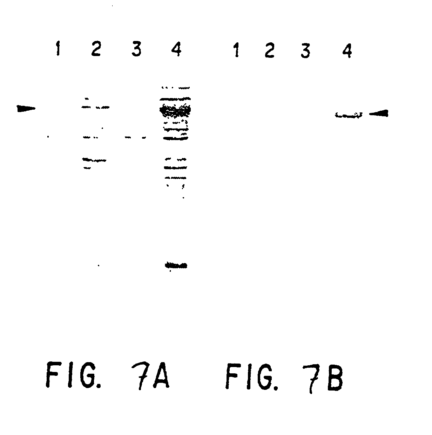

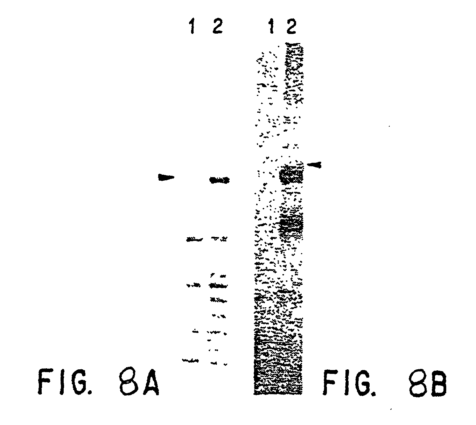

In order to construct a vector which expressed the various HCV CKS-C100 deletion antigens at high levels in E. coli. we replaced the EcoRl/Sall fragment from pHCV-24 with the various corresponding EcoRl/Sall fragments from the deletion clones described above. The EcoRl/Sall fragments from pHCV-54 and pHCV-55 were used to replace the EcoRl/Sall fragment of pHCV-24 to create the plasmids pHCV-57 and pHCV-58 as described in Example 2. The HCV CKS-C100 antigens encoded by pHCV-57 and pHCV-58 expressed well in E. coli. The 3' end of these clones were altered by PCR changing the linker associated amino acid sequence from WDPLDCRHAK (SEQ. ID. NO. 9) to VHHKR (SEQ. ID. NO. 10). The resulting plasmids were designated pHCV-62 and pHCV-63, respectively. SEQ.ID.NO. 11 presents the amino acid sequence of the antigen produced by pHCV-62. SEQ.ID.NO. 12 presents the amino acid sequence of the antigen produced by pHCV-63. The HCV CKS-C100 antigens encoded by pHCV-62 and pHCV-63 expressed well in E. coli. Figure 7A presents the expression of these HCV CKS-C100 antigens in E. coli. Figure 7B presents an immunoblot of the antigens produced by pHCV-62 and pHCV-63. Lanes 1 and 2 contained the E. coli lysate from pHCV-62 expressing the HCV CKS-C100 antigen prior to induction and four hours post induction, respectively. Lanes 3 and 4 contained the E. coil lysate from pHCV-63 expressing the HCV⁻CKS-C100 antigen prior to induction and four hours post induction, respectively. The EcoRl/Sall fragment from pHCV-202 was used to replace the EcoRl/Sall fragment of pHCV-63 to create the plasmid pHCV-204. SEQ.ID.NO. 13 presents the amino acid sequence of the antigen produced by pHCV-204. Table 2 summarizes the deletion analysis conducted on the HCV CKS-C100 antigens and their expression levels in E. coli. The expression levels of the HCV CKS-C100 deletion clones corresponded with the expression levels of the HCV CKS-C100A deletion clones from which they were derived. Figure 8A presents the expression of pHCV-204 protein in E. coli. Figure 8B presents an immunoblot of the antigen produced by pHCV-204. Lanes 1 and 2 contained the E. coli lysate from pHCV-204 expressing the HCV CKS-C100 antigen prior to induction and four hours post induction, respectively.

**TABLE 2**

| PLASMID | HCV a.a. DELETED | EXPRESSION LEVEL |
|---|---|---|
| HCV-57 | 1575-1597 | +++ |
| pHCV-58 | 1600-1620 | +++ |
| pHCV-62 | 1575-1597 | +++ |
| pHCV-63 | 1600-1620 | +++ |
| pHCV-204 | 1600-1601 | +++ |
| +/- = expression detectable by Western blot only | | |
| +++ = expression level > 10% of total cell protein by coomassie stained gel | | |

### EXAMPLE 11. HCV CKS-C200

### Construction of HCV CKS-C200 Expression Clones

The construction of a clone which expressed the HCV CKS-C200 antigen (HCV a.a. 1192-1931) at high levels of E. coli required the steps described hereinbelow.

First, a clone expressing the HCV CKS-33C antigen (HCV a.a. 1192-1457) was constructed as follows and designated pHCV-29.

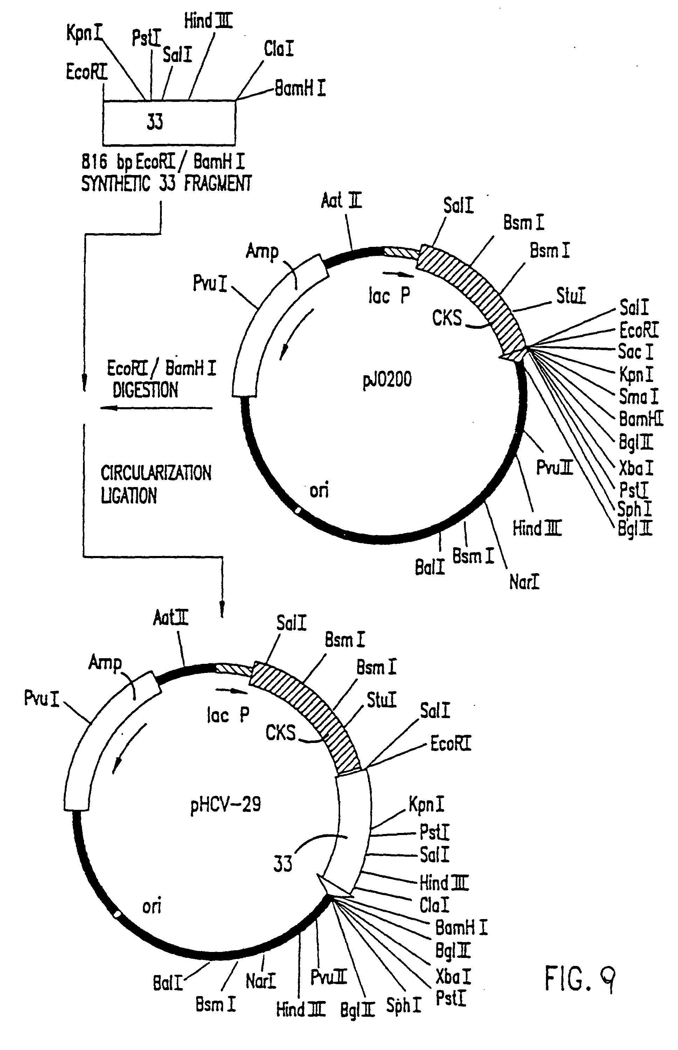

To construct the plasmid pHCV-29 twelve individual oligonucleotides representing amino acids 1192-1457 of the HCV genome were ligated together and cloned as two separate EcoRl-BamHl subfragments in the CKS fusion vector pJO200. After subsequent DNA sequence confirmation, the two subfragments were digested with the appropriate restriction enzymes, gel purified, ligated together and cloned again as an 816 base pair EcoRl-BamHl fragment in the CKS fusion vector pJO200, as illustrated in Figure 9. The resulting plasmid, designated pHCV-29, expresses the CKS-33 antigen under control of the lac promoter. The HCV CKS-33 antigen consists of 239 amino acids of CKS, eight amino acids contributed by linker DNA sequences, and 266 amino acids from the HCV NS3 region (amino acids 1192-1457). Second, a clone containing the DNA sequence encoding HCV a.a. 1454-1569 was constructed using a PCR methodology as follows and designated as pHCV-108 (SEQ. ID. NO. 20). This DNA sequence was later cloned as an in-frame fusion to CKS in order to express the HCV CKS-NS3-1 antigen (HCV a.a. 1454-1568), designated pHCV-112 (SEQ. ID. 14). In the general procedure for preparing HCV DNA fragments using PCR, RNA was extracted from the serum of various chimpanzees or humans infected with HCV by first subjecting the samples to digestion with Proteinase K and SDS for 1 hour at 37° centigrade followed by numerous phenol:chloroform extractions. The RNA was then concentrated by several ethanol precipitations and resuspended in water. RNA samples were then reverse transcribed according to supplier's instructions using a specific primer. A second primer was then added and PCR amplification was performed according to supplier's instructions. An aliquot of this PCR reaction was then subjected to an additional round of PCR using nested primers located internal to the first set of primers. In general, these primers also contained restriction endonuclease recognition sequences to be used for subsequent cloning. An aliquot of this second round nested PCR reaction was then subjected to agarose gel electrophoresis and Southern blot analysis to confirm the specificity of the PCR reaction. The remainder of the PCR reaction was then digested with the appropriate restriction enzymes, the HCV DNA fragment of interest gel purified, and ligated to an appropriate cloning vector. This ligation was then transformed into E. coli and single colonies were isolated and plasmid DNA prepared for DNA sequences analysis. The DNA sequences was then evaluated to confirm that the specific HCV coding region of interest was intacL HCV DNA fragments obtained in this manner were then cloned into appropriate vectors for expression analysis.

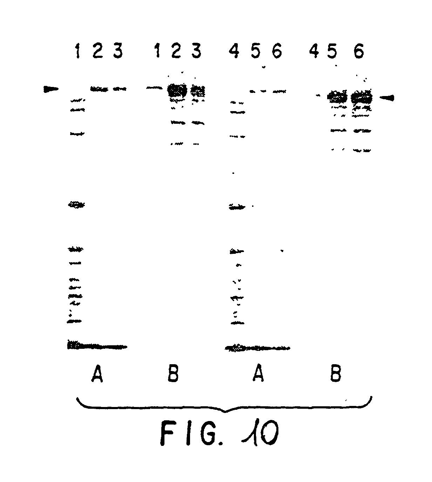

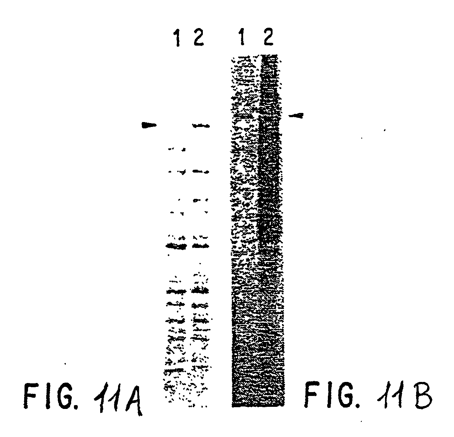

Third, a clone expressing the HCV CKS-C100 deletion antigen (HCV a.a. 1569-1574 and 1598-1931) was constructed, designated pHCV-62 described above. Fourth, the Ncol fragment containing the C100 coding region was excised from pHCV-62 and inserted into the Ncol site of pHCV-108 to create pHCV-68 (SEQ. ID. 15). Lastly, the Clal/BamHl fragment containing the HCV NS3/C100 coding region (HCV a.a. 1454-1574 and 1598-1931) was excised from pHCV-68 and inserted into the Clal/BamHl sites of pHCV-29. The resultant clone, designated pHCV-72, expresses the HCV CKS C200 antigen (HCV a.a. 1192-1574 and 1598-1931). SEQ.ID.NO. 16 presents the amino acid sequence of the antigen produced by pHCV-72. In a similar manner, the C100 coding region of pHCV-63 was substituted for that of pHCV-62 to generate pHCV-69 (SEQ. ID. 21). The Clal/BamHl fragment containing the HCV NS3/C100 coding region (HCV a.a. 1454-1599 and 1621-1931) was excised frompHCV-69 and inserted into the Clal/BamHl sites of pHCV-29. The resultant clone, designated pHCV-73, expresses the HCV CKS-C200 antigen (HCV a.a. 1192-1599 and 1621-1931). SEQ.ID.NO. 17 presents the amino acid sequence of the antigen produced by pHCV-73. Figure 10A presents the expression of these HCV CKS-C200 antigens in E. coli. FIG. 10B presents an immunoblot of the antigen produced from pHCV-73. Lanes 1, 2, and 3 contained the E. coli lysate from pHCV-72 expressing the HCV CKS-C200 antigen before induction and two and four hours post induction, respectively. Lanes 4, 5, and 6 contained the E. coli lysate from pHCV-73 expressing the HCV CKS-C200 antigen before induction and two and four hours post induction, respectively. A different HCV CKS-C200 construct (HCV a.a. 1192-1599 and 1602-1931) was assembled by first obtaining the HCV C100A region (HCV a.a. 1569-1599 and 1602-1677) as a Clal/Ncol 352 base pair fragment from pHCV-202. The NS3-1 antigen (HCV a.a. 1454-1568) was obtained as a Clal/Ncol 352 base pair fragment from pHCV-72. The last fragment used was the 792 base pair fragment from pHCV-72 which contained C100 BCD (HCV a.a. 1678-1931). These three fragments were ligated to each other Clal/Ncol/Sall/BamHl and ligated into the vector backbone pHCV-29 Clal/BamHl, which contributed the HCV CKS-33C antigen (HCV a.a. 1192-1453). The HCV CKS-C200 construct, designated pHCV-205, was expressed as a CKS fusion in pJO200 and expressed at high levels as determined by coosmasie stained gel and Western blot analysis. SEQ.ID.NO. 18 presents the amino acid sequence of the antigen produced by pHCV-205. Figure 11A presents the expression of the HCV CKS-C200 antigen in E. coli. Figure 11B presents an immunoblot of the antigen produced by pHCV-205. Lane 1 contained the E. coli lysate from pHCV-205 expressing the HCV CKS-C200 antigen before induction and lane 2 presents two hours post induction.

The present invention provides unique antigens corresponding to a distinct antigenic region of the HCV genome which can be utilized as a reagent for the detection and/or confirmation of antibodies and antigen in test samples from individuals exposed to HCV. Although the exact function of the NS4 region is unknown, the antigens described herein are located in the putative immunodominant region of the HCV genome.

The recombinant antigens, either alone or in combination, can be used in the assay formats provided herein It also is contemplated that these recombinant antigens can be used to develop specific inhibitors of viral replication and used for therapeutic purposes, such as for vaccines. Other applications and modifications of the use of these antigens and the specific embodiments of this invention as set forth herein, will be apparent to those skilled in the art. Accordingly, the invention is intended to be limited only in accordance with the appended claims.

## Claims (Claims for the following Contracting State(s): DE, FR, IT)

1. A recombinant fusion protein having SEQ. ID. NO. 12.

2. A recombinant fusion protein having SEQ. ID. NO. 13.

3. A recombinant fusion protein having SEQ. ID. NO. 14.

4. A recombinant fusion protein having SEQ. ID. NO. 17.

5. A recombinant fusion protein having SEQ. ID. NO. 18.

6. A polypeptide containing an HCV sequence corresponding to the HCV sequence contained in the fusion protein SEQ. ID. NO. 12, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS).

7. A polypeptide containing an HCV sequence corresponding to the HCV sequence contained in the fusion protein SEQ. ID. NO. 13, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS).

8. A polypeptide containing an HCV sequence corresponding to the HCV sequence contained in the fusion protein SEQ. ID. NO. 14, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS).

9. A polypeptide containing an HCV sequence corresponding to the HCV sequence contained in the fusion protein SEQ. ID. NO. 17, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS).

10. A polypeptide containing an HCV sequence corresponding to the HCV sequence contained in the fusion protein, SEQ. ID. NO. 18, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS).

11. An assay for identifying the presence of an antibody immunologically reactive with an HCV antigen in a fluid sample comprising contacting the sample with at least one polypeptide selected from
a recombinant protein having SEQ. ID. NO. 12, SEQ. ID. NO. 13, SEQ. ID. NO. 14, SEQ. ID. NO. 17 or SEQ. ID. NO. 18 and
a polypeptide containing an HCV sequence corresponding to the HCV sequence contained in the fusion protein SEQ. ID. NO. 12, SEQ. ID. NO. 13, SEQ. ID. NO. 14, SEQ. ID. NO. 17 or SEQ. ID. NO. 18, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS)
under conditions suitable for complexing the antibody with the polypeptide; and detecting the antibody-polypeptide complex.

12. A confirmatory assay for identifying the presence of an antibody in a fluid sample immunologically reactive with an HCV antigen wherein the sample is used to prepare first and second immunologically equivalent aliquots and the first aliquot is contacted with at least one polypeptide selected from
a recombinant protein having SEQ. ID. NO. 12, SEQ. ID. NO. 13, SEQ. ID. NO. 14, SEQ. ID. NO. 17 or SEQ. ID. NO. 18 and
a polypeptide containing an HCV sequence corresponding to the HCV sequence contained in the fusion protein SEQ. ID. NO. 12, SEQ. ID. NO. 13, SEQ. ID. NO. 14, SEQ. ID. NO. 17 or SEQ. ID. NO. 18, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS)
under conditions suitable for complexing the antibody with the polypeptide and wherein the first antibody-antigen complex is detected, and:
contacting the second aliquot with a polypeptide selected from
a recombinant protein having SEQ. ID. NO. 12, SEQ. ID. NO. 13, SEQ. ID. NO. 14, SEQ. ID. NO. 17 or SEQ. ID. NO. 18 and
a polypeptide containing an HCV sequence corresponding to the HCV sequence contained in the fusion protein SEQ. ID. NO. 12, SEQ. ID. NO. 13, SEQ. ID. NO. 14, SEQ. ID. NO. 17 or SEQ. ID. NO. 18, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS)
under conditions suitable to form a second antibody-antigen complex; and detecting the second antibody-antigen complex; wherein the polypeptide selected in the first aliquot is not the same as the polypeptide selected in the second aliquot.

13. An immunodot assay for identifying the presence of an antibody immunologically reactive with an HCV antigen in a fluid sample wherein the sample is concurrently contacted with at least two polypeptides separately bound to distinct regions of the solid support, each containing distinct epitopes of an HCV antigen under conditions suitable for complexing the antibody with the polypeptide; and detecting the antibody-polypeptide complex, and
wherein said polypeptides are selected from
a recombinant protein having SEQ. ID. NO. 12, SEQ. ID. NO. 13, SEQ. ID. NO. 14, SEQ. ID. NO. 17 or SEQ. ID. NO. 18 and
a polypeptide containing an HCV sequence corresponding to the HCV sequence contained in the fusion protein SEQ. ID. NO. 12, SEQ. ID. NO. 13, SEQ. ID. NO. 14, SEQ. ID. NO. 17 or SEQ. ID. NO. 18, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS).

14. A competition assay for identifying the presence of an antibody immunologically reactive with an HCV antigen in a fluid sample wherein the sample is used to prepare first and second immunologically equivalent aliquots wherein the first aliquot is contacted with a polypeptide bound to a solid support under conditions suitable for complexing the antibody with the polypeptide to form a detectable antibody-polypeptide complex, and wherein the second aliquot is first contacted with unbound polypeptide and then contacted with said bound polypeptide wherein the polypeptide is selected from
a recombinant protein having SEQ. ID. NO. 12, SEQ. ID. NO. 13, SEQ. ID. NO. 14, SEQ. ID. NO. 17 or SEQ. ID. NO. 18 and
a polypeptide containing an HCV sequence corresponding to the HCV sequence contained in the fusion protein SEQ. ID. NO. 12, SEQ. ID. NO. 13, SEQ. ID. NO. 14, SEQ. ID. NO. 17 or SEQ. ID. NO. 18, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS).

15. A competition assay for identifying the presence of an antibody immunologically reactive with an HCV antigen in a fluid sample wherein the sample is used to prepare first and second immunologically equivalent aliquots wherein the first aliquot is contacted with a polypeptide bound to a solid support under conditions suitable for complexing the antibody with the polypeptide to form a detectable antibody-polypeptide complex, and wherein the second aliquot is first contacted with unbound polypeptide and then contacted with said bound polypeptide wherein the polypeptide is selected from
a recombinant protein having SEQ. ID. NO. 12, SEQ. ID. NO. 13, SEQ. ID. NO. 14, SEQ. ID. NO. 17 or SEQ. ID. NO. 18 and
a polypeptide containing an HCV sequence corresponding to the HCV sequence contained in the fusion protein SEQ. ID. NO. 12, SEQ. ID. NO. 13, SEQ. ID. NO. 14, SEQ. ID. NO. 17 or SEQ. ID. NO. 18, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS);
wherein the second aliquot is contacted with unbound and bound polypeptide simultaneously.

16. A neutralization assay for identifying the presence of an antibody immunologically reactive with an HCV antigen in a fluid sample wherein the sample is used to prepare first and second immunologically equivalent aliquots wherein the first aliquot is contacted with a polypeptide bound to a solid support under conditions suitable for complexing the antibody with the polypeptide to form a detectable antibody-polypeptide complex, wherein the bound polypeptide is selected from
a recombinant protein having SEQ. ID. NO. 12, SEQ. ID. NO. 13, SEQ. ID. NO. 14, SEQ. ID. NO. 17 or SEQ. ID. NO. 18 and
a polypeptide containing an HCV sequence corresponding to the HCV sequence contained in the fusion protein SEQ. ID. NO. 12, SEQ. ID. NO. 13, SEQ. ID. NO. 14, SEQ. ID. NO. 17 or SEQ. ID. NO. 18, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS);
and wherein the second aliquot is first contacted with unbound polypeptide and then contacted with said bound polypeptide wherein the unbound polypeptide is selected from
a recombinant protein having SEQ. ID. NO. 12, SEQ. ID. NO. 13, SEQ. ID. NO. 14, SEQ. ID. NO. 17 or SEQ. ID. NO. 18 and
a polypeptide containing an HCV sequence corresponding to the HCV sequence contained in the fusion protein SEQ. ID. NO. 12, SEQ. ID. NO. 13, SEQ. ID. NO. 14, SEQ. ID. NO. 17 or SEQ. ID. NO. 18, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS);
and wherein the bound polypeptide selected is not the same as the unbound polypeptide selected.

17. A neutralization assay for identifying the presence of an antibody immunologically reactive with an HCV antigen in a fluid sample wherein the sample is used to prepare first and second immunologically equivalent aliquots wherein the first aliquot is contacted with a polypeptide bound to a solid support under conditions suitable for complexing the antibody with the polypeptide to form a detectable antibody-polypeptide complex, wherein the bound polypeptide is selected from
a recombinant protein having SEQ. ID. NO. 12, SEQ. ID. NO. 13, SEQ. ID. NO. 14, SEQ. ID. NO. 17 or SEQ. ID. NO. 18 and
a polypeptide containing an HCV sequence corresponding to the HCV sequence contained in the fusion protein SEQ. ID. NO. 12, SEQ. ID. NO. 13, SEQ. ID. NO. 14, SEQ. ID. NO. 17 or SEQ. ID. NO. 18, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS);
and wherein the second aliquot is first contacted with unbound polypeptide and then contacted with said bound polypeptide wherein the unbound polypeptide is selected from
a recombinant protein having SEQ. ID. NO. 12, SEQ. ID. NO. 13, SEQ. ID. NO. 14, SEQ. ID. NO. 17 or SEQ. ID. NO. 18 and
a polypeptide containing an HCV sequence corresponding to the HCV sequence contained in the fusion protein SEQ. ID. NO. 12, SEQ. ID. NO. 13, SEQ. ID. NO. 14, SEQ. ID. NO. 17 or SEQ. ID. NO. 18, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS);
and wherein the bound polypeptide selected is not the same as the unbound polypeptide selected;
and wherein the second aliquot is contacted with unbound and bound polypeptide simultaneously.

18. An immunoassay kit comprising:
a polypeptide containing at least one HCV antigen selected from
a recombinant protein having SEQ. ID. NO. 12, SEQ. ID. NO. 13, SEQ. ID. NO. 14, SEQ. ID. NO. 17 or SEQ. ID. NO. 18 and
a polypeptide containing an HCV sequence corresponding to the HCV sequence contained in the fusion protein SEQ. ID. NO. 12, SEQ. ID. NO. 13, SEQ. ID. NO. 14, SEQ. ID. NO. 17 or SEQ. ID. NO. 18, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS);
one or more sample preparation reagents;
and one or more detection and signal producing reagents.

19. A kit of Claim 18 wherein the polypeptides are bound to a solid support.

## Claims (Claims for the following Contracting State(s): ES)

1. An assay for identifying the presence of an antibody immunologically reactive with an HCV antigen in a fluid sample comprising contacting the sample with at least one polypeptide selected from
a recombinant protein having SEQ. ID. NO. 12, SEQ. ID. NO. 13, SEQ. ID. NO. 14, SEQ. ID. NO. 17 or SEQ. ID. NO. 18 and
a polypeptide containing an HCV sequence corresponding to the HCV sequence contained in the fusion protein SEQ. ID. NO. 12, SEQ. ID. NO. 13, SEQ. ID, NO. 14, SEQ. ID. NO. 17 or SEQ. ID. NO. 18, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS)
under conditions suitable for complexing the antibody with the polypeptide; and detecting the antibody-polypeptide complex.

2. A confirmatory assay for identifying the presence of an antibody in a fluid sample immunologically reactive with an HCV antigen wherein the sample is used to prepare first and second immunologically equivalent aliquots and the first aliquot is contacted with at least one polypeptide selected from
a recombinant protein having SEQ. ID. NO. 12, SEQ. ID. NO. 13, SEQ. ID. NO. 14, SEQ. ID. NO. 17 or SEQ. ID. NO. 18 and
a polypeptide containing an HCV sequence corresponding to the HCV sequence contained in the fusion protein SEQ. ID. NO. 12, SEQ. ID. NO. 13, SEQ. ID. NO. 14, SEQ. ID. NO. 17 or SEQ. ID. NO. 18, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS)
under conditions suitable for complexing the antibody with the polypeptide and wherein the first antibody-antigen complex is detected, and:
contacting the second aliquot with a polypeptide selected from
a recombinant protein having SEQ. ID. NO. 12, SEQ. ID. NO. 13, SEQ. ID. NO. 14, SEQ. ID. NO. 17 or SEQ. ID. NO. 18 and
a polypeptide containing an HCV sequence corresponding to the HCV sequence contained in the fusion protein SEQ. ID. NO. 12, SEQ. ID. NO. 13, SEQ. ID. NO. 14, SEQ. ID. NO. 17 or SEQ. ID. NO. 18, said HCV sequence in said polypeptide not-forming part of a longer HCV sequence, said polypeptide being prepared by recombinant methodologies other than through expression as fusions with a polypeptide encoded by the E. coli CMP-KDO synthetase gene (CKS)
under conditions suitable to form a second antibody-antigen complex; and detecting the second antibody-antigen complex; wherein the polypeptide selected in the first aliquot is not the same as the polypeptide selected in the second aliquot.

3. An immunodot assay for identifying the presence of an antibody immunologically reactive with an HCV antigen in a fluid sample wherein the sample is concurrently contacted with at least two polypeptides separately bound to distinct regions of the solid support, each containing distinct epitopes of an HCV antigen under conditions suitable for complexing the antibody with the polypeptide; and detecting the antibody-polypeptide complex, and
wherein said polypeptides are selected from
a recombinant protein having SEQ. ID. NO. 12, -SEQ. ID. NO. 13, SEQ. ID. NO. 14, SEQ. ID. NO. 17 or SEQ. ID. NO. 18 and
a polypeptide containing an HCV sequence corresponding to the HCV sequence contained in the fusion protein SEQ. ID. NO. 12, SEQ. ID. NO. 13, SEQ. ID. NO. 14, SEQ. ID. NO. 17 or SEQ. ID. NO. 18, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS).

4. A competition assay for identifying the presence of an antibody immunologically reactive with an HCV antigen in a fluid sample wherein the sample is used to prepare first and second immunologically equivalent aliquots wherein the first aliquot is contacted with a polypeptide bound to a solid support under conditions suitable for complexing the antibody with the polypeptide to form a detectable antibody-polypeptide complex, and wherein the second aliquot is first contacted with unbound polypeptide and then contacted with said bound polypeptide wherein the polypeptide is selected from
a recombinant protein having SEQ. ID. NO, 12, SEQ. ID. NO. 13, SEQ. ID. NO. 14, SEQ. ID. NO. 17 or SEQ. ID. NO. 18 and
a polypeptide containing an HCV sequence corresponding to the HCV sequence contained in the fusion protein SEQ. ID. NO. 12, SEQ. ID. NO. 13, SEQ. ID. NO. 14, SEQ. ID. NO. 17 or SEQ. ID. NO. 18, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS).

5. A competition assay for identifying the presence of an antibody immunologically reactive with an HCV antigen in a fluid sample wherein the sample is used to prepare first and second immunologically equivalent aliquots wherein the first aliquot is contacted with a polypeptide bound to a solid support under conditions suitable for complexing the antibody with the polypeptide to form a detectable antibody-polypeptide complex, and wherein the second aliquot is first contacted with unbound polypeptide and then contacted with said bound polypeptide wherein the polypeptide is selected from
a recombinant protein having SEQ. ID. NO. 12, SEQ. ID. NO. 13, SEQ. ID. NO. 14, SEQ. ID. NO. 17 or SEQ. ID. NO. 18 and
a polypeptide containing an HCV sequence corresponding to the HCV sequence contained in the fusion protein SEQ. ID. NO. 12, SEQ. ID. NO. 13, SEQ. ID. NO. 14, SEQ. ID. NO. 17 or SEQ. ID. NO. 18, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS);
wherein the second aliquot is contacted with unbound and bound polypeptide simultaneously.

6. A neutralization assay for identifying the presence of an antibody immunologically reactive with an HCV antigen in a fluid sample wherein the sample is used to prepare first and second immunologically equivalent aliquots wherein the first aliquot is contacted with a polypeptide bound to a solid support under conditions suitable for complexing the antibody with the polypeptide to form a detectable antibody-polypeptide complex, wherein the bound polypeptide is selected from
a recombinant protein having SEQ. ID. NO. 12, SEQ. ID. NO. 13, SEQ, ID. NO. 14, SEQ. ID. NO. 17 or SEQ. ID. NO. 18 and
a polypeptide containing an HCV sequence corresponding to the HCV sequence contained in the fusion protein SEQ. ID. NO. 12, SEQ. ID. NO. 13, SEQ. ID. NO. 14, SEQ. ID. NO. 17 or SEQ. ID. NO. 18, said HCV sequence in said polypeptide not forming part of a longer HCV sequence, said polypeptide-being prepared by recombinant methodologies other than through expression as fusions with a polypeptide encoded by the E. coli CMP-KDO synthetase gene (CKS);
and wherein the second aliquot is first contacted with unbound polypeptide and then contacted with said bound polypeptide wherein the unbound polypeptide is selected from
a recombinant protein having SEQ. ID. NO. 12, SEQ. ID. NO. 13, SEQ. ID. NO. 14, SEQ. ID. NO. 17 or SEQ. ID. NO. 18 and
a polypeptide containing an HCV sequence corresponding to the HCV sequence contained in the fusion protein SEQ. ID. NO. 12, SEQ. ID. NO. 13, SEQ. ID. NO. 14, SEQ. ID. NO. 17 or SEQ. ID. NO. 18, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS);
and wherein the bound polypeptide selected is not the same as the unbound polypeptide selected.

7. A neutralization assay for identifying the presence of an antibody immunologically reactive with an HCV antigen in a fluid sample wherein the sample is used to prepare first and second immunologically equivalent aliquots wherein the first aliquot is contacted with a polypeptide bound to a solid support under conditions suitable for complexing the antibody with the polypeptide to form a detectable antibody-polypeptide complex, wherein the bound polypeptide is selected from
a recombinant protein having SEQ. ID. NO. 12, SEQ. ID. NO. 13, SEQ. ID. NO. 14, SEQ. ID. NO. 17 or SEQ. ID. NO. 18 and
a polypeptide containing an HCV sequence corresponding to the HCV sequence contained in the fusion protein SEQ. ID. NO. 12, SEQ. ID. NO. 13, SEQ. ID. NO. 14, SEQ. ID. NO. 17 or SEQ. ID. NO. 18, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS);
and wherein the second aliquot is first contacted with unbound polypeptide and then contacted with said bound polypeptide wherein the unbound polypeptide is selected from
a recombinant protein having SEQ. ID. NO. 12, SEQ. ID. NO. 13, SEQ. ID. NO. 14, SEQ. ID. NO. 17 or SEQ. ID. NO. 18 and
a polypeptide containing an HCV sequence corresponding to the HCV sequence contained in the fusion protein SEQ. ID. NO. 12, SEQ. ID. NO. 13, SEQ. ID. NO. 14, SEQ. ID. NO. 17 or SEQ. ID. NO. 18, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS);
and wherein the bound polypeptide selected is not the same as the unbound polypeptide selected;
and wherein the second aliquot is contacted with unbound and bound polypeptide simultaneously.

8. An immunoassay kit comprising:
a polypeptide containing at least one HCV antigen selected from
a recombinant protein having SEQ. ID. NO. 12, SEQ. ID. NO. 13, SEQ. ID. NO. 14, SEQ. ID. NO. 17 or SEQ. ID. NO. 18 and
a polypeptide containing an HCV sequence corresponding to the HCV sequence contained in the fusion protein SEQ. ID. NO. 12, SEQ. ID. NO. 13, SEQ. ID. NO. 14, SEQ. ID. NO. 17 or SEQ. ID. NO. 18, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS);
one or more sample preparation reagents;
and one or more detection and signal producing reagents.

9. A kit of Claim 8 wherein the polypeptides are bound to a solid support.

10. Use of an immunoassay kit comprising:
a polypeptide containing at least one HCV antigen selected from
a recombinant protein having SEQ. ID. NO. 12, SEQ. ID. NO. 13, SEQ. ID. NO. 14, SEQ. ID. NO. 17 or SEQ. ID. NO. 18 and
a polypeptide containing an HCV sequence corresponding to the HCV sequence contained in the fusion protein SEQ. ID. NO. 12, SEQ. ID. NO. 13, SEQ. ID. NO. 14, SEQ. ID. NO. 17 or SEQ. ID. NO. 18, said HCV sequence in said polypeptide not forming part of a longer HCV sequence, said polypeptide being prepared by recombinant methodologies other than through expression as fusions with a polypeptide encoded by the E. coli CMP-KDO synthetase gene (CKS);
one or more sample preparation reagents;
and one or more detection and signal producing reagents,
for detecting the presence of an antibody to an HCV antigen in a sample.

11. The use of Claim 10 wherein the polypeptides are bound to a solid support.

## Patentansprüche (Patentansprüche für folgende(n) Vertragsstaat(en): DE, FR, IT)

1. Ein rekombinantes Fusionsprotein mit der Sequenzidentifikationsnr. 12.

2. Ein rekombinantes Fusionsprotein mit der Sequenzidentifikationsnr. 13.

3. Ein rekombinantes Fusionsprotein mit der Sequenzidentifikationsnr. 14.

4. Ein rekombinantes Fusionsprotein mit der Sequenzidentifikationsnr. 17.

5. Ein rekombinantes Fusionsprotein mit der Sequenzidentifikationsnr. 18.

6. Ein Polypeptid, das eine HCV Sequenz enthält entsprechend der HCV Sequenz, die in dem Fusionsprotein mit der Sequenzidentifikationsnr. 12 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird.

7. Ein Polypeptid, das eine HCV Sequenz enthält entsprechend der HCV Sequenz, die in dem Fusionsprotein mit der Sequenzidentifikationsnr. 13 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird.

8. Ein Polypeptid, das eine HCV Sequenz enthält entsprechend der HCV Sequenz, die in dem Fusionsprotein mit der Sequenzidentifikationsnr. 14 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird.

9. Ein Polypeptid, das eine HCV Sequenz enthält entsprechend der HCV Sequenz, die in dem Fusionsprotein mit der Sequenzidentifikationsnr. 17 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird.

10. Ein Polypeptid, das eine HCV Sequenz enthält entsprechend der HCV Sequenz, die in dem Fusionsprotein mit der Sequenzidentifikationsnr. 18 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird.

11. Ein Assay zum Nachweis der Anwesenheit eines immunologisch reaktiven Antikörpers mit einem HCV-Antigen in einer flüssigen Probe bestehend aus In-Kontakt-bringen der Probe mit mindestens einem Polypeptid gewählt aus
einem rekombinanten Protein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 und
einem Polypeptid, das eine HCV-Sequenz enthält entsprechend der HCV-Sequenz, die in dem Fusionsprotein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird,
unter Vorraussetzungen, die zur Komplexierung des Antikörpers mit dem Polypeptid geeignet sind; und Detektieren des Anitkörper-Polypeptid-Komplexes.

12. Ein Bestätigungsassay zum Nachweis der Anwesenheit von einem immunologisch reaktiven Antikörper in einer flüssigen Probe mit einem HCV-Antigen, wobei die Probe verwendet wird zum Herstellen von ersten und zweiten immunologisch äquivalenten Aliquoten und das erste Aliquot mit mindestens einem Polypeptid in Kontakt gebracht wird, gewählt aus
einem rekombinanten Protein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 und
einem Polypeptid, das eine HCV-Sequenz enthält entsprechend der HCV-Sequenz, die in dem Fusionsprotein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird
unter Vorraussetzungen, die zur Komplexierung des Antikörpers mit dem Polypeptid geeignet sind und worin der erste Antikörper-Antigen-Komplex detektiert wird, und:
In-Kontakt-bringen des zweiten Aliquots mit einem Polypeptid gewählt aus
einem rekombinanten Protein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 und
einem Polypeptid, das eine HCV-Sequenz enthält entsprechend der HCV-Sequenz, die in dem Fusionsprotein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird
unter Vorraussetzungen, die geeignet sind, um einen zweiten Antikörper-Antigen-Komplex zu bilden; und Detektieren des zweiten Antikörper-Antigen-Komplexes; wobei das Polypeptid, das im ersten Aliquot gewählt wurde, nicht das gleiche ist wie das Polypeptid, das im zweiten Aliquot gewählt wurde.

13. Ein Immunodotassay zum Nachweis der Anwesenheit eines immunologisch reaktiven Antikörpers mit einem HCV-Antigen in einer flüssigen Probe, wobei die Probe gleichzeitig mit mindestens zwei Polypeptiden in Kontakt gebracht wird, die getrennt voneinander an verschiedene Regionen des festen Trägers gebunden sind, wobei jedes verschiedene Epitope eines HCV-Antigens enthält, unter Bedingungen, die zur Komplexierung des Antikörpers mit dem Polypeptid geeignet sind; und Detektieren des Antikörper-Polypeptid-Komplexes, und
wobei diese Polypeptide gewählt sind
aus einem rekombinatem Protein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 und
einem Polypeptid, das eine HCV-Sequenz enthält entsprechend der HCV-Sequenz, die in dem Fusionsprotein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird.

14. Ein kompetitiver Assay zum Nachweis der Anwesenheit eines immunologisch reaktiven Antikörpers mit einem HCV-Antigen in einer flüssigen Probe, wobei die Probe verwendet wird, um erste und zweite immunologisch äquivalente Aliquote herzustellen, wobei das erste Aliquot mit einem Polypeptid in Kontakt gebracht wird, das an einen festen Träger gebunden ist, unter Bedingungen, die geeignet sind zur Komplexierung des Antikörpers mit dem Polypeptid, um einen detektierbaren Antikörper-Polypeptid-Komplex zu bilden, und wobei das zweite Aliquot zuerst mit ungebundenem Polypeptid und dann mit dem gebundenen Polypeptid in Kontakt gebracht wird, wobei das Polypeptid gewählt ist aus
einem rekombinaten Protein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 und
einem Polypeptid, das eine HCV-Sequenz enthält entsprechend der HCV-Sequenz, die in dem Fusionsprotein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird.

15. Ein kompetitiver Assay zum Nachweis der Anwesenheit eines immunologisch reaktiven Antikörpers mit einem HCV-Antigen in einer flüssigen Probe, wobei die Probe verwendet wird, um erste und zweite immunologisch äquivalente Aliquote herzustellen, wobei das erste Aliquot mit einem Polypeptid in Kontakt gebracht wird, das an einen festen Träger gebunden ist, unter Bedingungen, die geeignet sind zur Komplexierung des Antikörpers mit dem Polypeptid, um einen detektierbaren Antikörper-Polypeptid-Komplex zu bilden, und wobei das zweite Aliquot zuerst mit ungebundenem Polypeptid und dann mit dem gebundenen Polypeptid in Kontakt gebracht wird, wobei das Polypeptid gewählt ist aus
einem rekombinaten Protein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 und
einem Polypeptid das eine HCV-Sequenz enthält entsprechend der HCV-Sequenz, die in dem Fusionsprotein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird;
wobei das zweite Aliquot gleichzeitig mit ungebundenem und gebundenem Polypeptid in Kontakt gebracht wird.

16. Ein Neutralisationsassay zum Nachweis der Anwesenheit eines immunologisch reaktiven Antikörpers mit einem HCV-Antigen in einer flüssigen Probe, wobei die Probe verwendet wird, um erste und zweite immunologisch äquivalente Aliquote herzustellen, wobei das erste Aliquot mit einem Polypeptid in Kontakt gebracht wird, das an einen festen Träger gebunden ist, unter Bedingungen, die zur Komplexierung des Antikörpers mit dem Polypeptid geeignet sind, um einen detektierbaren Antikörper-Polypeptid-Komplex zu bilden, wobei das gebundene Polypeptid gewählt ist aus
einem rekombinaten Protein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 und
einem Polypeptid, das eine HCV-Sequenz enthält entsprechend der HCV-Sequenz, die in dem Fusionsprotein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird;
und wobei das zweite Aliquot zuerst mit ungebundenem Polypeptid und dann mit dem gebundenen Polypeptid in Kontakt gebracht wird, wobei das ungebundene Polypeptid gewählt ist aus
einem rekombinaten Protein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 und
einem Polypeptid, das eine HCV-Sequenz enthält entsprechend der HCV-Sequenz, die in dem Fusionsprotein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird;
und wobei das gewählte gebundene Polypeptid nicht das gleiche ist wie das gewählte ungebundene Polypeptid.

17. Ein Neutralisationsassay zum Nachweis der Anwesenheit eines immunologisch reaktiven Antikörpers mit einem HCV-Antigen in einer flüssigen Probe, wobei die Probe verwendet wird, um erste und zweite immunologisch äquivalente Aliquote herzustellen, wobei das erste Aliquot mit einem Polypeptid in Kontakt gebracht wird, das an einen festen Träger gebunden ist, unter Bedingungen, die zur Komplexierung des Antikörpers mit dem Polypeptid geeignet sind, um einen detektierbaren Antikörper-Polypeptid-Komplex zu bilden, wobei das gebundene Polypeptid gewählt ist aus der Gruppe bestehend aus
einem rekombinanten Protein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 und
einem Polypeptid, das eine HCV-Sequenz enthält entsprechend der HCV-Sequenz, die in dem Fusionsprotein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird;
und wobei das zweite Aliquot zuerst mit ungebundenem Polypeptid und dann mit dem gebundenen Polypeptid in Kontakt gebracht wird, wobei das ungebundene Polypeptid gewählt ist aus
einem rekombinanten Protein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 und
einem Polypeptid, das eine HCV-Sequenz enthält entsprechend der HCV-Sequenz, die in dem Fusionsprotein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird;
und wobei das gewählte gebundene Polypeptid nicht das gleiche ist wie das gewählte ungebundene Polypeptid;
und wobei das zweite Aliquot gleichzeitig mit ungebundenem und gebundenem Polypeptid in Kontakt gebracht wird.

18. Ein Immunoassaykit bestehend aus:
einem Polypeptid, das mindestens ein HCV-Antigen enthält, gewählt aus
einem rekombinantem Protein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 und
einem Polypeptid, das eine HCV-Sequenz enthält entsprechend der HCV-Sequenz, die in dem Fusionsprotein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird;
einem oder mehreren Reagenzien zur Probenherstellung;
und einem oder mehreren Reagenzien zur Detektion und Signalerzeugung.

19. Ein Kit gemäß Anspruch 18, wobei die Polypeptide an einen festen Träger gebunden sind.

## Patentansprüche (Patentansprüche für folgende(n) Vertragsstaat(en): ES)

1. Ein Assay zum Nachweis der Anwesenheit eines immunologisch reaktiven Antikörpers mit einem HCV-Antigen in einer flüssigen Probe bestehend aus In-Kontakt-bringen der Probe mit mindestens einem Polypeptid gewählt aus
einem rekombinanten Protein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 und
einem Polypeptid, das eine HCV-Sequenz enthält entsprechend der HCV-Sequenz, die in dem Fusionsprotein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird,
unter Vorraussetzungen, die zur Komplexierung des Antikörpers mit dem Polypeptid geeignet sind; und Detektieren des Anitkörper-Polypeptid-Komplexes.

2. Ein Bestätigungsassay zum Nachweis der Anwesenheit vor. einem immunologisch reaktiven Antikörper in einer flüssigen Probe mit einem HCV-Antigen, wobei die Probe verwendet wird zum Herstellen von ersten und zweiten immunologisch äquivalenten Aliquoten und das erste Aliquot mit mindestens einem Polypeptid in Kontakt gebracht wird, gewählt aus
einem rekombinanten Protein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 und
einem Polypeptid, das eine HCV-Sequenz entsprechend der HCV-Sequenz die in dem Fusionsprotein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird
unter Vorraussetzungen, die zur Komplexierung des Antikörpers mit dem Polypeptid geeignet sind und worin der erste Antikörper-Antigen-Komplex detektiert wird, und:
In-Kontakt-bringen des zweiten Aliquots mit einem Polypeptid gewählt aus
einem rekombinanten Protein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 und
einem Polypeptid, das eine HCV-Sequenz enthält entsprechend der HCV-Sequenz, die in dem Fusionsprotein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird
unter Vorraussetzungen, die geeignet sind, um einen zweiten Antikörper-Antigen-Komplex zu bilden; und Detektieren des zweiten Antikörper-Antigen-Komplexes; wobei das Polypeptid, das im ersten Aliquot gewählt wurde, nicht das gleiche ist wie das Polypeptid, das im zweiten Aliquot gewählt wurde.

3. Ein Immunodotassay zum Nachweis der Anwesenheit eines immunologisch reaktiven Antikörpers mit einem HCV-Antigen in einer flüssigen Probe, wobei die Probe gleichzeitig mit mindestens zwei Polypeptiden in Kontakt gebracht wird, die getrennt voneinander an verschiedene Regionen des festen Trägers gebunden sind, wobei jedes verschiedene Epitope eines HCV-Antigens enthält, unter Bedingungen, die zur Komplexierung des Antikörpers mit dem Polypeptid geeignet sind; und Detektieren des Antikörper-Polypeptid-Komplexes, und
wobei diese Polypeptide gewählt sind
aus einem rekombinatem Protein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 und
einem Polypeptid, das eine HCV-Sequenz enthält entsprechend der HCV-Sequenz, die in dem Fusionsprotein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird.

4. Ein kompetitiver Assay zum Nachweis der Anwesenheit eines immunologisch reaktiven Antikörpers mit einem HCV-Antigen in einer flüssigen Probe, wobei die Probe verwendet wird, um erste und zweite immunologisch äquivalente Aliquote herzustellen, wobei das erste Aliquot mit einem Polypeptid in Kontakt gebracht wird, das an einen festen Träger gebunden ist, unter Bedingungen, die geeignet sind zur Komplexierung des Antikörpers mit dem Polypeptid, um einen detektierbaren Antikörper-Polypeptid-Komplex zu bilden, und wobei das zweite Aliquot zuerst mit ungebundenem Polypeptid und dann mit dem gebundenen Polypeptid in Kontakt gebracht wird, wobei das Polypeptid gewählt ist aus
einem rekombinaten Protein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 und
einem Polypeptid, das eine HCV-Sequenz enthält entsprechend der HCV-Sequenz, die in dem Fusionsprotein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird.

5. Ein kompetitiver Assay zum Nachweis der Anwesenheit eines immunologisch reaktiven Antikörpers mit einem HCV-Antigen in einer flüssigen Probe, wobei die Probe verwendet wird, um erste und zweite immunologisch äquivalente Aliquote herzustellen, wobei das erste Aliquot mit einem Polypeptid in Kontakt gebracht wird, das an einen festen Träger gebunden ist, unter Bedingungen, die geeignet sind zur Komplexierung des Antikörpers mit dem Polypeptid, um einen detektierbaren Antikörper-Polypeptid-Komplex zu bilden, und wobei das zweite Aliquot zuerst mit ungebundenem Polypeptid und dann mit dem gebundenen Polypeptid in Kontakt gebracht wird, wobei das Polypeptid gewählt ist aus
einem rekombinaten Protein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 und
einem Polypeptid das eine HCV-Sequenz enthält entsprechend der HCV-Sequenz, die in dem Fusionsprotein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird;
wobei das zweite Aliquot gleichzeitig mit ungebundenem und gebundenem Polypeptid in Kontakt gebracht wird.

6. Ein Neutralisationsassay zum Nachweis der Anwesenheit eines immunologisch reaktiven Antikörpers mit einem HCV-Antigen in einer flüssigen Probe, wobei die Probe verwendet wird, um erste und zweite immunologisch äquivalente Aliquote herzustellen, wobei das erste Aliquot mit einem Polypeptid in Kontakt gebracht wird, das an einen festen Träger gebunden ist, unter Bedingungen, die zur Komplexierung des Antikörpers mit dem Polypeptid geeignet sind, um einen detektierbaren Antikörper-Polypeptid-Komplex zu bilden, wobei das gebundene Polypeptid gewählt ist aus
einem rekombinaten Protein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 und
einem Polypeptid, das eine HCV-Sequenz enthält entsprechend der HCV-Sequenz, die in dem Fusionsprotein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird;
und wobei das zweite Aliquot zuerst mit ungebundenem Polypeptid und dann mit dem gebundenen Polypeptid in Kontakt gebracht wird, wobei das ungebundene Polypeptid gewählt ist aus
einem rekombinaten Protein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 und
einem Polypeptid, das eine HCV-Sequenz enthält entsprechend der HCV-Sequenz, die in dem Fusionsprotein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird;
und wobei das gewählte gebundene Polypeptid nicht das gleiche ist wie das gewählte ungebundene Polypeptid.

7. Ein Neutralisationsassay zum Nachweis der Anwesenheit eines immunologisch reaktiven Antikörpers mit einem HCV-Antigen in einer flüssigen Probe, wobei die Probe verwendet wird, um erste und zweite immunologisch äquivalente Aliquote herzustellen, wobei das erste Aliquot mit einem Polypeptid in Kontakt gebracht wird, das an einen festen Träger gebunden ist, unter Bedingungen, die zur Komplexierung des Antikörpers mit dem Polypeptid geeignet sind, um einen detektierbaren Antikörper-Polypeptid-Komplex zu bilden, wobei das gebundene Polypeptid gewählt ist aus der Gruppe bestehend aus
einem rekombinanten Protein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 und
einem Polypeptid, das eine HCV-Sequenz enthält entsprechend der HCV-Sequenz, die in dem Fusionsprotein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird;
und wobei das zweite Aliquot zuerst mit ungebundenem Polypeptid und dann mit dem gebundenen Polypeptid in Kontakt gebracht wird, wobei das ungebundene Polypeptid gewählt ist aus
einem rekombinanten Protein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 und
einem Polypeptid, das eine HCV-Sequenz enthält entsprechend der HCV-Sequenz, die in dem Fusionsprotein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird;
und wobei das gewählte gebundene Polypeptid nicht das gleiche ist wie das gewählte ungebundene Polypeptid;
und wobei das zweite Aliquot gleichzeitig mit ungebundenem und gebundenem Polypeptid in Kontakt gebracht wird.

8. Ein Immunoassaykit bestehend aus:
einem Polypeptid, das mindestens ein HCV-Antigen enthält, gewählt aus
einem rekombinantem Protein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 und
einem Polypeptid, das eine HCV-Sequenz enthält entsprechend der HCV-Sequenz, die in dem Fusionsprotein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird;
einem oder mehreren Reagenzien zur Probenherstellung;
und einem oder mehreren Reagenzien zur Detektion und Signalerzeugung.

9. Ein Kit gemäß Anspruch 8, wobei die Polypeptide an einen festen Träger gebunden sind.

10. Verwendung eines Immunoassaykits bestehend aus:
einem Polypeptid, das mindestens ein HCV-Antigen enthält, gewählt aus
einem rekombinantem Protein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 und
einem Polypeptid, das eine HCV-Sequenz enthält entsprechend der HCV-Sequenz, die in dem Fusionsprotein mit der Sequenzidentifikationsnr. 12, Sequenzidentifikationsnr. 13, Sequenzidentifikationsnr. 14, Sequenzidentifikationsnr. 17 oder Sequenzidentifikationsnr. 18 enthalten ist, wobei die HCV-Sequenz in dem Polypeptid kein Teil von einer längeren HCV-Sequenz ist, wobei das Polypeptid durch rekombinante Verfahrensweisen hergestellt wird, anders als durch Expression als Fusionen mit einem Polypeptid, das durch das E. coli CMP-KDO Synthetase-Gen (CKS) kodiert wird;
einem oder mehreren Reagenzien zur Probenherstellung;
und einem oder mehreren Reagenzien zur Detektion und Signalerzeugung,
zur Detektion der Anwesenheit eines Antikörpers zu einem HCV-Antigen in einer Probe.

11. Verwendung gemäß Anspruch 10, worin die Polypeptide an einen festen Träger gebunden sind.

## Revendications (Revendications pour l'(les) Etat(s) contractant(s) suivant(s): DE, FR, IT)

1. Protéine de fusion recombinée ayant la séquence SEQ ID N° 12.

2. Protéine de fusion recombinée ayant la séquence SEQ ID N° 13.

3. Protéine de fusion recombinée ayant la séquence SEQ ID N° 14.

4. Protéine de fusion recombinée ayant la séquence SEQ ID N° 17.

5. Protéine de fusion recombinée ayant la séquence SEQ ID N° 18.

6. Polypeptide contenant une séquence du VHC correspondant à la séquence de VHC contenue dans la protéine de fusion ayant la séquence SEQ ID N° 12, ladite séquence de VHC dans ledit polypeptide ne formant pas une partie d'une séquence de VHC plus longue, ledit polypeptide étant préparé par des techniques de génie génétique autres que l'expression sous forme de fusions avec un polypeptide codé par le gène de la CMP-KDO synthétase (CKS) de *E. coli*.

7. Polypeptide contenant une séquence de VHC correspondant à la séquence de VHC contenue dans la protéine de fusion ayant la séquence SEQ ID N° 13, ladite séquence de VHC dans ledit polypeptide ne formant pas une partie d'une séquence de VHC plus longue, ledit polypeptide étant préparé par des techniques de génie génétique autres que l'expression sous forme de rusions avec un polypeptide codé par le gène de la CMP-KDO synthétase (CKS) de *E. coli*.

8. Polypeptide contenant une séquence de VHC correspondant à la séquence de VHC contenue dans la protéine de fusion ayant la séquence SEQ ID N° 14, ladite séquence de VHC dans ledit polypeptide ne formant pas une partie d'une séquence de VHC plus longue, ledit polypeptide étant préparé par des techniques de génie génétique autres que l'expression sous forme de fusions avec un polypeptide codé par le gène de la CMP-KDO synthétase (CKS) de *E. coli.*

9. Polypeptide contenant une séquence de VHC correspondant à la séquence de VHC contenue dans la protéine de fusion ayant la séquence SEQ ID N° 17, ladite séquence de VHC dans ledit polypeptide ne formant pas une partie d'une séquence de VHC plus longue, ledit polypeptide étant préparé par des techniques de génie génétique autres que l'expression sous forme de fusions avec un polypeptide codé par le gène de la CMP-KDO synthétase (CKS) de *E. coli*.

10. Polypeptide contenant une séquence de VHC correspondant à la séquence de VHC contenue dans la protéine de fusion ayant la séquence SEQ ID N° 18, ladite séquence de VHC dans ledit polypeptide ne formant pas une partie d'une séquence de VHC plus longue, ledit polypeptide étant préparé par des techniques de génie génétique autres que l'expression sous forme de fusions avec un polypeptide codé par le gène de la CMP-KDO synthétase (CKS) de *E. coli*.

11. Analyse destinée à identifier la présence d'un anticorps immunologiquement réactif avec un antigène du VHC dans un échantillon liquide, comprenant la mise en contact de l'échantillon avec au moins un polypeptide choisi parmi
une protéine recombinée ayant la séquence SEQ ID N° 12, SEQ ID N° 13, SEQ ID N° 14, SEQ ID N° 17 ou SEQ ID N° 18 et
un polypeptide contenant une séquence de VHC correspondant à la séquence de VHC contenue dans la protéine de fusion ayant la séquence SEQ ID N° 12, SEQ ID N° 13, SEQ ID N° 14, SEQ ID N° 17 ou SEQ ID N° 18, ladite séquence de VHC dans ledit polypeptide ne formant pas une partie d'une séquence de VHC plus longue, ledit polypeptide étant préparé par des techniques de génie génétique autres que l'expression sous forme de fusions avec un polypeptide codé par le gène de la CMP-KDO synthétase (CKS) de *E. coli*,
dans des conditions appropriées à la complexation de l'anticorps avec le polypeptide; et la détection du complexe anticorps-polypeptide.

12. Analyse de confirmation pour l'identification de la présence d'un anticorps immunologiquement réactif avec un antigène de VHC dans un échantillon liquide, dans lequel on utilise l'échantillon pour préparer une première et une deuxième aliquotes immunologiquement équivalentes et on met en contact la première aliquote avec au moins un polypeptide choisi parmi
une protéine recombinée ayant la séquence SEQ ID N° 12, SEQ ID N° 13, SEQ ID N° 14, SEQ ID N° 17 ou SEQ ID N° 18 et
un polypeptide contenant une séquence de VHC correspondant à la séquence de VHC contenue dans la protéine de fusion ayant la séquence SEQ ID N° 12, SEQ ID N° 13, SEQ ID N° 14, SEQ ID N° 17 ou SEQ ID N° 18, ladite séquence de VHC dans ledit polypeptide ne formant pas une partie d'une séquence de VHC plus longue, ledit polypeptide étant préparé par des techniques de génie génétique autres que l'expression sous forme de fusions avec un polypeptide codé par le gène de la CMP-KDO synthétase (CKS) de *E. coli,*
dans des conditions appropriées pour la complexation de l'anticorps avec le polypeptide et on détecte le premier complexe anticorps-antigène, et
on met en contact la deuxième aliquote avec un polypeptide choisi parmi
une protéine recombinée ayant la séquence SEQ ID N° 12, SEQ ID N° 13, SEQ ID N° 14, SEQ ID N° 17 ou SEQ ID N° 18 et
un polypeptide contenant une séquence de VHC correspondant à la séquence de VHC contenue dans la protéine de fusion ayant la séquence SEQ ID N° 12, SEQ ID N° 13, SEQ ID N° 14, SEQ ID N° 17 ou SEQ ID N° 18, ladite séquence de VHC dans ledit polypeptide ne formant pas une partie d'une séquence de VHC plus longue, ledit polypeptide étant préparé par des techniques de génie génétique autres que l'expression sous forme de fusions avec un polypeptide codé par le gène de la CMP-KDO synthétase (CKS) de *E. coli,*
dans des conditions appropriées pour la formation d'un deuxième complexe anticorps-antigène et on détecte le deuxième complexe anticorps-antigène, le polypeptide choisi pour la première aliquote n'étant pas le même que celui choisi pour la deuxième aliquote.

13. Analyse par immunoempreinte pour l'identification de la présence d'un anticorps immunologiquement réactif avec un antigène de VHC dans un échantillon liquide, dans laquelle on met l'échantillon en contact simultanément avec au moins deux polypeptides liés séparément à des régions distinctes du support solide, contenant chacun des épitopes distincts d'un antigène de VHC, dans des conditions appropriées pour la complexation de l'anticorps avec le polypeptide; et on détecte le complexe anticorps-polypeptide, lesdits polypeptides étant choisis parmi
une protéine recombinée ayant la séquence SEQ ID N° 12, SEQ ID N° 13, SEQ ID N° 14, SEQ ID N° 17 ou SEQ ID N° 18 et
un polypeptide contenant une séquence de VHC correspondant à la séquence de VHC contenue dans la protéine de fusion ayant la séquence SEQ ID N° 12, SEQ ID N° 13, SEQ ID N° 14, SEQ ID N° 17 ou SEQ ID N° 18, ladite séquence de VHC dans ledit polypeptide ne formant pas une partie d'une séquence de VHC plus longue, ledit polypeptide étant préparé par des techniques de génie génétique autres que l'expression sous forme de fusions avec un polypeptide codé par le gène de la CMP-KDO synthétase (CKS) de *E. coli*.

14. Analyse faisant intervenir une compétition pour l'identification de la présence d'un anticorps immunologiquement réactif avec un antigène de VHC dans un échantillon liquide, dans laquelle on utilise l'échantillon pour préparer une première et une deuxième aliquotes immunologiquement équivalentes et on met en contact la première aliquote avec un polypeptide lié à un support solide dans des conditions appropriées pour la complexation de l'anticorps avec le polypeptide pour former un complexe anticorps-polypeptide décelable, et on met la deuxième aliquote d'abord en contact avec un polypeptide non lié, puis avec ledit polypeptide lié, le polypeptide étant choisi parmi
une protéine recombinée ayant la séquence SEQ ID N° 12, SEQ ID N° 13, SEQ ID N° 14, SEQ ID N° 17 ou SEQ ID N° 18 et
un polypeptide contenant une séquence de VHC correspondant à la séquence de VHC contenue dans la protéine de fusion ayant la séquence SEQ ID N° 12, SEQ ID N° 13, SEQ ID N° 14, SEQ ID N° 17 ou SEQ ID N° 18, ladite séquence de VHC dans ledit polypeptide ne formant pas une partie d'une séquence de VHC plus longue, ledit polypeptide étant préparé par des techniques de génie génétique autres que l'expression sous forme de fusions avec un polypeptide codé par le gène de la CMP-KDO synthétase (CKS) de *E. coli.*

15. Analyse faisant intervenir une compétition pour l'identification de la présence d'un anticorps immunologiquement réactif avec un antigène de VHC dans un échantillon liquide, dans laquelle on utilise l'échantillon pour préparer une première et une deuxième aliquotes immunologiquement équivalentes et on met en contact la première aliquote avec un polypeptide lié à un support solide dans des conditions appropriées pour la complexation de l'anticorps avec le polypeptide pour former un complexe anticorps-polypeptide décelable, et on met la deuxième aliquote d'abord en contact avec un polypeptide non lié, puis avec ledit polypeptide lié, le polypeptide étant choisi parmi
une protéine recombinée ayant la séquence SEQ ID N° 12, SEQ ID N° 13, SEQ ID N° 14, SEQ ID N° 17 ou SEQ ID N° 18 et
un polypeptide contenant une séquence de VHC correspondant à la séquence de VHC contenue dans la protéine de fusion ayant la séquence SEQ ID N° 12, SEQ ID N° 13, SEQ ID N° 14, SEQ ID N° 17 ou SEQ ID N° 18, ladite séquence de VHC dans ledit polypeptide ne formant pas une partie d'une séquence de VHC plus longue, ledit polypeptide étant préparé par des techniques de génie génétique autres que l'expression sous forme de fusions avec un polypeptide codé par le gène de la CMP-KDO synthétase (CKS) de *E*. *coli,*
la deuxième aliquote étant mise en contact simultanément avec le polypeptide non lié et le polypeptide lié.

16. Analyse faisant intervenir une neutralisation pour l'identification de la présence d'un anticorps immunologiquement réactif avec un antigène de VHC dans un échantillon liquide, dans laquelle on utilise l'échantillon pour préparer une première et une deuxième aliquotes immunologiquement équivalentes et on met en contact la première aliquote avec un polypeptide lié à un support solide dans des conditions appropriées pour la complexation de l'anticorps avec le polypeptide pour former un complexe anticorps-polypeptide décelable, le polypeptide lié étant choisi parmi
une protéine recombinée ayant la séquence SEQ ID N° 12, SEQ ID N° 13, SEQ ID N° 14, SEQ ID N° 17 ou SEQ ID N° 18 et
un polypeptide contenant une séquence de VHC correspondant à la séquence de VHC contenue dans la protéine de fusion ayant la séquence SEQ ID N° 12, SEQ ID N° 13, SEQ ID N° 14, SEQ ID N° 17 ou SEQ ID N° 18, ladite séquence de VHC dans ledit polypeptide ne formant pas une partie d'une séquence de VHC plus longue, ledit polypeptide étant préparé par des techniques de génie génétique autres que l'expression sous forme de fusions avec un polypeptide codé par le gène de la CMP-KDO synthétase (CKS) de *E. coli,*
et on met en contact la deuxième aliquote d'abord avec un polypeptide non lié, puis avec ledit polypeptide lié, le polypeptide non lié étant choisi parmi
une protéine recombinée ayant la séquence SEQ ID N° 12, SEQ ID N° 13, SEQ ID N° 14, SEQ ID N° 17 ou SEQ ID N° 18 et
un polypeptide contenant une séquence de VHC correspondant à la séquence de VHC contenue dans la protéine de fusion ayant la séquence SEQ ID N° 12, SEQ ID N° 13, SEQ ID N° 14, SEQ ID N° 17 ou SEQ ID N° 18, ladite séquence de VHC dans ledit polypeptide ne formant pas une partie d'une séquence de VHC plus longue, ledit polypeptide étant préparé par des techniques de génie génétique autres que l'expression sous forme de fusions avec un polypeptide codé par le gène de la CMP-KDO synthétase (CKS) de *E*. *coli,*
le polypeptide lié choisi n'étant pas le même que le polypeptide non lié choisi.

17. Analyse faisant intervenir une neutralisation pour l'identification de la présence d'un anticorps immunologiquement réactif avec un antigène de VHC dans un échantillon liquide, dans laquelle on utilise l'échantillon pour préparer une première et une deuxième aliquotes immunologiquement équivalentes et on met en contact la première aliquote avec un polypeptide lié à un support solide dans des conditions appropriées pour la complexation de l'anticorps avec le polypeptide pour former un complexe anticorps-polypeptide décelable, le polypeptide lié étant choisi parmi
une protéine recombinée ayant la séquence SEQ ID N° 12, SEQ ID N° 13, SEQ ID N° 14, SEQ ID N° 17 ou SEQ ID N° 18 et
un polypeptide contenant une séquence de VHC correspondant à la séquence de VHC contenue dans la protéine de fusion ayant la séquence SEQ ID N° 12, SEQ ID N° 13, SEQ ID N° 14, SEQ ID N° 17 ou SEQ ID N° 18, ladite séquence de VHC dans ledit polypeptide ne formant pas une partie d'une séquence de VHC plus longue, ledit polypeptide étant préparé par des techniques de génie génétique autres que l'expression sous forme de fusions avec un polypeptide codé par le gène de la CMP-KDO synthétase (CKS) de *E. coli*,
et on met en contact la deuxième aliquote d'abord avec un polypeptide non lié, puis avec ledit polypeptide lié, le polypeptide non lié étant choisi parmi
une protéine recombinée ayant la séquence SEQ ID N° 12, SEQ ID N° 13, SEQ ID N° 14, SEQ ID N° 17 ou SEQ ID N° 18 et
un polypeptide contenant une séquence de VHC correspondant à la séquence de VHC contenue dans la protéine de fusion ayant la séquence SEQ ID N° 12, SEQ ID N° 13, SEQ ID N° 14, SEQ ID N° 17 ou SEQ ID N° 18, ladite séquence de VHC dans ledit polypeptide ne formant pas une partie d'une séquence de VHC plus longue, ledit polypeptide étant préparé par des techniques de génie génétique autres que l'expression sous forme de fusions avec un polypeptide codé par le gène de la CMP-KDO synthétase (CKS) de *E. coli,*
le polypeptide lié choisi n'étant pas le même que le polypeptide non lié choisi,
et la deuxième aliquote étant mise en contact simultanément avec le polypeptide non lié et le polypeptide lié.

18. Trousse d'analyse immunologique, comprenant:
un polypeptide contenant au moins un antigène de VHC choisis parmi
une protéine recombinée ayant la séquence SEQ ID N° 12, SEQ ID N° 13, SEQ ID N° 14, SEQ ID N° 17 ou SEQ ID N° 18 et
un polypeptide contenant une séquence de VHC correspondant à la séquence de VHC contenue dans la protéine de fusion ayant la séquence SEQ ID N° 12, SEQ ID N° 13, SEQ ID N° 14, SEQ ID N° 17 ou SEQ ID N° 18, ladite séquence de VHC dans ledit polypeptide ne formant pas une partie d'une séquence de VHC plus longue, ledit polypeptide étant préparé par des techniques de génie génétique autres que l'expression sous forme de fusions avec un polypeptide codé par le gène de la CMP-KDO synthétase (CKS) de *E. coli*,
un ou plusieurs réactifs de préparation;
et un ou plusieurs réactifs de détection et de production de signaux.

19. Trousse selon la revendication 18, dans laquelle les polypeptides sont liés à un support solide.

## Revendications (Revendications pour l'(les) Etat(s) contractant(s) suivant(s): ES)

1. Analyse destinée à identifier la présence d'un anticorps immunologiquement réactif avec un antigène du VHC dans un échantillon liquide, comprenant la mise en contact de l'échantillon avec au moins un polypeptide choisi parmi
une protéine recombinée ayant la séquence SEQ ID N° 12, SEQ ID N° 13, SEQ ID N° 14, SEQ ID N° 17 ou SEQ ID N° 18 et
un polypeptide contenant une séquence de VHC correspondant à la séquence de VHC contenue dans la protéine de fusion ayant la séquence SEQ ID N° 12, SEQ ID N° 13, SEQ ID N° 14, SEQ ID N° 17 ou SEQ ID N° 18, ladite séquence de VHC dans ledit polypeptide ne formant pas une partie d'une séquence de VHC plus longue, ledit polypeptide étant préparé par des techniques de génie génétique autres que l'expression sous forme de fusions avec un polypeptide codé par le gène de la CMP-KDO synthétase (CKS) de *E. coli*,
dans des conditions appropriées à la complexation de l'anticorps avec le polypeptide; et la détection du complexe anticorps-polypeptide.

2. Analyse de confirmation pour l'identification de la présence d'un anticorps immunologiquement réactif avec un antigène de VHC dans un échantillon liquide, dans lequel on utilise l'échantillon pour préparer une première et une deuxième aliquotes immunologiquement équivalentes et on met en contact la première aliquote avec au moins un polypeptide choisi parmi
une protéine recombinée ayant la séquence SEQ ID N° 12, SEQ ID N° 13, SEQ ID N° 14, SEQ ID N° 17 ou SEQ ID N° 18 et
un polypeptide contenant une séquence de VHC correspondant à la séquence de VHC contenue dans la protéine de fusion ayant la séquence SEQ ID N° 12, SEQ ID N° 13, SEQ ID N° 14, SEQ ID N° 17 ou SEQ ID N° 18, ladite séquence de VHC dans ledit polypeptide ne formant pas une partie d'une séquence de VHC plus longue, ledit polypeptide étant préparé par des techniques de génie génétique autres que l'expression sous forme de fusions avec un polypeptide codé par le gène de la CMP-KDO synthétase (CKS) de *E. coli*,
dans des conditions appropriées pour la complexation de l'anticorps avec le polypeptide et on détecte le premier complexe anticorps-antigène, et
on met en contact la deuxième aliquote avec un polypeptide choisi parmi
une protéine recombinée ayant la séquence SEQ ID N° 12, SEQ ID N° 13, SEQ ID N° 14, SEQ ID N° 17 ou SEQ ID N° 18 et
un polypeptide contenant une séquence de VHC correspondant à la séquence de VHC contenue dans la protéine de fusion ayant la séquence SEQ ID N° 12, SEQ ID N° 13, SEQ ID N° 14, SEQ ID N° 17 ou SEQ ID N° 18, ladite séquence de VHC dans ledit polypeptide ne formant pas une partie d'une séquence de VHC plus longue, ledit polypeptide étant préparé par des techniques de génie génétique autres que l'expression sous forme de fusions avec un polypeptide codé par le gène de la CMP-KDO synthétase (CKS) de *E. coli,*
dans des conditions appropriées pour la formation d'un deuxième complexe anticorps-antigène et on détecte le deuxième complexe anticorps-antigène, le polypeptide choisi pour la première aliquote n'étant pas le même que celui choisi pour la deuxième aliquote.

3. Analyse par immunoempreinte pour l'identification de la présence d'un anticorps immunologiquement réactif avcc un antigène de VHC dans un échantillon liquide, dans laquelle on met l'échantillon en contact simultanément avec au moins deux polypeptides liés séparément à des régions distinctes du support solide, contenant chacun des épitopes distincts d'un antigène de VHC, dans des conditions appropriées pour la complexation de l'anticorps avec le polypeptide; et on détecte complexe anticorps-polypeptide, lesdits polypeptides étant choisis parmi
une protéine recombinée ayant la séquence SEQ ID N° 12, SEQ ID N° 13, SEQ ID N° 14, SEQ ID N° 17 ou SEQ ID N° 18 et
un polypeptide contenant une séquence de VHC correspondant à la séquence de VHC contenue dans la protéine de fusion ayant la séquence SEQ ID N° 12, SEQ ID N° 13, SEQ ID N° 14, SEQ ID N° 17 ou SEQ ID N° 18, ladite séquence de VHC dans ledit polypeptide ne formant pas une partie d'une séquence de VHC plus longue, ledit polypeptide étant préparé par des techniques de génie génétique autres que l'expression sous forme de fusions avec un polypeptide codé par le gène de la CMP-KDO synthétase (CKS) de *E*. *coli.*

4. Analyse faisant intervenir une compétition pour l'identification de la présence d'un anticorps immunologiquement réactif avec un antigène de VHC dans un échantillon liquide, dans laquelle on utilise l'échantillon pour préparer une première et une deuxième aliquotes immunologiquement équivalentes et on met en contact la première aliquote avec un polypeptide lié à un support solide dans des conditions appropriées pour la complexation de l'anticorps avec le polypeptide pour former un complexe anticorps-polypeptide décelable, et on met la deuxième aliquote d'abord en contact avec un polypeptide non lié, puis avec ledit polypeptide lié, le polypeptide étant choisi parmi
une protéine recombinée ayant la séquence SEQ ID N° 12, SEQ ID N° 13, SEQ ID N° 14, SEQ ID N° 17 ou SEQ ID N° 18 et
un polypeptide contenant une séquence de VHC correspondant à la séquence de VHC contenue dans la protéine de fusion ayant la séquence SEQ ID N° 12, SEQ ID N° 13, SEQ ID N° 14, SEQ ID N° 17 ou SEQ ID N° 18, ladite séquence de VHC dans ledit polypeptide ne formant pas une partie d'une séquence de VHC plus longue, ledit polypeptide étant préparé par des techniques de génie génétique autres que l'expression sous forme de fusions avec un polypeptide codé par le gène de la CMP-KDO synthétase (CKS) de *E. coli*.

5. Analyse faisant intervenir une compétition pour l'identification de la présence d'un anticorps immunologiquement réactif avec un antigène de VHC dans un échantillon liquide, dans laquelle on utilise l'échantillon pour préparer une première et une deuxième aliquotes immunologiquement équivalentes et on met en contact la première aliquote avec un polypeptide lié à un support solide dans des conditions appropriées pour la complexation de l'anticorps avec le polypeptide pour former un complexe anticorps-polypeptide décelable, et on met là deuxième aliquote d'abord en contact avec un polypeptide non lié, puis avec ledit polypeptide lié, le polypeptide étant choisi parmi
une protéine recombinée ayant la séquence SEQ ID N° 12, SEQ ID N° 13, SEQ ID N° 14, SEQ ID N° 17 ou SEQ ID N° 18 et
un polypeptide contenant une séquence de VHC correspondant à la séquence de VHC contenue dans la protéine de fusion ayant la séquence SEQ ID N° 12, SEQ ID N° 13, SEQ ID N° 14, SEQ ID N° 17 ou SEQ ID N° 18, ladite séquence de VHC dans ledit polypeptide ne formant pas une partie d'une séquence de VHC plus longue, ledit polypeptide étant préparé par des techniques de génie génétique autres que l'expression sous forme de fusions avec un polypeptide codé par le gène de la CMP-KDO synthétase (CKS) de *E. coli,*
la deuxième aliquote étant mise en contact simultanément avec le polypeptide non lié et le polypeptide lié.

6. Analyse faisant intervenir une neutralisation pour l'identification de la présence d'un anticorps immunologiquement réactif avec un antigène de VHC dans un échantillon liquide, dans laquelle on utilise l'échantillon pour préparer une première et une deuxième aliquotes immunologiquement équivalentes et on met en contact la première aliquote avec un polypeptide lié à un support solide dans des conditions appropriées pour la complexation de l'anticorps avec le polypeptide pour former un complexe anticorps-polypeptide décelable, le polypeptide lié étant choisi parmi
une protéine recombinée ayant la séquence SEQ ID N° 12, SEQ ID N° 13, SEQ ID N° 14, SEQ ID N° 17 ou SEQ ID N° 18 et
un polypeptide contenant une séquence de VHC correspondant à la séquence de VHC contenue dans la protéine de fusion ayant la séquence SEQ ID N° 12, SEQ ID N° 13, SEQ ID N° 14, SEQ ID N° 17 ou SEQ ID N° 18, ladite séquence de VHC dans ledit polypeptide ne formant pas une partie d'une séquence de VHC plus longue, ledit polypeptide étant préparé par des techniques de génie génétique autres que l'expression sous forme de fusions avec un polypeptide codé par le gène de la CMP-KDO synthétase (CKS) de *E. coli,*
et on met en contact la deuxième aliquote d'abord avec un polypeptide non lié, puis avec ledit polypeptide lié, le polypeptide non lié étant choisi parmi
une protéine recombinée ayant la séquence SEQ ID N° 12, SEQ ID N° 13, SEQ ID N° 14, SEQ ID N° 17 ou SEQ ID N° 18 et
un polypeptide contenant une séquence de VHC correspondant à la séquence de VHC contenue dans la protéine de fusion ayant la séquence SEQ ID N° 12, SEQ ID N° 13, SEQ ID N° 14, SEQ ID N° 17 ou SEQ ID N° 18, ladite séquence de VIIC dans ledit polypeptide ne formant pas une partie d'une séquence de VHC plus longue, ledit polypeptide étant préparé par des techniques de génie génétique autres que l'expression sous forme de fusions avec un polypeptide codé par le gène de la CMP-KDO synthétase (CKS) de *E. coli,*
le polypeptide lié choisi n'étant pas le même que le polypeptide non lié choisi.

7. Analyse faisant intervenir une neutralisation pour l'identification de la présence d'un anticorps immunologiquement réactif avec un antigène de VHC dans un échantillon liquide, dans laquelle on utilise l'échantillon pour préparer une première et une deuxième aliquotes immunologiquement équivalentes et on met en contact la première aliquote avec un polypeptide lié à un support solide dans des conditions appropriées pour la complexation de l'anticorps avec le polypeptide pour former un complexe anticorps-polypeptide décelable, le polypeptide lié étant choisi parmi
une protéine recombinée ayant la séquence SEQ ID N° 12, SEQ ID N° 13, SEQ ID N° 14, SEQ ID N° 17 ou SEQ ID N° 18 et
un polypeptide contenant une séquence de VHC correspondant à la séquence de VHC contenue dans la protéine de fusion ayant la séquence SEQ ID N° 12, SEQ ID N° 13, SEQ ID N° 14, SEQ ID N° 17 ou SEQ ID N° 18, ladite séquence de VHC dans ledit polypeptide ne formant pas une partie d'une séquence de VHC plus longue, ledit polypeptide étant prépare par des techniques de génie génétique autres que l'expression sous forme de fusions avec un polypeptide codé par le gène de la CMP-KDO synthétase (CKS) de *E. coli,*
et on met en contact la deuxième aliquote d'abord avec un polypeptide non lié, puis avec ledit polypeptide lié, le polypeptide non lié étant choisi parmi
une protéine recombinée ayant la séquence SEQ ID N° 12, SEQ ID N° 13, SEQ ID N° 14, SEQ ID N° 17 ou SEQ ID N° 18 et
un polypeptide contenant une séquence de VHC correspondant à la séquence de VHC contenue dans la protéine de fusion ayant la séquence SEQ ID N° 12, SEQ ID N° 13, SEQ ID N° 14, SEQ ID N° 17 ou SEQ ID N° 18, ladite séquence de VHC dans ledit polypeptide ne formant pas une partie d'une séquence de VHC plus longue, ledit polypeptide étant preparé par des techniques de génie génétique autres que l'expression sous forme de fusions avec un polypeptide codé par le gène de la CMP-KDO synthétase (CKS) de *E. coli,*
le polypeptide lié choisi n'étant pas le même quc le polypeptide non lié choisi,
et la deuxième aliquote étant mise en contact simultanément avec le polypeptide non lié et le polypeptide lié.

8. Trousse d'analyse immunologique, comprenant:
un polypeptide contenant au moins un antigène de VHC choisis parmi
une protéine recombinée ayant la séquence SEQ ID N° 12, SEQ ID N° 13, SEQ ID N° 14, SEQ ID N° 17 ou SEQ ID N° 18 et
un polypeptide contenant une séquence de VHC correspondant à la séquence de VHC contenue dans la protéine de fusion ayant la séquence SEQ ID N° 12, SEQ ID N° 13, SEQ ID N° 14, SEQ ID N° 17 ou SEQ ID N° 18, ladite séquence de VHC dans ledit polypeptide ne formant pas une partie d'une séquence de VHC plus longue, ledit polypeptide étant préparé par des techniques de génie génétique autres que l'expression sous forme de fusions avec un polypeptide codé par le gène de la CMP-KDO synthétase (CKS) de *E. coli,*
un ou plusieurs réactifs de préparation;
et un ou plusieurs réactifs de détection et de production de signaux.

9. Trousse selon la revendication 8, dans laquelle les polypeptides sont liés à un support solide.

10. Utilisation d'unc trousse d'analyse immunologique, comprenant :
un polypeptidc contenant au moins un antigène de VHC choisis parmi
une protéine recombinée ayant la séquence SEQ ID N° 12, SEQ ID N° 13, SEQ ID N° 14, SEQ ID N° 17 ou SEQ ID N° 18 et
un polypeptide contenant une séquence de VHC correspondant à la séquence de VHC contenue dans la protéine de fusion ayant la séquence SEQ ID N° 12, SEQ ID N° 13, SEQ ID N° 14, SEQ ID N° 17 ou SEQ ID N° 18, ladite séquence de VHC dans ledit polypeptide ne formant pas une partie d'une séquence de VHC plus longue, ledit polypeptide étant préparé par des techniques de génie génétique autres que l'expression sous forme de fusions avec un polypeptide codé par le gène de la CMP-KDO synthétase (CKS) de *E. Coli* ;
un ou plusieurs réactifs de préparation ;
et un ou plusieurs réactifs de détection et de production de signaux, pour détecter la présence d'un anticorps dirigé contre un antigène de VHC dans un échantillon.

11. Utilisation selon la revendication 10, dans laquelle les polypeptides sont liés à un support solide.


### SEQUENCE LISTING

### (1) GENERAL INFORMATION:

(i) APPLICANT:
   DESAI, SURESH M.
   CASEY, JAMES M.
   RUPPRECHT, KEVIN R.
   DEVARE, SUSHIL G.
(ii) TITLE OF INVENTION: HEPATITIS C ASSAY UTILIZING RECOMBINANT ANTIGENS TO C-100 REGION
(iii) NUMBER OF SEQUENCES: 25
(iv) CORRESPONDENCE ADDRESS:
   (A) ADDRESSEE: ABBOTT LABORATORIES CHAD377/AP6D
   (B) STREET: ONE ABBOTT PARK ROAD
   (C) CITY: ABBOTT PARK
   (D) STATE: ILLINOIS
   (E) COUNTRY: USA
   (F) ZIP: 60064-3500
(v) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE Patentln Release #1.0, Version #1.25
(vi) CURRENT APPLICATION DATA:
   (A) APPLICATION NUMBER: US 07/748,566
   (B) RUNG DATE: 21-AUG-1991
   (C) CLASSIFICATION:
(viii) ATTORNEY/AGENT INFORMATION:
   (A) NAME: POREMBSKI, PRISCILLA E,
   (B) REGISTRATION NUMBER: 33,207
   (C) REFERENCE/DOCKET NUMBER: 4834.PC.04
(ix) TELECOMMUNICATION INFORMATION:
   (A) TELEPHONE: 708-937-6365
   (B) TELEFAX: 708-937-9556

### (2) INFORMATION FOR SEQ ID NO:1:

(i) SEQUENCE CHARACTERISTICS:
   (A) LENGTH: 342 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:1:
   Met Ser Phe Val Val Ile Ile Pro Ala Arg Tyr Ala Ser Thr Arg Leu
   1 5 10 15
   Pro Gly Lys Pro Leu Val Asp Ile Asn Gly Lys Pro Met Ile Val His
   20 25 30
   Val Leu Glu Arg Ala Arg Glu Ser Gly Ala Glu Arg Ile Ile Val Ala
   35 40 45
   Thr Asp His Glu Asp Val Ala Arg Ala Val Glu Ala Ala Gly Gly Glu
   50 55 60
   Val Cys Met Thr Arg Ala Asp His Gln Ser Gly Thr Glu Arg Leu Ala
   65 70 75 80
   Glu Val Val Glu Lys Cys Ala Phe Ser Asp Asp Thr Val Ile Val Asn
   85 90 95
   Val Gln Gly Asp Glu Pro Met Ile Pro Ala Thr Ile Ile Arg Gln Val
   100 105 110
   Ala Asp Asn Leu Ala Gln Arg Gln Val Gly Met Ala Thr Leu Ala Val
   115 120 125
   Pro Ile His Asn Ala Glu Glu Ala Phe Asn Pro Asn Ala Val Lys Val
   130 135 140
   Val Leu Asp Ala Glu Gly Tyr Ala Leu Tyr Phe Ser Arg Ala Thr Ile
   145 150 155 160
   Pro Trp Asp Arg Asp Arg Phe Ala Glu Gly Leu Glu Thr Val Gly Asp
   165 170 175
   Asn Phe Leu Arg His Leu Gly Ile Tyr Gly Tyr Arg Ala Gly Phe Ile
   180 185 190
   Arg Arg Tyr Val Asn Trp Gln Pro Ser Pro Leu Glu His Ile Glu Met
   195 200 205
   Leu Glu Gln Leu Arg Val Leu Trp Tyr Gly Glu Lys Ile His Val Ala
   210 215 220
   Val Ala Gln Glu Val Pro Gly Thr Gly Val Asp Thr Pro Glu Asp Leu
   225 230 235 240
   Asp Pro Ser Thr Asn Ser Met Asp Ala His Phe Leu Ser Gln Ala Pro
   245 250 255
   Pro Pro Ser Trp Asp Gln Met Trp Lys Cys Leu Ile Arg Leu Lys Pro
   260 265 270
   Thr Leu His Gly Pro Thr Pro Leu Leu Tyr Arg Leu Gly Ala Val Gln
   275 280 285
   Asn Glu Ile Thr Leu Thr His Pro Val Thr Lys Tyr Ile Met Thr Cys
   290 295 300
   Met Ser Ala Asp Leu Glu Val Val Thr Ser Thr Trp Val Leu Val Gly
   305 310 315 320
   Gly Val Leu Ala Ala Leu Ala Ala Tyr Cys Leu Ser Thr Asp Pro Leu
   325 330 335
   Asp Cys Arg His Ala Lys
   340

### (2) INFORMATION FOR SEQ ID NO:2:

(i) SEQUENCE CHARACTERISTICS:
   (A) LENGTH: 344 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:2:
   Met Ser Phe Val Val Ile Ile Pro Ala Arg Tyr Ala Ser Thr Arg Leu
   1 5 10 15
   Pro Gly Lys Pro Leu Val Asp Ile Asn Gly Lys Pro Met Ile Val His
   20 25 30
   Val Leu Glu Arg Ala Arg Glu Ser Gly Ala Glu Arg Ile Ile Val Ala
   35 40 45
   Thr Asp His Glu Asp Val Ala Arg Ala Val Glu Ala Ala Gly Gly Glu
   50 55 60
   Val Cys Met Thr Arg Ala Asp His Gln Ser Gly Thr Glu Arg Leu Ala
   65 70 75 80
   Glu Val Val Glu Lys Cys Ala Phe Ser Asp Asp Thr Val Ile Val Asn
   85 90 95
   Val Gln Gly Asp Glu Pro Met Ile Pro Ala Thr Ile Ile Arg Gln Val
   100 105 110
   Ala Asp Asn Leu Ala Gln Arg Gln Val Gly Met Ala Thr Leu Ala Val
   115 120 125
   Pro Ile His Asn Ala Glu Glu Ala Phe Asn Pro Asn Ala Val Lys Val
   130 135 140
   Val Leu Asp Ala Glu Gly Tyr Ala Leu Tyr Phe Ser Arg Ala Thr Ile
   145 150 155 160
   Pro Trp Asp Arg Asp Arg Phe Ala Glu Gly Leu Glu Thr Val Gly Asp
   165 170 175
   Asn Phe Leu Arg His Leu Gly Ile Tyr Gly Tyr Arg Ala Gly Phe Ile
   180 185 190
   Arg Arg Tyr Val Asn Trp Gln Pro Ser Pro Leu Glu His Ile Glu Met
   195 200 205
   Leu Glu Gln Leu Arg Val Leu Trp Tyr Gly Glu Lys Ile His Val Ala
   210 215 220
   Val Ala Gln Glu Val Pro Gly Thr Gly Val Asp Thr Pro Glu Asp Leu
   225 230 235 240
   Asp Pro Ser Thr Asn Ser Met Asp Ala His Phe Leu Ser Gln Thr Lys
   245 250 255
   Gln Ser Gly Glu Asn Leu Pro Tyr Leu Val Ala Tyr Gln Ala Thr Val
   260 265 270
   Cys Ala Arg Ala Gln Ala Pro Thr Pro Leu Leu Tyr Arg Leu Gly Ala
   275 280 285
   Val Gln Asn Glu Ile Thr Leu Thr His Pro Val Thr Lys Tyr Ile Met
   290 295 300
   Thr Cys Met Ser Ala Asp Leu Glu Val Val Thr Ser Thr Trp Val Leu
   305 310 315 320
   Val Gly Gly Val Leu Ala Ala Leu Ala Ala Tyr Cys Leu Ser Thr Asp
   325 330 335
   Pro Leu Asp Cys Arg His Ala Lys
   340

### (2) INFORMATION FOR SEQ ID NO:3:

(i) SEQUENCE CHARACTERISTICS:
   (A) LENGTH: 352 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:3:
   Met Ser Phe Val Val Ile Ile Pro Ala Arg Tyr Ala Ser Thr Arg Leu
   1 5 10 15
   Pro Gly Lys Pro Leu Val Asp Ile Asn Gly Lys Pro Met Ile Val His
   20 25 30
   Val Leu Glu Arg Ala Arg Glu Ser Gly Ala Glu Arg Ile Ile Val Ala
   35 40 45
   Thr Asp His Glu Asp Val Ala Arg Ala Val Glu Ala Ala Gly Gly Glu
   50 55 60
   Val Cys Met Thr Arg Ala Asp His Gln Ser Gly Thr Glu Arg Leu Ala
   65 70 75 80
   Glu Val Val Glu Lys Cys Ala Phe Ser Asp Asp Thr Val Ile Val Asn
   85 90 95
   Val Gln Gly Asp Glu Pro Met Ile Pro Ala Thr Ile Ile Arg Gln Val
   100 105 110
   Ala Asp Asn Leu Ala Gln Arg Gln Val Gly Met Thr Thr Leu Ala Val
   115 120 125
   Pro Ile His Asn Ala Glu Glu Ala Phe Asn Pro Asn Ala Val Lys Val
   130 135 140
   Val Leu Asp Ala Glu Gly Tyr Ala Leu Tyr Phe Ser Arg Ala Thr Ile
   145 150 155 160
   Pro Trp Asp Arg Asp Arg Phe Ala Glu Gly Leu Glu Thr Val Gly Asp
   165 170 175
   Asn Phe Leu Arg His Leu Gly Ile Tyr Gly Tyr Arg Ala Gly Phe Ile
   180 185 190
   Arg Arg Tyr Val Asn Trp Gln Pro Ser Pro Leu Glu His Ile Glu Met
   195 200 205
   Leu Glu Gln Leu Arg Val Leu Trp Tyr Gly Glu Lys Ile His Val Ala
   210 215 220
   Val Ala Gln Glu Val Pro Gly Thr Gly Val Asp Thr Pro Glu Asp Leu
   225 230 235 240
   Asp Pro Ser Thr Asn Ser Met Asp Ala His Phe Leu Ser Gln Thr Lys
   245 250 255
   Gln Ser Gly Glu Asn Leu Pro Tyr Leu Val Ala Tyr Gln Ala Thr Val
   260 265 270
   Cys Ala Arg Ala Gln Ala Arg Leu Lys Pro Thr Leu His Gly Pro Thr
   275 280 285
   Pro Leu Leu Tyr Arg Leu Gly Ala Val Gln Asn Glu Ile Thr Leu Thr
   290 295 300
   His Pro Val Thr Lys Tyr Ile Met Thr Cys Met Ser Ala Asp Leu Glu
   305 310 315 320
   Val Val Thr Ser Thr Trp Val Leu Val Gly Gly Val Leu Ala Ala Leu
   325 330 335
   Ala Ala Tyr Cys Leu Ser Thr Asp Pro Leu Asp Cys Arg His Ala Lys
   340 345 350

### (2) INFORMATION FOR SEQ ID NO:4:

(i) SEQUENCE CHARACTERISTICS:
   (A) LENGTH: 357 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:4:
   Met Ser Phe Val Val Ile Ile Pro Ala Arg Tyr Ala Ser Thr Arg Leu
   1 5 10 15
   Pro Gly Lys Pro Leu Val Asp Ile Asn Gly Lys Pro Met Ile Val His
   20 25 30
   Val Leu Glu Arg Ala Arg Glu Ser Gly Ala Glu Arg Ile Ile Val Ala
   35 40 45
   Thr Asp His Glu Asp Val Ala Arg Ala Val Glu Ala Ala Gly Gly Glu
   50 55 60
   Val Cys Met Thr Arg Ala Asp His Gln Ser Gly Thr Glu Arg Leu Ala
   65 70 75 80
   Glu Val Val Glu Lys Cys Ala Phe Ser Asp Asp Thr Val Ile Val Asn
   85 90 95
   Val Gln Gly Asp Glu Pro Met Ile Pro Ala Thr Ile Ile Arg Gln Val
   100 105 110
   Ala Asp Asn Leu Ala Gln Arg Gln Val Gly Met Thr Thr Leu Ala Val
   115 120 125
   Pro Ile His Asn Ala Glu Glu Ala Phe Asn Pro Asn Ala Val Lys Val
   130 135 140
   Val Leu Asp Ala Glu Gly Tyr Ala Leu Tyr Phe Ser Arg Ala Thr Ile
   145 150 155 160
   Pro Trp Asp Arg Asp Arg Phe Ala Glu Gly Leu Glu Thr Val Gly Asp
   165 170 175
   Asn Phe Leu Arg His Leu Gly Ile Tyr Gly Tyr Arg Ala Gly Phe Ile
   180 185 190
   Arg Arg Tyr Val Asn Trp Gln Pro Ser Pro Leu Glu His Ile Glu Met
   195 200 205
   Leu Glu Gln Leu Arg Val Leu Trp Tyr Gly Glu Lys Ile His Val Ala
   210 215 220
   Val Ala Gln Glu Val Pro Gly Thr Gly Val Asp Thr Pro Glu Asp Leu
   225 230 235 240
   Asp Pro Ser Thr Asn Ser Met Asp Ala His Phe Leu Ser Gln Thr Lys
   245 250 255
   Gln Ser Gly Glu Asn Leu Pro Tyr Leu Val Ala Tyr Gln Ala Thr Val
   260 265 270
   Cys Ala Arg Ala Gln Ala Trp Lys Cys Leu Ile Arg Leu Lys Pro Thr
   275 280 285
   Leu His Gly Pro Thr Pro Leu Leu Tyr Arg Leu Gly Ala Val Gln Asn
   290 295 300
   Glu Ile Thr Leu Thr His Pro Val Thr Lys Tyr Ile Met Thr Cys Met
   305 310 315 320
   Ser Ala Asp Leu Glu Val Val Thr Ser Thr Trp Val Leu Val Gly Gly
   325 330 335
   Val Leu Ala Ala Leu Ala Ala Tyr Cys Leu Ser Thr Asp Pro Leu Asp
   340 345 350
   Cys Arg His Ala Lys
   355

### (2) INFORMATION FOR SEQ ID NO:5:

(i) SEQUENCE CHARACTERISTICS:
   (A) LENGTH: 362 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:5:
   Met Ser Phe Val Val Ile Ile Pro Ala Arg Tyr Ala Ser Thr Arg Leu
   1 5 10 15
   Pro Gly Lys Pro Leu Val Asp Ile Asn Gly Lys Pro Met Ile Val His
   20 25 30
   Val Leu Glu Arg Ala Arg Glu Ser Gly Ala Glu Arg Ile Ile Val Ala
   35 40 45
   Thr Asp His Glu Asp Val Ala Arg Ala Val Glu Ala Ala Gly Gly Glu
   50 55 60
   Val Cys Met Thr Arg Ala Asp His Gln Ser Gly Thr Glu Arg Leu Ala
   65 70 75 80
   Glu Val Val Glu Lys Cys Ala Phe Ser Asp Asp Thr Val Ile Val Asn
   85 90 95
   Val Gln Gly Asp Glu Pro Met Ile Pro Ala Thr Ile Ile Arg Gln Val
   100 105 110
   Ala Asp Asn Leu Ala Gln Arg Gln Val Gly Met Thr Thr Leu Ala Val
   115 120 125
   Pro Ile His Asn Ala Glu Glu Ala Phe Asn Pro Asn Ala Val Lys Val
   130 135 140
   Val Leu Asp Ala Glu Gly Tyr Ala Leu Tyr Phe Ser Arg Ala Thr Ile
   145 150 155 160
   Pro Trp Asp Arg Asp Arg Phe Ala Glu Gly Leu Glu Thr Val Gly Asp
   165 170 175
   Asn Phe Leu Arg His Leu Gly Ile Tyr Gly Tyr Arg Ala Gly Phe Ile
   180 185 190
   Arg Arg Tyr Val Asn Trp Gln Pro Ser Pro Leu Glu His Ile Glu Met
   195 200 205
   Leu Glu Gln Leu Arg Val Leu Trp Tyr Gly Glu Lys Ile His Val Ala
   210 215 220
   Val Ala Gln Glu Val Pro Gly Thr Gly Val Asp Thr Pro Glu Asp Leu
   225 230 235 240
   Asp Pro Ser Thr Asn Ser Met Asp Ala His Phe Leu Ser Gln Thr Lys
   245 250 255
   Gln Ser Gly Glu Asn Leu Pro Tyr Leu Val Ala Tyr Gln Ala Thr Val
   260 265 270
   Cys Ala Arg Ala Gln Ala Ser Trp Asp Gln Met Trp Lys Cys Leu Ile
   275 280 285
   Arg Leu Lys Pro Thr Leu His Gly Pro Thr Pro Leu Leu Tyr Arg Leu
   290 295 300
   Gly Ala Val Gln Asn Glu Ile Thr Leu Thr His Pro Val Thr Lys Tyr
   305 310 315 320
   Ile Met Thr Cys Met Ser Ala Asp Leu Glu Val Val Thr Ser Thr Trp
   325 330 335
   Val Leu Val Gly Gly Val Leu Ala Ala Leu Ala Ala Tyr Cys Leu Ser
   340 345 350
   Thr Asp Pro Leu Asp Cys Arg His Ala Lys
   355 360

### (2) INFORMATION FOR SEQ ID NO:6:

(i) SEQUENCE CHARACTERISTICS:
   (A) LENGTH: 365 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:6:
   Met Ser Phe Val Val Ile Ile Pro Ala Arg Tyr Ala Ser Thr Arg Leu
   1 5 10 15
   Pro Gly Lys Pro Leu Val Asp Ile Asn Gly Lys Pro Met Ile Val His
   20 25 30
   Val Leu Glu Arg Ala Arg Glu Ser Gly Ala Glu Arg Ile Ile Val Ala
   35 40 45
   Thr Asp His Glu Asp Val Ala Arg Ala Val Glu Ala Ala Gly Gly Glu
   50 55 60
   Val Cys Met Thr Arg Ala Asp His Gln Ser Gly Thr Glu Arg Leu Ala
   65 70 75 80
   Glu Val Val Glu Lys Cys Ala Phe Ser Asp Asp Thr Val Ile Val Asn
   85 90 95
   Val Gln Gly Asp Glu Pro Met Ile Pro Ala Thr Ile Ile Arg Gln Val
   100 105 110
   Ala Asp Asn Leu Ala Gln Arg Gln Val Gly Met Thr Thr Leu Ala Val
   115 120 125
   Pro Ile His Asn Ala Glu Glu Ala Phe Asn Pro Asn Ala Val Lys Val
   130 135 140
   Val Leu Asp Ala Glu Gly Tyr Ala Leu Tyr Phe Ser Arg Ala Thr Ile
   145 150 155 160
   Pro Trp Asp Arg Asp Arg Phe Ala Glu Gly Leu Glu Thr Val Gly Asp
   165 170 175
   Asn Phe Leu Arg His Leu Gly Ile Tyr Gly Tyr Arg Ala Gly Phe Ile
   180 185 190
   Arg Arg Tyr Val Asn Trp Gln Pro Ser Pro Leu Glu His Ile Glu Met
   195 200 205
   Leu Glu Gln Leu Arg Val Leu Trp Tyr Gly Glu Lys Ile His Val Ala
   210 215 220
   Val Ala Gln Glu Val Pro Gly Thr Gly Val Asp Thr Pro Glu Asp Leu
   225 230 235 240
   Asp Pro Ser Thr Asn Ser Met Asp Ala His Phe Leu Ser Gln Thr Lys
   245 250 255
   Gln Ser Gly Glu Asn Leu Pro Tyr Leu Val Ala Tyr Gln Ala Thr Val
   260 265 270
   Cys Ala Arg Ala Gln Ala Pro Pro Pro Ser Trp Asp Gln Met Trp Lys
   275 280 285
   Cys Leu Ile Arg Leu Lys Pro Thr Leu His Gly Pro Thr Pro Leu Leu
   290 295 300
   Tyr Arg Leu Gly Ala Val Gln Asn Glu Ile Thr Leu Thr His Pro Val
   305 310 315 320
   Thr Lys Tyr Ile Met Thr Cys Met Ser Ala Asp Leu Glu Val Val Thr
   325 330 335
   Ser Thr Trp Val Leu Val Gly Gly Val Leu Ala Ala Leu Ala Ala Tyr
   340 345 350
   Cys Leu Ser Thr Asp Pro Leu Asp Cys Arg His Ala Lys
   355 360 365

### (2) INFORMATION FOR SEQ ID NO:7:

(i) SEQUENCE CHARACTERISTICS:
   (A) LENGTH: 363 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:7:
   Met Ser Phe Val Val Ile Ile Pro Ala Arg Tyr Ala Ser Thr Arg Leu
   1 5 10 15
   Pro Gly Lys Pro Leu Val Asp Ile Asn Gly Lys Pro Met Ile Val His
   20 25 30
   Val Leu Glu Arg Ala Arg Glu Ser Gly Ala Glu Arg Ile Ile Val Ala
   35 40 45
   Thr Asp His Glu Asp Val Ala Arg Ala Val Glu Ala Ala Gly Gly Glu
   50 55 60
   Val Cys Met Thr Arg Ala Asp His Gln Ser Gly Thr Glu Arg Leu Ala
   65 70 75 80
   Glu Val Val Glu Lys Cys Ala Phe Ser Asp Asp Thr Val Ile Val Asn
   85 90 95
   Val Gln Gly Asp Glu Pro Met Ile Pro Ala Thr Ile Ile Arg Gln Val
   100 105 110
   Ala Asp Asn Leu Ala Gln Arg Gln Val Gly Met Thr Thr Leu Ala Val
   115 120 125
   Pro Ile His Asn Ala Glu Glu Ala Phe Asn Pro Asn Ala Val Lys Val
   130 135 140
   Val Leu Asp Ala Glu Gly Tyr Ala Leu Tyr Phe Ser Arg Ala Thr Ile
   145 150 155 160
   Pro Trp Asp Arg Asp Arg Phe Ala Glu Gly Leu Glu Thr Val Gly Asp
   165 170 175
   Asn Phe Leu Arg His Leu Gly Ile Tyr Gly Tyr Arg Ala Gly Phe Ile
   180 185 190
   Arg Arg Tyr Val Asn Trp Gln Pro Ser Pro Leu Glu His Ile Glu Met
   195 200 205
   Leu Glu Gln Leu Arg Val Leu Trp Tyr Gly Glu Lys Ile His Val Ala
   210 215 220
   Val Ala Gln Glu Val Pro Gly Thr Gly Val Asp Thr Pro Glu Asp Leu
   225 230 235 240
   Asp Pro Ser Thr Asn Ser Met Asp Ala His Phe Leu Ser Gln Thr Lys
   245 250 255
   Gln Ser Gly Glu Asn Leu Pro Tyr Leu Val Ala Tyr Gln Ala Thr Val
   260 265 270
   Cys Ala Arg Ala Gln Ala Pro Ser Trp Asp Gln Met Trp Lys Cys Leu
   275 280 285
   Ile Arg Leu Lys Pro Thr Leu His Gly Pro Thr Pro Leu Leu Tyr Arg
   290 295 300
   Leu Gly Ala Val Gln Asn Glu Ile Thr Leu Thr His Pro Val Thr Lys
   305 310 315 320
   Tyr Ile Met Thr Cys Met Ser Ala Asp Leu Glu Val Val Thr Ser Thr
   325 330 335
   Trp Val Leu Val Gly Gly Val Leu Ala Ala Leu Ala Ala Tyr Cys Leu
   340 345 350
   Ser Thr Asp Pro Leu Asp Cys Arg His Ala Lys
   355 360

### (2) INFORMATION FOR SEQ ID NO:8:

(i) SEQUENCE CHARACTERISTICS:
   (A) LENGTH: 364 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:8:
   Met Ser Phe Val Val Ile Ile Pro Ala Arg Tyr Ala Ser Thr Arg Leu
   1 5 10 15
   Pro Gly Lys Pro Leu Val Asp Ile Asn Gly Lys Pro Met Ile Val His
   20 25 30
   Val Leu Glu Arg Ala Arg Glu Ser Gly Ala Glu Arg Ile Ile Val Ala
   35 40 45
   Thr Asp His Glu Asp Val Ala Arg Ala Val Glu Ala Ala Gly Gly Glu
   50 55 60
   Val Cys Met Thr Arg Ala Asp His Gln Ser Gly Thr Glu Arg Leu Ala
   65 70 75 80
   Glu Val Val Glu Lys Cys Ala Phe Ser Asp Asp Thr Val Ile Val Asn
   85 90 95
   Val Gln Gly Asp Glu Pro Met Ile Pro Ala Thr Ile Ile Arg Gln Val
   100 105 110
   Ala Asp Asn Leu Ala Gln Arg Gln Val Gly Met Thr Thr Leu Ala Val
   115 120 125
   Pro Ile His Asn Ala Glu Glu Ala Phe Asn Pro Asn Ala Val Lys Val
   130 135 140
   Val Leu Asp Ala Glu Gly Tyr Ala Leu Tyr Phe Ser Arg Ala Thr Ile
   145 150 155 160
   Pro Trp Asp Arg Asp Arg Phe Ala Glu Gly Leu Glu Thr Val Gly Asp
   165 170 175
   Asn Phe Leu Arg His Leu Gly Ile Tyr Gly Tyr Arg Ala Gly Phe Ile
   180 185 190
   Arg Arg Tyr Val Asn Trp Gln Pro Ser Pro Leu Glu His Ile Glu Met
   195 200 205
   Leu Glu Gln Leu Arg Val Leu Trp Tyr Gly Glu Lys Ile His Val Ala
   210 215 220
   Val Ala Gln Glu Val Pro Gly Thr Gly Val Asp Thr Pro Glu Asp Leu
   225 230 235 240
   Asp Pro Ser Thr Asn Ser Met Asp Ala His Phe Leu Ser Gln Thr Lys
   245 250 255
   Gln Ser Gly Glu Asn Leu Pro Tyr Leu Val Ala Tyr Gln Ala Thr Val
   260 265 270
   Cys Ala Arg Ala Gln Ala Pro Pro Ser Trp Asp Gln Met Trp Lys Cys
   275 280 285
   Leu Ile Arg Leu Lys Pro Thr Leu His Gly Pro Thr Pro Leu Leu Tyr
   290 295 300
   Arg Leu Gly Ala Val Gln Asn Glu Ile Thr Leu Thr His Pro Val Thr
   305 310 315 320
   Lys Tyr Ile Met Thr Cys Met Ser Ala Asp Leu Glu Val Val Thr Ser
   325 330 335
   Thr Trp Val Leu Val Gly Gly Val Leu Ala Ala Leu Ala Ala Tyr Cys
   340 345 350
   Leu Ser Thr Asp Pro Leu Asp Cys Arg His Ala Lys
   355 360

### (2) INFORMATION FOR SEQ ID NO:9:

(i) SEQUENCE CHARACTERISTICS:
   (A) LENGTH: 10 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:9:
   Trp Asp Pro Leu Asp Cys Arg His Ala Lys
   1 5 10

### (2) INFORMATION FOR SEQ ID NO:10:

(i) SEQUENCE CHARACTERISTICS:
   (A) LENGTH: 5 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:10:
   Val His His Lys Arg
   1 5

### (2) INFORMATION FOR SEQ ID NO:11:

(i) SEQUENCE CHARACTERISTICS:
   (A) LENGTH: 592 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:11:
   Met Ser Phe Val Val Ile Ile Pro Ala Arg Tyr Ala Ser Thr Arg Leu
   1 5 10 15
   Pro Gly Lys Pro Leu Val Asp Ile Asn Gly Lys Pro Met Ile Val His
   20 25 30
   Val Leu Glu Arg Ala Arg Glu Ser Gly Ala Glu Arg Ile Ile Val Ala
   35 40 45
   Thr Asp His Glu Asp Val Ala Arg Ala Val Glu Ala Ala Gly Gly Glu
   50 55 60
   Val Cys Met Thr Arg Ala Asp His Gln Ser Gly Thr Glu Arg Leu Ala
   65 70 75 80
   Glu Val Val Glu Lys Cys Ala Phe Ser Asp Asp Thr Val Ile Val Asn
   85 90 95
   Val Gln Gly Asp Glu Pro Met Ile Pro Ala Thr Ile Ile Arg Gln Val
   100 105 110
   Ala Asp Asn Leu Ala Gln Arg Gln Val Gly Met Ala Thr Leu Ala Val
   115 120 125
   Pro Ile His Asn Ala Glu Glu Ala Phe Asn Pro Asn Ala Val Lys Val
   130 135 140
   Val Leu Asp Ala Glu Gly Tyr Ala Leu Tyr Phe Ser Arg Ala Thr Ile
   145 150 155 160
   Pro Trp Asp Arg Asp Arg Phe Ala Glu Gly Leu Glu Thr Val Gly Asp
   165 170 175
   Asn Phe Leu Arg His Leu Gly Ile Tyr Gly Tyr Arg Ala Gly Phe Ile
   180 185 190
   Arg Arg Tyr Val Asn Trp Gln Pro Ser Pro Leu Glu His Ile Glu Met
   195 200 205
   Leu Glu Gln Leu Arg Val Leu Trp Tyr Gly Glu Lys Ile His Val Ala
   210 215 220
   Val Ala Gln Glu Val Pro Gly Thr Gly Val Asp Thr Pro Glu Asp Leu
   225 230 235 240
   Asp Pro Ser Thr Asn Ser Met Asp Ala His Phe Leu Ser Gln Ala Pro
   245 250 255
   Pro Pro Ser Trp Asp Gln Met Trp Lys Cys Leu Ile Arg Leu Lys Pro
   260 265 270
   Thr Leu His Gly Pro Thr Pro Leu Leu Tyr Arg Leu Gly Ala Val Gln
   275 280 285
   Asn Glu Ile Thr Leu Thr His Pro Val Thr Lys Tyr Ile Met Thr Cys
   290 295 300
   Met Ser Ala Asp Leu Glu Val Val Thr Ser Thr Trp Val Leu Val Gly
   305 310 315 320
   Gly Val Leu Ala Ala Leu Ala Ala Tyr Cys Leu Ser Thr Gly Cys Val
   325 330 335
   Val Ile Val Gly Arg Val Val Leu Ser Gly Lys Pro Ala Ile Ile Pro
   340 345 350
   Asp Arg Glu Val Leu Tyr Arg Glu Phe Asp Glu Met Glu Glu Cys Ser
   355 360 365
   Gln His Leu Pro Tyr Ile Glu Gln Gly Met Met Leu Ala Glu Gln Phe
   370 375 380
   Lys Gln Lys Ala Leu Gly Leu Leu Gln Thr Ala Ser Arg Gln Ala Glu
   385 390 395 400
   Val Ile Ala Pro Ala Val Gln Thr Asn Trp Gln Lys Leu Glu Thr Phe
   405 410 415
   Trp Ala Lys His Met Trp Asn Phe Ile Ser Gly Ile Gln Tyr Leu Ala
   420 425 430
   Gly Leu Ser Thr Leu Pro Gly Asn Pro Ala Ile Ala Ser Leu Met Ala
   435 440 445
   Phe Thr Ala Ala Val Thr Ser Pro Leu Thr Thr Ser Gln Thr Leu Leu
   450 455 460
   Phe Asn Ile Leu Gly Gly Trp Val Ala Ala Gln Leu Ala Ala Pro Gly
   465 470 475 480
   Ala Ala Thr Ala Phe Val Gly Ala Gly Leu Ala Gly Ala Ala Ile Gly
   485 490 495
   Ser Val Gly Leu Gly Lys Val Leu Ile Asp Ile Leu Ala Gly Tyr Gly
   500 505 510
   Ala Gly Val Ala Gly Ala Leu Val Ala Phe Lys Ile Met Ser Gly Glu
   515 520 525
   Val Pro Ser Thr Glu Asp Leu Val Asn Leu Leu Pro Ala Ile Leu Ser
   530 535 540
   Pro Gly Ala Leu Val Val Gly Val Val Cys Ala Ala Ile Leu Arg Arg
   545 550 555 560
   His Val Gly Pro Gly Glu Gly Ala Val Gln Trp Met Asn Arg Leu Ile
   565 570 575
   Ala Phe Ala Ser Arg Gly Asn His Val Ser Pro Val His His Lys Arg
   580 585 590

### (2) INFORMATION FOR SEQ ID NO:12:

(i) SEQUENCE CHARACTERISTICS:
   (A) LENGTH: 594 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:12:
   Met Ser Phe Val Val Ile Ile Pro Ala Arg Tyr Ala Ser Thr Arg Leu
   1 5 10 15
   Pro Gly Lys Pro Leu Val Asp Ile Asn Gly Lys Pro Met Ile Val His
   20 25 30
   Val Leu Glu Arg Ala Arg Glu Ser Gly Ala Glu Arg Ile Ile Val Ala
   35 40 45
   Thr Asp His Glu Asp Val Ala Arg Ala Val Glu Ala Ala Gly Gly Glu
   50 55 60
   Val Cys Met Thr Arg Ala Asp His Gln Ser Gly Thr Glu Arg Leu Ala
   65 70 75 80
   Glu Val Val Glu Lys Cys Ala Phe Ser Asp Asp Thr Val Ile Val Asn
   85 90 95
   Val Gln Gly Asp Glu Pro Met Ile Pro Ala Thr Ile Ile Arg Gln Val
   100 105 110
   Ala Asp Asn Leu Ala Gln Arg Gln Val Gly Met Ala Thr Leu Ala Val
   115 120 125
   Pro Ile His Asn Ala Glu Glu Ala Phe Asn Pro Asn Ala Val Lys Val
   130 135 140
   Val Leu Asp Ala Glu Gly Tyr Ala Leu Tyr Phe Ser Arg Ala Thr Ile
   145 150 155 160
   Pro Trp Asp Arg Asp Arg Phe Ala Glu Gly Leu Glu Thr Val Gly Asp
   165 170 175
   Asn Phe Leu Arg His Leu Gly Ile Tyr Gly Tyr Arg Ala Gly Phe Ile
   180 185 190
   Arg Arg Tyr Val Asn Trp Gln Pro Ser Pro Leu Glu His Ile Glu Met
   195 200 205
   Leu Glu Gln Leu Arg Val Leu Trp Tyr Gly Glu Lys Ile His Val Ala
   210 215 220
   Val Ala Gln Glu Val Pro Gly Thr Gly Val Asp Thr Pro Glu Asp Leu
   225 230 235 240
   Asp Pro Ser Thr Asn Ser Met Asp Ala His Phe Leu Ser Gln Thr Lys
   245 250 255
   Gln Ser Gly Glu Asn Leu Pro Tyr Leu Val Ala Tyr Gln Ala Thr Val
   260 265 270
   Cys Ala Arg Ala Gln Ala Pro Thr Pro Leu Leu Tyr Arg Leu Gly Ala
   275 280 285
   Val Gln Asn Glu Ile Thr Leu Thr His Pro Val Thr Lys Tyr Ile Met
   290 295 300
   Thr Cys Met Ser Ala Asp Leu Glu Val Val Thr Ser Thr Trp Val Leu
   305 310 315 320
   Val Gly Gly Val Leu Ala Ala Leu Ala Ala Tyr Cys Leu Ser Thr Gly
   325 330 335
   Cys Val Val Ile Val Gly Arg Val Val Leu Ser Gly Lys Pro Ala Ile
   340 345 350
   Ile Pro Asp Arg Glu Val Leu Tyr Arg Glu Phe Asp Glu Met Glu Glu
   355 360 365
   Cys Ser Gln His Leu Pro Tyr Ile Glu Gln Gly Met Met Leu Ala Glu
   370 375 380
   Gln Phe Lys Gln Lys Ala Leu Gly Leu Leu Gln Thr Ala Ser Arg Gln
   385 390 395 400
   Ala Glu Val Ile Ala Pro Ala Val Gln Thr Asn Trp Gln Lys Leu Glu
   405 410 415
   Thr Phe Trp Ala Lys His Met Trp Asn Phe Ile Ser Gly Ile Gln Tyr
   420 425 430
   Leu Ala Gly Leu Ser Thr Leu Pro Gly Asn Pro Ala Ile Ala Ser Leu
   435 440 445
   Met Ala Phe Thr Ala Ala Val Thr Ser Pro Leu Thr Thr Ser Gln Thr
   450 455 460
   Leu Leu Phe Asn Ile Leu Gly Gly Trp Val Ala Ala Gln Leu Ala Ala
   465 470 475 480
   Pro Gly Ala Ala Thr Ala Phe Val Gly Ala Gly Leu Ala Gly Ala Ala
   485 490 495
   Ile Gly Ser Val Gly Leu Gly Lys Val Leu Ile Asp Ile Leu Ala Gly
   500 505 510
   Tyr Gly Ala Gly Val Ala Gly Ala Leu Val Ala Phe Lys Ile Met Ser
   515 520 525
   Gly Glu Val Pro Ser Thr Glu Asp Leu Val Asn Leu Leu Pro Ala Ile
   530 535 540
   Leu Ser Pro Gly Ala Leu Val Val Gly Val Val Cys Ala Ala Ile Leu
   545 550 555 560
   Arg Arg His Val Gly Pro Gly Glu Gly Ala Val Gln Trp Met Asn Arg
   565 570 575
   Leu Ile Ala Phe Ala Ser Arg Gly Asn His Val Ser Pro Val His His
   580 585 590
   Lys Arg

### (2) INFORMATION FOR SEQ ID NO:13:

(i) SEQUENCE CHARACTERISTICS:
   (A) LENGTH: 613 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:13:
   Met Ser Phe Val Val Ile Ile Pro Ala Arg Tyr Ala Ser Thr Arg Leu
   1 5 10 15
   Pro Gly Lys Pro Leu Val Asp Ile Asn Gly Lys Pro Met Ile Val His
   20 25 30
   Val Leu Glu Arg Ala Arg Glu Ser Gly Ala Glu Arg Ile Ile Val Ala
   35 40 45
   Thr Asp His Glu Asp Val Ala Arg Ala Val Glu Ala Ala Gly Gly Glu
   50 55 60
   Val Cys Met Thr Arg Ala Asp His Gln Ser Gly Thr Glu Arg Leu Ala
   65 70 75 80
   Glu Val Val Glu Lys Cys Ala Phe Ser Asp Asp Thr Val Ile Val Asn
   85 90 95
   Val Gln Gly Asp Glu Pro Met Ile Pro Ala Thr Ile Ile Arg Gln Val
   100 105 110
   Ala Asp Asn Leu Ala Gln Arg Gln Val Gly Met Ala Thr Leu Ala Val
   115 120 125
   Pro Ile His Asn Ala Glu Glu Ala Phe Asn Pro Asn Ala Val Lys Val
   130 135 140
   Val Leu Asp Ala Glu Gly Tyr Ala Leu Tyr Phe Ser Arg Ala Thr Ile
   145 150 155 160
   Pro Trp Asp Arg Asp Arg Phe Ala Glu Gly Leu Glu Thr Val Gly Asp
   165 170 175
   Asn Phe Leu Arg His Leu Gly Ile Tyr Gly Tyr Arg Ala Gly Phe Ile
   180 185 190
   Arg Arg Tyr Val Asn Trp Gln Pro Ser Pro Leu Glu His Ile Glu Met
   195 200 205
   Leu Glu Gln Leu Arg Val Leu Trp Tyr Gly Glu Lys Ile His Val Ala
   210 215 220
   Val Ala Gln Glu Val Pro Gly Thr Gly Val Asp Thr Pro Glu Asp Leu
   225 230 235 240
   Asp Pro Ser Thr Asn Ser Met Asp Ala His Phe Leu Ser Gln Thr Lys
   245 250 255
   Gln Ser Gly Glu Asn Leu Pro Tyr Leu Val Ala Tyr Gln Ala Thr Val
   260 265 270
   Cys Ala Arg Ala Gln Ala Pro Ser Trp Asp Gln Met Trp Lys Cys Leu
   275 280 285
   Ile Arg Leu Lys Pro Thr Leu His Gly Pro Thr Pro Leu Leu Tyr Arg
   290 295 300
   Leu Gly Ala Val Gln Asn Glu Ile Thr Leu Thr His Pro Val Thr Lys
   305 310 315 320
   Tyr Ile Met Thr Cys Met Ser Ala Asp Leu Glu Val Val Thr Ser Thr
   325 330 335
   Trp Val Leu Val Gly Gly Val Leu Ala Ala Leu Ala Ala Tyr Cys Leu
   340 345 350
   Ser Thr Gly Cys Val Val Ile Val Gly Arg Val Val Leu Ser Gly Lys
   355 360 365
   Pro Ala Ile Ile Pro Asp Arg Glu Val Leu Tyr Arg Glu Phe Asp Glu
   370 375 380
   Met Glu Glu Cys Ser Gln His Leu Pro Tyr Ile Glu Gln Gly Met Met
   385 390 395 400
   Leu Ala Glu Gln Phe Lys Gln Lys Ala Leu Gly Leu Leu Gln Thr Ala
   405 410 415
   Ser Arg Gln Ala Glu Val Ile Ala Pro Ala Val Gln Thr Asn Trp Gln
   420 425 430
   Lys Leu Glu Thr Phe Trp Ala Lys His Met Trp Asn Phe Ile Ser Gly
   435 440 445
   Ile Gln Tyr Leu Ala Gly Leu Ser Thr Leu Pro Gly Asn Pro Ala Ile
   450 455 460
   Ala Ser Leu Met Ala Phe Thr Ala Ala Val Thr Ser Pro Leu Thr Thr
   465 470 475 480
   Ser Gln Thr Leu Leu Phe Asn Ile Leu Gly Gly Trp Val Ala Ala Gln
   485 490 495
   Leu Ala Ala Pro Gly Ala Ala Thr Ala Phe Val Gly Ala Gly Leu Ala
   500 505 510
   Gly Ala Ala Ile Gly Ser Val Gly Leu Gly Lys Val Leu Ile Asp Ile
   515 520 525
   Leu Ala Gly Tyr Gly Ala Gly Val Ala Gly Ala Leu Val Ala Phe Lys
   530 535 540
   Ile Met Ser Gly Glu Val Pro Ser Thr Glu Asp Leu Val Asn Leu Leu
   545 550 555 560
   Pro Ala Ile Leu Ser Pro Gly Ala Leu Val Val Gly Val Val Cys Ala
   565 570 575
   Ala Ile Leu Arg Arg His Val Gly Pro Gly Glu Gly Ala Val Gln Trp
   580 585 590
   Met Asn Arg Leu Ile Ala Phe Ala Ser Arg Gly Asn His Val Ser Pro
   595 600 605
   Val His His Lys Arg
   610

### (2) INFORMATION FOR SEQ ID NO:14:

(i) SEQUENCE CHARACTERISTICS:
   (A) LENGTH: 375 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:14:
   Met Ser Phe Val Val Ile Ile Pro Ala Arg Tyr Ala Ser Thr Arg Leu
   1 5 10 15
   Pro Gly Lys Pro Leu Val Asp Ile Asn Gly Lys Pro Met Ile Val His
   20 25 30
   Val Leu Glu Arg Ala Arg Glu Ser Gly Ala Glu Arg Ile Ile Val Ala
   35 40 45
   Thr Asp His Glu Asp Val Ala Arg Ala Val Glu Ala Ala Gly Gly Glu
   50 55 60
   Val Cys Met Thr Arg Ala Asp His Gln Ser Gly Thr Glu Arg Leu Ala
   65 70 75 80
   Glu Val Val Glu Lys Cys Ala Phe Ser Asp Asp Thr Val Ile Val Asn
   85 90 95
   Val Gln Gly Asp Glu Pro Met Ile Pro Ala Thr Ile Ile Arg Gln Val
   100 105 110
   Ala Asp Asn Leu Ala Gln Arg Gln Val Gly Met Ala Thr Leu Ala Val
   115 120 125
   Pro Ile His Asn Ala Glu Glu Ala Phe Asn Pro Asn Ala Val Lys Val
   130 135 140
   Val Leu Asp Ala Glu Gly Tyr Ala Leu Tyr Phe Ser Arg Ala Thr Ile
   145 150 155 160
   Pro Trp Asp Arg Asp Arg Phe Ala Glu Gly Leu Glu Thr Val Gly Asp
   165 170 175
   Asn Phe Leu Arg His Leu Gly Ile Tyr Gly Tyr Arg Ala Gly Phe Ile
   180 185 190
   Arg Arg Tyr Val Asn Trp Gln Pro Ser Pro Leu Glu His Ile Glu Met
   195 200 205
   Leu Glu Gln Leu Arg Val Leu Trp Tyr Gly Glu Lys Ile His Val Ala
   210 215 220
   Val Ala Gln Glu Val Pro Gly Thr Gly Val Asp Thr Pro Glu Asp Leu
   225 230 235 240
   Asp Pro Ser Thr Glu Phe Val Ile Asp Cys Asn Thr Cys Val Thr Gln
   245 250 255
   Thr Val Asp Phe Ser Leu Asp Pro Thr Phe Thr Ile Glu Thr Thr Thr
   260 265 270
   Leu Pro Gln Asp Ala Val Ser Arg Thr Gln Arg Arg Gly Arg Thr Gly
   275 280 285
   Arg Gly Lys Pro Gly Ile Tyr Arg Phe Val Ala Pro Gly Glu Arg Pro
   290 295 300
   Ser Gly Met Phe Asp Ser Ser Val Leu Cys Glu Cys Tyr Asp Ala Gly
   305 310 315 320
   Cys Ala Trp Tyr Glu Leu Thr Pro Ala Glu Thr Thr Val Arg Leu Arg
   325 330 335
   Ala Tyr Met Asn Thr Pro Gly Leu Pro Val Cys Gln Asp His Leu Glu
   340 345 350
   Phe Trp Glu Gly Val Phe Thr Gly Leu Thr His Ile Ala Met Asp Pro
   355 360 365
   Leu Asp Cys Arg His Ala Lys
   370 375

### (2) INFORMATION FOR SEQ ID NO:15:

(i) SEQUENCE CHARACTERISTICS:
   (A) LENGTH: 1414 base pairs
   (B) TYPE: nucleic acid
   (C) STRANDEDNESS: single
   (D) TOPOLOGY: linear
(ii) MOLECULE TYPE: DNA (genomic)
(xi) SEQUENCE DESCRIPTION: SEQ ID NO:15:
   GAATTCGTGA TCGATTGCAA TACGTGTGTC ACCCAGACAG TCGACTTCAG CCTTGACCCT 60
   ACCTTCACCA TTGAGACAAC CACGCTTCCC CAGGATGCTG TCTCCCGTAC TCAGCGTCGG 120
   GGCAGGACTG GTAGGGGGAA GCCAGGCATC TACAGATTTG TGGCACCGGG GGAGCGCCCT 180
   TCCGGCATGT TTGACTCGTC CGTCCTCTGC GAGTGCTATG ACGCGGGCTG TGCTTGGTAT 240
   GAGCTTACAC CCGCCGAGAC CACAGTTAGG CTACGAGCTT ACATGAACAC TCCGGGGCTT 300
   CCCGTGTGCC AAGATCATCT TGAATTTTGG GAGGGCGTCT TTACAGGCCT CACTCATATA 360
   GCCATGGACG CTCACTTCCT GTCTCAGGCG CCGCCGCCGT CTTGGGATCA GATGTGGAAA 420
   TGCCTGATCC GTCTGAAACC GACCCTGCAC GGCCCGACCC CGCTGCTGTA CCGTCTGGGT 480
   GCTGTTCAGA ACGAAATCAC CCTGACCCAC CCGGTTACCA AATACATCAT GACCTGCATG 540
   TCTGCTGATC TAGAAGTTGT TACCTCTACC TGGGTTCTGG TTGGTGGTGT TCTGGCTGCT 600
   CTGGCTGCTT ACTGCCTGTC GACCGGTTGC GTTGTTATCG TTGGTCGTGT TGTTCTGTCT 660
   GGTAAACCGG CCATTATCCC GGACCGTGAA GTTCTGTACC GTGAGTTCGA CGAAATGGAA 720
   GAATGCTCTC AGCACCTGCC GTACATCGAA CAGGGTATGA TGCTGGCTGA ACAGTTCAAA 780
   CAGAAAGCTC TGGGTCTGCT GCAGACCGCT TCTCGTCAGG CTGAAGTTAT CGCTCCGGCT 840
   GTTCAGACCA ACTGGCAGAA ACTCGAGACC TTCTGGGCTA AACACATGTG GAACTTCATC 900
   TCTGGTATCC AGTACCTGGC TGGTCTGTCT ACCCTGCCGG GTAACCCGGC TATCGCAAGC 960
   TTGATGGCTT TCACCGCTGC TGTTACCTCT CCGCTGACCA CCTCTCAGAC CCTGCTGTTC 1020
   AACATTCTGG GTGGTTGGGT TGCTGCTCAG CTGGCTGCTC CGGGTGCTGC TACCGCTTTC 1080
   GTTGGTGCTG GTCTGGCTGG TGCTGCTATC GGTTCTGTAG GCCTGGGTAA AGTTCTGATC 1140
   GACATTCTGG CTGGTTACGG TGCTGGTGTT GCTGGAGCTC TGGTTGCTTT CAAAATCATG 1200
   TCTGGTGAAG TTCCGTCTAC CGAAGATCTG GTTAACCTGC TGCCGGCTAT CCTGTCTCCG 1260
   GGTGCTCTGG TTGTTGGTGT TGTTTGCGCT GCTATCCTGC GTCGTCACGT TGGCCCGGGT 1320
   GAAGGTGCTG TTCAGTGGAT GAACCGTCTG ATCGCTTTCG CTTCTCGTGG TAACCACGTT 1380
   TCTCCGGTTC ACCACAAACG TTAACCATGG ATCC 1414

### (2) INFORMATION FOR SEQ ID NO:16:

(i) SEQUENCE CHARACTERISTICS:
   (A) LENGTH: 971 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:16:
   Met Ser Phe Val Val Ile Ile Pro Ala Arg Tyr Ala Ser Thr Arg Leu
   1               5                   10                  15
   Pro Gly Lys Pro Leu Val Asp Ile Asn Gly Lys Pro Met Ile Val His
               20                  25                  30
   Val Leu Glu Arg Ala Arg Glu Ser Gly Ala Glu Arg Ile Ile Val Ala
           35                  40                  45
   Thr Asp His Glu Asp Val Ala Arg Ala Val Glu Ala Ala Gly Gly Glu
       50                  55                  60
   Val Cys Met Thr Arg Ala Asp His Gln Ser Gly Thr Glu Arg Leu Ala
   65                  70                  75                  80
   Glu Val Val Glu Lys Cys Ala Phe Ser Asp Asp Thr Val Ile Val Asn
                   85                  90                  95
   Val Gln Gly Asp Glu Pro Met Ile Pro Ala Thr Ile Ile Arg Gln Val
               100                 105                 110
   Ala Asp Asn Leu Ala Gln Arg Gln Val Gly Met Ala Thr Leu Ala Val
           115                 120                 125
   Pro Ile His Asn Ala Glu Glu Ala Phe Asn Pro Asn Ala Val Lys Val
       130                 135                 140
   Val Leu Asp Ala Glu Gly Tyr Ala Leu Tyr Phe Ser Arg Ala Thr Ile
   145                 150                 155                 160
   Pro Trp Asp Arg Asp Arg Phe Ala Glu Gly Leu Glu Thr Val Gly Asp
                   165                 170                 175
   Asn Phe Leu Arg His Leu Gly Ile Tyr Gly Tyr Arg Ala Gly Phe Ile
               180                 185                 190
   Arg Arg Tyr Val Asn Trp Gln Pro Ser Pro Leu Glu His Ile Glu Met
           195                 200                 205
   Leu Glu Gln Leu Arg Val Leu Trp Tyr Gly Glu Lys Ile His Val Ala
       210                 215                 220
   Val Ala Gln Glu Val Pro Gly Thr Gly Val Asp Thr Pro Glu Asp Leu
   225                 230                 235                 240
   Asp Pro Ser Thr Asn Ser Met Ala Val Asp Phe Ile Pro Val Glu Asn
                   245                 250                 255
   Leu Glu Thr Thr Met Arg Ser Pro Val Phe Thr Asp Asn Ser Ser Pro
               260                 265                 270
   Pro Val Val Pro Gln Ser Phe Gln Val Ala His Leu His Ala Pro Thr
           275                 280                 285
   Gly Ser Gly Lys Ser Thr Lys Val Pro Ala Ala Tyr Ala Ala Gln Gly
       290                 295                 300
   Tyr Lys Val Leu Val Leu Asn Pro Ser Val Ala Ala Thr Leu Gly Phe
   305                 310                 315                 320
   Gly Ala Tyr Met Ser Lys Ala His Gly Ile Asp Pro Asn Ile Arg Thr
   325 330 335
   Gly Val Arg Thr Ile Thr Thr Gly Ser Pro Ile Thr Tyr Ser Thr Tyr
   340 345 350
   Gly Lys Phe Leu Ala Asp Gly Gly Cys Ser Gly Gly Ala Tyr Asp Ile
   355 360 365
   Ile Ile Cys Asp Glu Cys His Ser Thr Asp Ala Thr Ser Ile Leu Gly
   370 375 380
   Ile Gly Thr Val Leu Asp Gln Ala Glu Thr Ala Gly Ala Arg Leu Val
   385 390 395 400
   Val Leu Ala Thr Ala Thr Pro Pro Gly Ser Val Thr Val Pro His Pro
   405 410 415
   Asn Ile Glu Glu Val Ala Leu Ser Thr Thr Gly Glu Ile Pro Phe Tyr
   420 425 430
   Gly Lys Ala Ile Pro Leu Glu Val Ile Lys Gly Gly Arg His Leu Ile
   435 440 445
   Phe Cys His Ser Lys Lys Lys Cys Asp Glu Leu Ala Ala Lys Leu Val
   450 455 460
   Ala Leu Gly Ile Asn Ala Val Ala Tyr Tyr Arg Gly Leu Asp Val Ser
   465 470 475 480
   Val Ile Pro Thr Ser Gly Asp Val Val Val Val Ala Thr Asp Ala Leu
   485 490 495
   Met Thr Gly Tyr Thr Gly Asp Phe Asp Ser Val Ile Asp Cys Asn Thr
   500 505 510
   Cys Val Thr Gln Thr Val Asp Phe Ser Leu Asp Pro Thr Phe Thr Ile
   515 520 525
   Glu Thr Thr Thr Leu Pro Gln Asp Ala Val Ser Arg Thr Gln Arg Arg
   530 535 540
   Gly Arg Thr Gly Arg Gly Lys Pro Gly Ile Tyr Arg Phe Val Ala Pro
   545 550 555 560
   Gly Glu Arg Pro Ser Gly Met Phe Asp Ser Ser Val Leu Cys Glu Cys
   565 570 575
   Tyr Asp Ala Gly Cys Ala Trp Tyr Glu Leu Thr Pro Ala Glu Thr Thr
   580 585 590
   Val Arg Leu Arg Ala Tyr Met Asn Thr Pro Gly Leu Pro Val Cys Gln
   595 600 605
   Asp His Leu Glu Phe Trp Glu Gly Val Phe Thr Gly Leu Thr His Ile
   610 615 620
   Ala Met Asp Ala His Phe Leu Ser Gln Ala Pro Pro Pro Ser Trp Asp
   625 630 635 640
   Gln Met Trp Lys Cys Leu Ile Arg Leu Lys Pro Thr Leu His Gly Pro
   645 650 655
   Thr Pro Leu Leu Tyr Arg Leu Gly Ala Val Gln Asn Glu Ile Thr Leu
   660 665 670
   Thr His Pro Val Thr Lys Tyr Ile Met Thr Cys Met Ser Ala Asp Leu
   675 680 685
   Glu Val Val Thr Ser Thr Trp Val Leu Val Gly Gly Val Leu Ala Ala
   690 695 700
   Leu Ala Ala Tyr Cys Leu Ser Thr Gly Cys Val Val Ile Val Gly Arg
   705 710 715 720
   Val Val Leu Ser Gly Lys Pro Ala Ile Ile Pro Asp Arg Glu Val Leu
   725 730 735
   Tyr Arg Glu Phe Asp Glu Met Glu Glu Cys Ser Gln His Leu Pro Tyr
   740 745 750
   Ile Glu Gln Gly Met Met Leu Ala Glu Gln Phe Lys Gln Lys Ala Leu
   755 760 765
   Gly Leu Leu Gln Thr Ala Ser Arg Gln Ala Glu Val Ile Ala Pro Ala
   770 775 780
   Val Gln Thr Asn Trp Gln Lys Leu Glu Thr Phe Trp Ala Lys His Met
   785 790 795 800
   Trp Asn Phe Ile Ser Gly Ile Gln Tyr Leu Ala Gly Leu Ser Thr Leu
   805 810 815
   Pro Gly Asn Pro Ala Ile Ala Ser Leu Met Ala Phe Thr Ala Ala Val
   820 825 830
   Thr Ser Pro Leu Thr Thr Ser Gln Thr Leu Leu Phe Asn Ile Leu Gly
   835 840 845
   Gly Trp Val Ala Ala Gln Leu Ala Ala Pro Gly Ala Ala Thr Ala Phe
   850 855 860
   Val Gly Ala Gly Leu Ala Gly Ala Ala Ile Gly Ser Val Gly Leu Gly
   865 870 875 880
   Lys Val Leu Ile Asp Ile Leu Ala Gly Tyr Gly Ala Gly Val Ala Gly
   885 890 895
   Ala Leu Val Ala Phe Lys Ile Met Ser Gly Glu Val Pro Ser Thr Glu
   900 905 910
   Asp Leu Val Asn Leu Leu Pro Ala Ile Leu Ser Pro Gly Ala Leu Val
   915 920 925
   Val Gly Val Val Cys Ala Ala Ile Leu Arg Arg His Val Gly Pro Gly
   930 935 940
   Glu Gly Ala Val Gln Trp Met Asn Arg Leu Ile Ala Phe Ala Ser Arg
   945 950 955 960
   Gly Asn His Val Ser Pro Val His His Lys Arg
   965 970

### (2) INFORMATION FOR SEQ ID NO:17:

(i) SEQUENCE CHARACTERISTICS:
   (A) LENGTH: 973 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:17:
   Met Ser Phe Val Val Ile Ile Pro Ala Arg Tyr Ala Ser Thr Arg Leu
   1 5 10 15
   Pro Gly Lys Pro Leu Val Asp Ile Asn Gly Lys Pro Met Ile Val His
   20 25 30
   Val Leu Glu Arg Ala Arg Glu Ser Gly Ala Glu Arg Ile Ile Val Ala
   35 40 45
   Thr Asp His Glu Asp Val Ala Arg Ala Val Glu Ala Ala Gly Gly Glu
   50 55 60
   Val Cys Met Thr Arg Ala Asp His Gln Ser Gly Thr Glu Arg Leu Ala
   65 70 75 80
   Glu Val Val Glu Lys Cys Ala Phe Ser Asp Asp Thr Val Ile Val Asn
   85 90 95
   Val Gln Gly Asp Glu Pro Met Ile Pro Ala Thr Ile Ile Arg Gln Val
   100 105 110
   Ala Asp Asn Leu Ala Gln Arg Gln Val Gly Met Ala Thr Leu Ala Val
   115 120 125
   Pro Ile His Asn Ala Glu Glu Ala Phe Asn Pro Asn Ala Val Lys Val
   130 135 140
   Val Leu Asp Ala Glu Gly Tyr Ala Leu Tyr Phe Ser Arg Ala Thr Ile
   145 150 155 160
   Pro Trp Asp Arg Asp Arg Phe Ala Glu Gly Leu Glu Thr Val Gly Asp
   165 170 175
   Asn Phe Leu Arg His Leu Gly Ile Tyr Gly Tyr Arg Ala Gly Phe Ile
   180 185 190
   Arg Arg Tyr Val Asn Trp Gln Pro Ser Pro Leu Glu His Ile Glu Met
   195 200 205
   Leu Glu Gln Leu Arg Val Leu Trp Tyr Gly Glu Lys Ile His Val Ala
   210 215 220
   Val Ala Gln Glu Val Pro Gly Thr Gly Val Asp Thr Pro Glu Asp Leu
   225 230 235 240
   Asp Pro Ser Thr Asn Ser Met Ala Val Asp Phe Ile Pro Val Glu Asn
   245 250 255
   Leu Glu Thr Thr Met Arg Ser Pro Val Phe Thr Asp Asn Ser Ser Pro
   260 265 270
   Pro Val Val Pro Gln Ser Phe Gln Val Ala His Leu His Ala Pro Thr
   275 280 285
   Gly Ser Gly Lys Ser Thr Lys Val Pro Ala Ala Tyr Ala Ala Gln Gly
   290 295 300
   Tyr Lys Val Leu Val Leu Asn Pro Ser Val Ala Ala Thr Leu Gly Phe
   305 310 315 320
   Gly Ala Tyr Met Ser Lys Ala His Gly Ile Asp Pro Asn Ile Arg Thr
   325 330 335
   Gly Val Arg Thr Ile Thr Thr Gly Ser Pro Ile Thr Tyr Ser Thr Tyr
   340 345 350
   Gly Lys Phe Leu Ala Asp Gly Gly Cys Ser Gly Gly Ala Tyr Asp Ile
   355 360 365
   Ile Ile Cys Asp Glu Cys His Ser Thr Asp Ala Thr Ser Ile Leu Gly
   370 375 380
   Ile Gly Thr Val Leu Asp Gln Ala Glu Thr Ala Gly Ala Arg Leu Val
   385 390 395 400
   Val Leu Ala Thr Ala Thr Pro Pro Gly Ser Val Thr Val Pro His Pro
   405 410 415
   Asn Ile Glu Glu Val Ala Leu Ser Thr Thr Gly Glu Ile Pro Phe Tyr
   420 425 430
   Gly Lys Ala Ile Pro Leu Glu Val Ile Lys Gly Gly Arg His Leu Ile
   435 440 445
   Phe Cys His Ser Lys Lys Lys Cys Asp Glu Leu Ala Ala Lys Leu Val
   450 455 460
   Ala Leu Gly Ile Asn Ala Val Ala Tyr Tyr Arg Gly Leu Asp Val Ser
   465 470 475 480
   Val Ile Pro Thr Ser Gly Asp Val Val Val Val Ala Thr Asp Ala Leu
   485 490 495
   Met Thr Gly Tyr Thr Gly Asp Phe Asp Ser Val Ile Asp Cys Asn Thr
   500 505 510
   Cys Val Thr Gln Thr Val Asp Phe Ser Leu Asp Pro Thr Phe Thr Ile
   515 520 525
   Glu Thr Thr Thr Leu Pro Gln Asp Ala Val Ser Arg Thr Gln Arg Arg
   530 535 540
   Gly Arg Thr Gly Arg Gly Lys Pro Gly Ile Tyr Arg Phe Val Ala Pro
   545 550 555 560
   Gly Glu Arg Pro Ser Gly Met Phe Asp Ser Ser Val Leu Cys Glu Cys
   565 570 575
   Tyr Asp Ala Gly Cys Ala Trp Tyr Glu Leu Thr Pro Ala Glu Thr Thr
   580 585 590
   Val Arg Leu Arg Ala Tyr Met Asn Thr Pro Gly Leu Pro Val Cys Gln
   595 600 605
   Asp His Leu Glu Phe Trp Glu Gly Val Phe Thr Gly Leu Thr His Ile
   610 615 620
   Ala Met Asp Ala His Phe Leu Ser Gln Thr Lys Gln Ser Gly Glu Asn
   625 630 635 640
   Leu Pro Tyr Leu Val Ala Tyr Gln Ala Thr Val Cys Ala Arg Ala Gln
   645 650 655
   Ala Pro Thr Pro Leu Leu Tyr Arg Leu Gly Ala Val Gln Asn Glu Ile
   660 665 670
   Thr Leu Thr His Pro Val Thr Lys Tyr Ile Met Thr Cys Met Ser Ala
   675 680 685
   Asp Leu Glu Val Val Thr Ser Thr Trp Val Leu Val Gly Gly Val Leu
   690 695 700
   Ala Ala Leu Ala Ala Tyr Cys Leu Ser Thr Gly Cys Val Val Ile Val
   705 710 715 720
   Gly Arg Val Val Leu Ser Gly Lys Pro Ala Ile Ile Pro Asp Arg Glu
   725 730 735
   Val Leu Tyr Arg Glu Phe Asp Glu Met Glu Glu Cys Ser Gln His Leu
   740 745 750
   Pro Tyr Ile Glu Gln Gly Met Met Leu Ala Glu Gln Phe Lys Gln Lys
   755 760 765
   Ala Leu Gly Leu Leu Gln Thr Ala Ser Arg Gln Ala Glu Val Ile Ala
   770 775 780
   Pro Ala Val Gln Thr Asn Trp Gln Lys Leu Glu Thr Phe Trp Ala Lys
   785 790 795 800
   His Met Trp Asn Phe Ile Ser Gly Ile Gln Tyr Leu Ala Gly Leu Ser
   805 810 815
   Thr Leu Pro Gly Asn Pro Ala Ile Ala Ser Leu Met Ala Phe Thr Ala
   820 825 830
   Ala Val Thr Ser Pro Leu Thr Thr Ser Gln Thr Leu Leu Phe Asn Ile
   835 840 845
   Leu Gly Gly Trp Val Ala Ala Gln Leu Ala Ala Pro Gly Ala Ala Thr
   850 855 860
   Ala Phe Val Gly Ala Gly Leu Ala Gly Ala Ala Ile Gly Ser Val Gly
   865 870 875 880
   Leu Gly Lys Val Leu Ile Asp Ile Leu Ala Gly Tyr Gly Ala Gly Val
   885 890 895
   Ala Gly Ala Leu Val Ala Phe Lys Ile Met Ser Gly Glu Val Pro Ser
   900 905 910
   Thr Glu Asp Leu Val Asn Leu Leu Pro Ala Ile Leu Ser Pro Gly Ala
   915 920 925
   Leu Val Val Gly Val Val Cys Ala Ala Ile Leu Arg Arg His Val Gly
   930 935 940
   Pro Gly Glu Gly Ala Val Gln Trp Met Asn Arg Leu Ile Ala Phe Ala
   945 950 955 960
   Ser Arg Gly Asn His Val Ser Pro Val His His Lys Arg
   965 970

### (2) INFORMATION FOR SEQ ID NO:18:

(i) SEQUENCE CHARACTERISTICS:
   (A) LENGTH: 992 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:18:
   Met Ser Phe Val Val Ile Ile Pro Ala Arg Tyr Ala Ser Thr Arg Leu
   1 5 10 15
   Pro Gly Lys Pro Leu Val Asp Ile Asn Gly Lys Pro Met Ile Val His
   20 25 30
   Val Leu Glu Arg Ala Arg Glu Ser Gly Ala Glu Arg Ile Ile Val Ala
   35 40 45
   Thr Asp His Glu Asp Val Ala Arg Ala Val Glu Ala Ala Gly Gly Glu
   50 55 60
   Val Cys Met Thr Arg Ala Asp His Gln Ser Gly Thr Glu Arg Leu Ala
   65 70 75 80
   Glu Val Val Glu Lys Cys Ala Phe Ser Asp Asp Thr Val Ile Val Asn
   85 90 95
   Val Gln Gly Asp Glu Pro Met Ile Pro Ala Thr Ile Ile Arg Gln Val
   100 105 110
   Ala Asp Asn Leu Ala Gln Arg Gln Val Gly Met Ala Thr Leu Ala Val
   115 120 125
   Pro Ile His Asn Ala Glu Glu Ala Phe Asn Pro Asn Ala Val Lys Val
   130 135 140
   Val Leu Asp Ala Glu Gly Tyr Ala Leu Tyr Phe Ser Arg Ala Thr Ile
   145 150 155 160
   Pro Trp Asp Arg Asp Arg Phe Ala Glu Gly Leu Glu Thr Val Gly Asp
   165 170 175
   Asn Phe Leu Arg His Leu Gly Ile Tyr Gly Tyr Arg Ala Gly Phe Ile
   180 185 190
   Arg Arg Tyr Val Asn Trp Gln Pro Ser Pro Leu Glu His Ile Glu Met
   195 200 205
   Leu Glu Gln Leu Arg Val Leu Trp Tyr Gly Glu Lys Ile His Val Ala
   210 215 220
   Val Ala Gln Glu Val Pro Gly Thr Gly Val Asp Thr Pro Glu Asp Leu
   225 230 235 240
   Asp Pro Ser Thr Asn Ser Met Ala Val Asp Phe Ile Pro Val Glu Asn
   245 250 255
   Leu Glu Thr Thr Met Arg Ser Pro Val Phe Thr Asp Asn Ser Ser Pro
   260 265 270
   Pro Val Val Pro Gln Ser Phe Gln Val Ala His Leu His Ala Pro Thr
   275 280 285
   Gly Ser Gly Lys Ser Thr Lys Val Pro Ala Ala Tyr Ala Ala Gln Gly
   290 295 300
   Tyr Lys Val Leu Val Leu Asn Pro Ser Val Ala Ala Thr Leu Gly Phe
   305 310 315 320
   Gly Ala Tyr Met Ser Lys Ala His Gly Ile Asp Pro Asn Ile Arg Thr
   325 330 335
   Gly Val Arg Thr Ile Thr Thr Gly Ser Pro Ile Thr Tyr Ser Thr Tyr
   340 345 350
   Gly Lys Phe Leu Ala Asp Gly Gly Cys Ser Gly Gly Ala Tyr Asp Ile
   355 360 365
   Ile Ile Cys Asp Glu Cys His Ser Thr Asp Ala Thr Ser Ile Leu Gly
   370 375 380
   Ile Gly Thr Val Leu Asp Gln Ala Glu Thr Ala Gly Ala Arg Leu Val
   385 390 395 400
   Val Leu Ala Thr Ala Thr Pro Pro Gly Ser Val Thr Val Pro His Pro
   405 410 415
   Asn Ile Glu Glu Val Ala Leu Ser Thr Thr Gly Glu Ile Pro Phe Tyr
   420 425 430
   Gly Lys Ala Ile Pro Leu Glu Val Ile Lys Gly Gly Arg His Leu Ile
   435 440 445
   Phe Cys His Ser Lys Lys Lys Cys Asp Glu Leu Ala Ala Lys Leu Val
   450 455 460
   Ala Leu Gly Ile Asn Ala Val Ala Tyr Tyr Arg Gly Leu Asp Val Ser
   465 470 475 480
   Val Ile Pro Thr Ser Gly Asp Val Val Val Val Ala Thr Asp Ala Leu
   485 490 495
   Met Thr Gly Tyr Thr Gly Asp Phe Asp Ser Val Ile Asp Cys Asn Thr
   500 505 510
   Cys Val Thr Gln Thr Val Asp Phe Ser Leu Asp Pro Thr Phe Thr Ile
   515 520 525
   Glu Thr Thr Thr Leu Pro Gln Asp Ala Val Ser Arg Thr Gln Arg Arg
   530 535 540
   Gly Arg Thr Gly Arg Gly Lys Pro Gly Ile Tyr Arg Phe Val Ala Pro
   545 550 555 560
   Gly Glu Arg Pro Ser Gly Met Phe Asp Ser Ser Val Leu Cys Glu Cys
   565 570 575
   Tyr Asp Ala Gly Cys Ala Trp Tyr Glu Leu Thr Pro Ala Glu Thr Thr
   580 585 590
   Val Arg Leu Arg Ala Tyr Met Asn Thr Pro Gly Leu Pro Val Cys Gln
   595 600 605
   Asp His Leu Glu Phe Trp Glu Gly Val Phe Thr Gly Leu Thr His Ile
   610 615 620
   Ala Met Asp Ala His Phe Leu Ser Gln Thr Lys Gln Ser Gly Glu Asn
   625 630 635 640
   Leu Pro Tyr Leu Val Ala Tyr Gln Ala Thr Val Cys Ala Arg Ala Gln
   645 650 655
   Ala Pro Ser Trp Asp Gln Met Trp Lys Cys Leu Ile Arg Leu Lys Pro
   660 665 670
   Thr Leu His Gly Pro Thr Pro Leu Leu Tyr Arg Leu Gly Ala Val Gln
   675 680 685
   Asn Glu Ile Thr Leu Thr His Pro Val Thr Lys Tyr Ile Met Thr Cys
   690 695 700
   Met Ser Ala Asp Leu Glu Val Val Thr Ser Thr Trp Val Leu Val Gly
   705 710 715 720
   Gly Val Leu Ala Ala Leu Ala Ala Tyr Cys Leu Ser Thr Gly Cys Val
   725 730 735
   Val Ile Val Gly Arg Val Val Leu Ser Gly Lys Pro Ala Ile Ile Pro
   740 745 750
   Asp Arg Glu Val Leu Tyr Arg Glu Phe Asp Glu Met Glu Glu Cys Ser
   755 760 765
   Gln His Leu Pro Tyr Ile Glu Gln Gly Met Met Leu Ala Glu Gln Phe
   770 775 780
   Lys Gln Lys Ala Leu Gly Leu Leu Gln Thr Ala Ser Arg Gln Ala Glu
   785 790 795 800
   Val Ile Ala Pro Ala Val Gln Thr Asn Trp Gln Lys Leu Glu Thr Phe
   805 810 815
   Trp Ala Lys His Met Trp Asn Phe Ile Ser Gly Ile Gln Tyr Leu Ala
   820 825 830
   Gly Leu Ser Thr Leu Pro Gly Asn Pro Ala Ile Ala Ser Leu Met Ala
   835 840 845
   Phe Thr Ala Ala Val Thr Ser Pro Leu Thr Thr Ser Gln Thr Leu Leu
   850 855 860
   Phe Asn Ile Leu Gly Gly Trp Val Ala Ala Gln Leu Ala Ala Pro Gly
   865 870 875 880
   Ala Ala Thr Ala Phe Val Gly Ala Gly Leu Ala Gly Ala Ala Ile Gly
   885 890 895
   Ser Val Gly Leu Gly Lys Val Leu Ile Asp Ile Leu Ala Gly Tyr Gly
   900 905 910
   Ala Gly Val Ala Gly Ala Leu Val Ala Phe Lys Ile Met Ser Gly Glu
   915 920 925
   Val Pro Ser Thr Glu Asp Leu Val Asn Leu Leu Pro Ala Ile Leu Ser
   930 935 940
   Pro Gly Ala Leu Val Val Gly Val Val Cys Ala Ala Ile Leu Arg Arg
   945 950 955 960
   His Val Gly Pro Gly Glu Gly Ala Val Gln Trp Met Asn Arg Leu Ile
   965 970 975
   Ala Phe Ala Ser Arg Gly Asn His Val Ser Pro Val His His Lys Arg
   980 985 990

### (2) INFORMATION FOR SEQ ID NO:19:

(i) SEQUENCE CHARACTERISTICS:
   (A) LENGTH: 21 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:19:
   Pro Pro Pro Ser Tyr Asp Gln Met Trp Lys Cys Leu Ile Arg Leu Lys
   1 5 10 15
   Pro Thr Leu His Gly
   20

### (2) INFORMATION FOR SEQ ID NO:20:

(i) SEQUENCE CHARACTERISTICS:
   (A) LENGTH: 382 base pairs
   (B) TYPE: nucleic acid
   (C) STRANDEDNESS: single
   (D) TOPOLOGY: linear
(ii) MOLECULE TYPE: DNA (genomic)
(xi) SEQUENCE DESCRIPTION: SEQ ID NO:20:
   CTCCACTCTA TGAATTCGTG ATCGATTGCA ATACGTGTGT CACCCAGACA GTCGACTTCA    60
   GCCTTGACCC TACCTTCACC ATTGAGACAA CCACGCTTCC CCAGGATGCT GTCTCCCGTA   120
   CTCAGCGTCG GGGCAGGACT GGTAGGGGGA AGCCAGGCAT CTACAGATTT GTGGCACCGG   180
   GGGAGCGCCC TTCCGGCATG TTTGACTCGT CCGTCCTCTG CGAGTGCTAT GACGCGGGCT   240
   GTGCTTGGTA TGAGCTTACA CCCGCCGAGA CCACAGTTAG GCTACGAGCT TACATGAACA   300
   CTCCGGGGCT TCCCGTGTGC CAAGATCATC TTGAATTTTG GGAGGGCGTC TTTACAGGCC   360
   TCACTCATAT AGCCATGGAT CC                                            382

### (2) INFORMATION FOR SEQ ID NO:21:

(i) SEQUENCE CHARACTERISTICS:
   (A) LENGTH: 1420 base pairs
   (B) TYPE: nucleic acid
   (C) STRANDEDNESS: single
   (D) TOPOLOGY: linear
(ii) MOLECULE TYPE: DNA (genomic)
(xi) SEQUENCE DESCRIPTION: SEQ ID NO:21:
   GAATTCGTGA TCGATTGCAA TACGTGTGTC ACCCAGACAG TCGACTTCAG CCTTGACCCT 60
   ACCTTCACCA TTGAGACAAC CACGCTTCCC CAGGATGCTG TCTCCCGTAC TCAGCGTCGG 120
   GGCAGGACTG GTAGGGGGAA GCCAGGCATC TACAGATTTG TGGCACCGGG GGAGCGCCCT 180
   TCCGGCATGT TTGACTCGTC CGTCCTCTGC GAGTGCTATG ACGCGGGCTG TGCTTGGTAT 240
   GAGCTTACAC CCGCCGAGAC CACAGTTAGG CTACGAGCTT ACATGAACAC TCCGGGGCTT 300
   CCCGTGTGCC AAGATCATCT TGAATTTTGG GAGGGCGTCT TTACAGGCCT CACTCATATA 360
   GCCATGGACG CTCACTTCCT GTCTCAGACC AAACAGTCTG GTGAAAACCT TCCGTACCTG 420
   GTTGCTTACC AGGCTACCGT TTGCGCTCGT GCTCAGGCCC CGACCCCGCT GCTGTACCGT 480
   CTGGGTGCTG TTCAGAACGA AATCACCCTG ACCCACCCGG TTACCAAATA CATCATGACC 540
   TGCATGTCTG CTGATCTAGA AGTTGTTACC TCTACCTGGG TTCTGGTTGG TGGTGTTCTG 600
   GCTGCTCTGG CTGCTTACTG CCTGTCGACC GGTTGCGTTG TTATCGTTGG TCGTGTTGTT 660
   CTGTCTGGTA AACCGGCCAT TATCCCGGAC CGTGAAGTTC TGTACCGTGA GTTCGACGAA 720
   ATGGAAGAAT GCTCTCAGCA CCTGCCGTAC ATCGAACAGG GTATGATGCT GGCTGAACAG 780
   TTCAAACAGA AAGCTCTGGG TCTGCTGCAG ACCGCTTCTC GTCAGGCTGA AGTTATCGCT 840
   CCGGCTGTTC AGACCAACTG GCAGAAACTC GAGACCTTCT GGGCTAAACA CATGTGGAAC 900
   TTCATCTCTG GTATCCAGTA CCTGGCTGGT CTGTCTACCC TGCCGGGTAA CCCGGCTATC 960
   GCAAGCTTGA TGGCTTTCAC CGCTGCTGTT ACCTCTCCGC TGACCACCTC TCAGACCCTG 1020
   CTGTTCAACA TTCTGGGTGG TTGGGTTGCT GCTCAGCTGG CTGCTCCGGG TGCTGCTACC 1080
   GCTTTCGTTG GTGCTGGTCT GGCTGGTGCT GCTATCGGTT CTGTAGGCCT GGGTAAAGTT 1140
   CTGATCGACA TTCTGGCTGG TTACGGTGCT GGTGTTGCTG GAGCTCTGGT TGCTTTCAAA 1200
   ATCATGTCTG GTGAAGTTCC GTCTACCGAA GATCTGGTTA ACCTGCTGCC GGCTATCCTG 1260
   TCTCCGGGTG CTCTGGTTGT TGGTGTTGTT TGCGCTGCTA TCCTGCGTCG TCACGTTGGC 1320
   CCGGGTGAAG GTGCTGTTCA GTGGATGAAC CGTCTGATCG CTTTCGCTTC TCGTGGTAAC 1380
   CACGTTTCTC CGGTTCACCA CAAACGTTAA CCATGGATCC 1420

### (2) INFORMATION FOR SEQ ID NO : 22:

(i) SEQUENCE CHARACTERISTICS:
   (A) LENGTH: 1791 base pairs
   (B) TYPE: nucleic acid
   (C) STRANDEDNESS: single
   (D) TOPOLOGY: circular
(ii) MOLECULE TYPE: DNA (genomic)
(ix) FEATURE:
   (A) NAME/KEY: CDS
   (B) LOCATION: 1..1791
(xi) SEQUENCE DESCRIPTION: SEQ ID NO: 22:
   ATG AGT TTT GTG GTC ATT ATT CCC GCG CGC TAC GCG TCG ACG CGT CTG      48
   Met Ser Phe Val Val Ile Ile Pro Ala Arg Tyr Ala Ser Thr Arg Leu
     1               5                  10                  15
   CCC GGT AAA CCA TTG GTT GAT ATT AAC GGC AAA CCC ATG ATT GTT CAT      96
   Pro Gly Lys Pro Leu Val Asp Ile Asn Gly Lys Pro Met Ile Val His
                20                  25                  30
   GTT CTT GAA CGC GCG CGT GAA TCA GGT GCC GAG CGC ATC ATC GTG GCA     144
   Val Leu Glu Arg Ala Arg Glu Ser Gly Ala Glu Arg Ile Ile Val Ala
            35                  40                  45
   ACC GAT CAT GAG GAT GTT GCC CGC GCC GTT GAA GCC GCT GGC GGT GAA     192
   Thr Asp His Glu Asp Val Ala Arg Ala Val Glu Ala Ala Gly Gly Glu
        50                  55                  60
   GTA TGT ATG ACG CGC GCC GAT CAT CAG TCA GGA ACA GAA CGT CTG GCG     240
   Val Cys Met Thr Arg Ala Asp His Gln Ser Gly Thr Glu Arg Leu Ala
    65                  70                  75                  80
   GAA GTT GTC GAA AAA TGC GCA TTC AGC GAC GAC ACG GTG ATC GTT AAT     288
   Glu Val Val Glu Lys Cys Ala Phe Ser Asp Asp Thr Val Ile Val Asn
   85 90 95
   GTG CAG GGT GAT GAA CCG ATG ATC CCT GCG ACA ATC ATT CGT CAG GTT 336
   Val Gln Gly Asp Glu Pro Met Ile Pro Ala Thr Ile Ile Arg Gln Val
   100 105 110
   GCT GAT AAC CTC GCT CAG CGT CAG GTG GGT ATG GCG ACT CTG GCG GTG 384
   Ala Asp Asn Leu Ala Gln Arg Gln Val Gly Met Ala Thr Leu Ala Val
   115 120 125
   CCA ATC CAC AAT GCG GAA GAA GCG TTT AAC CCG AAT GCG GTG AAA GTG 432
   Pro Ile His Asn Ala Glu Glu Ala Phe Asn Pro Asn Ala Val Lys Val
   130 135 140
   GTT CTC GAC GCT GAA GGG TAT GCA CTG TAC TTC TCT CGC GCC ACC ATT 480
   Val Leu Asp Ala Glu Gly Tyr Ala Leu Tyr Phe Ser Arg Ala Thr Ile
   145 150 155 160
   CCT TGG GAT CGT GAT CGT TTT GCA GAA GGC CTT GAA ACC GTT GGC GAT 528
   Pro Trp Asp Arg Asp Arg Phe Ala Glu Gly Leu Glu Thr Val Gly Asp
   165 170 175
   AAC TTC CTG CGT CAT CTT GGT ATT TAT GGC TAC CGT GCA GGC TTT ATC 576
   Asn Phe Leu Arg His Leu Gly Ile Tyr Gly Tyr Arg Ala Gly Phe Ile
   180 185 190
   CGT CGT TAC GTC AAC TGG CAG CCA AGT CCG TTA GAA CAC ATC GAA ATG 624
   Arg Arg Tyr Val Asn Trp Gln Pro Ser Pro Leu Glu His Ile Glu Met
   195 200 205
   TTA GAG CAG CTT CGT GTT CTG TGG TAC GGC GAA AAA ATC CAT GTT GCT 672
   Leu Glu Gln Leu Arg Val Leu Trp Tyr Gly Glu Lys Ile His Val Ala
   210 215 220
   GTT GCT CAG GAA GTT CCT GGC ACA GGT GTG GAT ACC CCT GAA GAT CTC 720
   Val Ala Gln Glu Val Pro Gly Thr Gly Val Asp Thr Pro Glu Asp Leu
   225 230 235 240
   GAC CCG TCG ACG AAT TCC ATG GAC GCT CAC TTC CTG TCT CAG GCG CCG 768
   Asp Pro Ser Thr Asn Ser Met Asp Ala His Phe Leu Ser Gln Ala Pro
   245 250 255
   CCG CCG TCT TGG GAT CAG ATG TGG AAA TGC CTG ATC CGT CTG AAA CCG 816
   Pro Pro Ser Trp Asp Gln Met Trp Lys Cys Leu Ile Arg Leu Lys Pro
   260 265 270
   ACC CTG CAC GGC CCG ACC CCG CTG CTG TAC CGT CTG GGT GCT GTT CAG 864
   Thr Leu His Gly Pro Thr Pro Leu Leu Tyr Arg Leu Gly Ala Val Gln
   275 280 285
   AAC GAA ATC ACC CTG ACC CAC CCG GTT ACC AAA TAC ATC ATG ACC TGC 912
   Asn Glu Ile Thr Leu Thr His Pro Val Thr Lys Tyr Ile Met Thr Cys
   290 295 300
   ATG TCT GCT GAT CTA GAA GTT GTT ACC TCT ACC TGG GTT CTG GTT GGT 960
   Met Ser Ala Asp Leu Glu Val Val Thr Ser Thr Trp Val Leu Val Gly
   305 310 315 320
   GGT GTT CTG GCT GCT CTG GCT GCT TAC TGC CTG TCG ACC GGT TGC GTT 1008
   Gly Val Leu Ala Ala Leu Ala Ala Tyr Cys Leu Ser Thr Gly Cys Val
   325 330 335
   GTT ATC GTT GGT CGT GTT GTT CTG TCT GGT AAA CCG GCC ATT ATC CCG 1056
   Val Ile Val Gly Arg Val Val Leu Ser Gly Lys Pro Ala Ile Ile Pro
   340 345 350
   GAC CGT GAA GTT CTG TAC CGT GAG TTC GAC GAA ATG GAA GAA TGC TCT 1104
   Asp Arg Glu Val Leu Tyr Arg Glu Phe Asp Glu Met Glu Glu Cys Ser
   355 360 365
   CAG CAC CTG CCG TAC ATC GAA CAG GGT ATG ATG CTG GCT GAA CAG TTC 1152
   Gln His Leu Pro Tyr Ile Glu Gln Gly Met Met Leu Ala Glu Gln Phe
   370 375 380
   AAA CAG AAA GCT CTG GGT CTG CTG CAG ACC GCT TCT CGT CAG GCT GAA 1200
   Lys Gln Lys Ala Leu Gly Leu Leu Gln Thr Ala Ser Arg Gln Ala Glu
   385 390 395 400
   GTT ATC GCT CCG GCT GTT CAG ACC AAC TGG CAG AAA CTC GAG ACC TTC 1248
   Val Ile Ala Pro Ala Val Gln Thr Asn Trp Gln Lys Leu Glu Thr Phe
   405 410 415
   TGG GCT AAA CAC ATG TGG AAC TTC ATC TCT GGT ATC CAG TAC CTG GCT 1296
   Trp Ala Lys His Met Trp Asn Phe Ile Ser Gly Ile Gln Tyr Leu Ala
   420 425 430
   GGT CTG TCT ACC CTG CCG GGT AAC CCG GCT ATC GCA AGC TTG ATG GCT 1344
   Gly Leu Ser Thr Leu Pro Gly Asn Pro Ala Ile Ala Ser Leu Met Ala
   435 440 445
   TTC ACC GCT GCT GTT ACC TCT CCG CTG ACC ACC TCT CAG ACC CTG CTG 1392
   Phe Thr Ala Ala Val Thr Ser Pro Leu Thr Thr Ser Gln Thr Leu Leu
   450 455 460
   TTC AAC ATT CTG GGT GGT TGG GTT GCT GCT CAG CTG GCT GCT CCG GGT 1440
   Phe Asn Ile Leu Gly Gly Trp Val Ala Ala Gln Leu Ala Ala Pro Gly
   465 470 475 480
   GCT GCT ACC GCT TTC GTT GGT GCT GGT CTG GCT GGT GCT GCT ATC GGT 1488
   Ala Ala Thr Ala Phe Val Gly Ala Gly Leu Ala Gly Ala Ala Ile Gly
   485 490 495
   TCT GTA GGC CTG GGT AAA GTT CTG ATC GAC ATT CTG GCT GGT TAC GGT 1536
   Ser Val Gly Leu Gly Lys Val Leu Ile Asp Ile Leu Ala Gly Tyr Gly
   500 505 510
   GCT GGT GTT GCT GGA GCT CTG GTT GCT TTC AAA ATC ATG TCT GGT GAA 1584
   Ala Gly Val Ala Gly Ala Leu Val Ala Phe Lys Ile Met Ser Gly Glu
   515 520 525
   GTT CCG TCT ACC GAA GAT CTG GTT AAC CTG CTG CCG GCT ATC CTG TCT 1632
   Val Pro Ser Thr Glu Asp Leu Val Asn Leu Leu Pro Ala Ile Leu Ser
   530 535 540
   CCG GGT GCT CTG GTT GTT GGT GTT GTT TGC GCT GCT ATC CTG CGT CGT 1680
   Pro Gly Ala Leu Val Val Gly Val Val Cys Ala Ala Ile Leu Arg Arg
   545 550 555 560
   CAC GTT GGC CCG GGT GAA GGT GCT GTT CAG TGG ATG AAC CGT CTG ATC 1728
   His Val Gly Pro Gly Glu Gly Ala Val Gln Trp Met Asn Arg Leu Ile
   565 570 575
   GCT TTC GCT TCT CGT GGT AAC CAC GTT TCT CCA TGG GAT CCT CTA GAC 1776
   Ala Phe Ala Ser Arg Gly Asn His Val Ser Pro Trp Asp Pro Leu Asp
   580 585 590
   TGC AGG CAT GCT AAG 1791
   Cys Arg His Ala Lys
   595

### (2) INFORMATION FOR SEQ ID NO: 23:

(i) SEQUENCE CHARACTERISTICS:
   (A) LENGTH: 597 amino acids
   (B) TYPE: amino acid
   (D) TOPOLOGY: linear
(ii) MOLECULE TYPE: protein
(xi) SEQUENCE DESCRIPTION: SEQ ID NO: 23:
   Met Ser Phe Val Val Ile Ile Pro Ala Arg Tyr Ala Ser Thr Arg Leu
   1 5 10 15
   Pro Gly Lys Pro Leu Val Asp Ile Asn Gly Lys Pro Met Ile Val His
   20 25 30
   Val Leu Glu Arg Ala Arg Glu Ser Gly Ala Glu Arg Ile Ile Val Ala
   35 40 45
   Thr Asp His Glu Asp Val Ala Arg Ala Val Glu Ala Ala Gly Gly Glu
   50 55 60
   Val Cys Met Thr Arg Ala Asp His Gln Ser Gly Thr Glu Arg Leu Ala
   65 70 75 80
   Glu Val Val Glu Lys Cys Ala Phe Ser Asp Asp Thr Val Ile Val Asn
   85 90 95
   Val Gln Gly Asp Glu Pro Met Ile Pro Ala Thr Ile Ile Arg Gln Val
   100 105 110
   Ala Asp Asn Leu Ala Gln Arg Gln Val Gly Met Ala Thr Leu Ala Val
   115 120 125
   Pro Ile His Asn Ala Glu Glu Ala Phe Asn Pro Asn Ala Val Lys Val
   130 135 140
   Val Leu Asp Ala Glu Gly Tyr Ala Leu Tyr Phe Ser Arg Ala Thr Ile
   145 150 155 160
   Pro Trp Asp Arg Asp Arg Phe Ala Glu Gly Leu Glu Thr Val Gly Asp
   165 170 175
   Asn Phe Leu Arg His Leu Gly Ile Tyr Gly Tyr Arg Ala Gly Phe Ile
   180 185 190
   Arg Arg Tyr Val Asn Trp Gln Pro Ser Pro Leu Glu His Ile Glu Met
   195 200 205
   Leu Glu Gln Leu Arg Val Leu Trp Tyr Gly Glu Lys Ile His Val Ala
   210 215 220
   Val Ala Gln Glu Val Pro Gly Thr Gly Val Asp Thr Pro Glu Asp Leu
   225 230 235 240
   Asp Pro Ser Thr Asn Ser Met Asp Ala His Phe Leu Ser Gln Ala Pro
   245 250 255
   Pro Pro Ser Trp Asp Gln Met Trp Lys Cys Leu Ile Arg Leu Lys Pro
   260 265 270
   Thr Leu His Gly Pro Thr Pro Leu Leu Tyr Arg Leu Gly Ala Val Gln
   275 280 285
   Asn Glu Ile Thr Leu Thr His Pro Val Thr Lys Tyr Ile Met Thr Cys
   290 295 300
   Met Ser Ala Asp Leu Glu Val Val Thr Ser Thr Trp Val Leu Val Gly
   305 310 315 320
   Gly Val Leu Ala Ala Leu Ala Ala Tyr Cys Leu Ser Thr Gly Cys Val
   325 330 335
   Val Ile Val Gly Arg Val Val Leu Ser Gly Lys Pro Ala Ile Ile Pro
   340 345 350
   Asp Arg Glu Val Leu Tyr Arg Glu Phe Asp Glu Met Glu Glu Cys Ser
   355 360 365
   Gln His Leu Pro Tyr Ile Glu Gln Gly Met Met Leu Ala Glu Gln Phe
   370 375 380
   Lys Gln Lys Ala Leu Gly Leu Leu Gln Thr Ala Ser Arg Gln Ala Glu
   385 390 395 400
   Val Ile Ala Pro Ala Val Gln Thr Asn Trp Gln Lys Leu Glu Thr Phe
   405 410 415
   Trp Ala Lys His Met Trp Asn Phe Ile Ser Gly Ile Gln Tyr Leu Ala
   420 425 430
   Gly Leu Ser Thr Leu Pro Gly Asn Pro Ala Ile Ala Ser Leu Met Ala
   435 440 445
   Phe Thr Ala Ala Val Thr Ser Pro Leu Thr Thr Ser Gln Thr Leu Leu
   450 455 460
   Phe Asn Ile Leu Gly Gly Trp Val Ala Ala Gln Leu Ala Ala Pro Gly
   465 470 475 480
   Ala Ala Thr Ala Phe Val Gly Ala Gly Leu Ala Gly Ala Ala Ile Gly
   485 490 495
   Ser Val Gly Leu Gly Lys Val Leu Ile Asp Ile Leu Ala Gly Tyr Gly
   500 505 510
   Ala Gly Val Ala Gly Ala Leu Val Ala Phe Lys Ile Met Ser Gly Glu
   515 520 525
   Val Pro Ser Thr Glu Asp Leu Val Asn Leu Leu Pro Ala Ile Leu Ser
   530 535 540
   Pro Gly Ala Leu Val Val Gly Val Val Cys Ala Ala Ile Leu Arg Arg
   545 550 555 560
   His Val Gly Pro Gly Glu Gly Ala Val Gln Trp Met Asn Arg Leu Ile
   565 570 575
   Ala Phe Ala Ser Arg Gly Asn His Val Ser Pro Trp Asp Pro Leu Asp
   580 585 590
   Cys Arg His Ala Lys
   595

### (2) INFORMATION FOR SEQ ID NO: 24:

(i) SEQUENCE CHARACTERISTICS:
   (A) LENGTH: 1797 base pairs
   (B) TYPE: nucleic acid
   (C) STRANDEDNESS: single
   (D) TOPOLOGY: circular
(ii) MOLECULE TYPE: DNA (genomic)
(ix) FEATURE:
   (A) NAME/KEY: CDS
   (B) LOCATION: 1..1797
(xi) SEQUENCE DESCRIPTION: SEQ ID NO: 24:
   ATG AGT TTT GTG GTC ATT ATT CCC GCG CGC TAC GCG TCG ACG CGT CTG 48
   Met Ser Phe Val Val Ile Ile Pro Ala Arg Tyr Ala Ser Thr Arg Leu
   1 5 10 15
   CCC GGT AAA CCA TTG GTT GAT ATT AAC GGC AAA CCC ATG ATT GTT CAT 96
   Pro Gly Lys Pro Leu Val Asp Ile Asn Gly Lys Pro Met Ile Val His
   20 25 30
   GTT CTT GAA CGC GCG CGT GAA TCA GGT GCC GAG CGC ATC ATC GTG GCA 144
   Val Leu Glu Arg Ala Arg Glu Ser Gly Ala Glu Arg Ile Ile Val Ala
   35 40 45
   ACC GAT CAT GAG GAT GTT GCC CGC GCC GTT GAA GCC GCT GGC GGT GAA 192
   Thr Asp His Glu Asp Val Ala Arg Ala Val Glu Ala Ala Gly Gly Glu
   50 55 60
   GTA TGT ATG ACG CGC GCC GAT CAT CAG TCA GGA ACA GAA CGT CTG GCG 240
   Val Cys Met Thr Arg Ala Asp His Gln Ser Gly Thr Glu Arg Leu Ala
   65 70 75 80
   GAA GTT GTC GAA AAA TGC GCA TTC AGC GAC GAC ACG GTG ATC GTT AAT 288
   Glu Val Val Glu Lys Cys Ala Phe Ser Asp Asp Thr Val Ile Val Asn
   85 90 95
   GTG CAG GGT GAT GAA CCG ATG ATC CCT GCG ACA ATC ATT CGT CAG GTT 336
   Val Gln Gly Asp Glu Pro Met Ile Pro Ala Thr Ile Ile Arg Gln Val
   100 105 110
   GCT GAT AAC CTC GCT CAG CGT CAG GTG GGT ATG GCG ACT CTG GCG GTG 384
   Ala Asp Asn Leu Ala Gln Arg Gln Val Gly Met Ala Thr Leu Ala Val
   115 120 125
   CCA ATC CAC AAT GCG GAA GAA GCG TTT AAC CCG AAT GCG GTG AAA GTG 432
   Pro Ile His Asn Ala Glu Glu Ala Phe Asn Pro Asn Ala Val Lys Val
   130 135 140
   GTT CTC GAC GCT GAA GGG TAT GCA CTG TAC TTC TCT CGC GCC ACC ATT 480
   Val Leu Asp Ala Glu Gly Tyr Ala Leu Tyr Phe Ser Arg Ala Thr Ile
   145 150 155 160
   CCT TGG GAT CGT GAT CGT TTT GCA GAA GGC CTT GAA ACC GTT GGC GAT 528
   Pro Trp Asp Arg Asp Arg Phe Ala Glu Gly Leu Glu Thr Val Gly Asp
   165 170 175
   AAC TTC CTG CGT CAT CTT GGT ATT TAT GGC TAC CGT GCA GGC TTT ATC 576
   Asn Phe Leu Arg His Leu Gly Ile Tyr Gly Tyr Arg Ala Gly Phe Ile
   180 185 190
   CGT CGT TAC GTC AAC TGG CAG CCA AGT CCG TTA GAA CAC ATC GAA ATG 624
   Arg Arg Tyr Val Asn Trp Gln Pro Ser Pro Leu Glu His Ile Glu Met
   195 200 205
   TTA GAG CAG CTT CGT GTT CTG TGG TAC GGC GAA AAA ATC CAT GTT GCT 672
   Leu Glu Gln Leu Arg Val Leu Trp Tyr Gly Glu Lys Ile His Val Ala
   210 215 220
   GTT GCT CAG GAA GTT CCT GGC ACA GGT GTG GAT ACC CCT GAA GAT CTC 720
   Val Ala Gln Glu Val Pro Gly Thr Gly Val Asp Thr Pro Glu Asp Leu
   225 230 235 240
   GAC CCG TCG ACG AAT TCC ATG GAC GCT CAC TTC CTG TCT CAG ACC AAA 768
   Asp Pro Ser Thr Asn Ser Met Asp Ala His Phe Leu Ser Gln Thr Lys
   245 250 255
   CAG TCT GGT GAA AAC CTT CCG TAC CTG GTT GCT TAC CAG GCT ACC GTT 816
   Gln Ser Gly Glu Asn Leu Pro Tyr Leu Val Ala Tyr Gln Ala Thr Val
   260 265 270
   TGC GCT CGT GCT CAG GCC CCG ACC CCG CTG CTG TAC CGT CTG GGT GCT 864
   Cys Ala Arg Ala Gln Ala Pro Thr Pro Leu Leu Tyr Arg Leu Gly Ala
   275 280 285
   GTT CAG AAC GAA ATC ACC CTG ACC CAC CCG GTT ACC AAA TAC ATC ATG 912
   Val Gln Asn Glu Ile Thr Leu Thr His Pro Val Thr Lys Tyr Ile Met
   290 295 300
   ACC TGC ATG TCT GCT GAT CTA GAA GTT GTT ACC TCT ACC TGG GTT CTG 960
   Thr Cys Met Ser Ala Asp Leu Glu Val Val Thr Ser Thr Trp Val Leu
   305 310 315 320
   GTT GGT GGT GTT CTG GCT GCT CTG GCT GCT TAC TGC CTG TCG ACC GGT 1008
   Val Gly Gly Val Leu Ala Ala Leu Ala Ala Tyr Cys Leu Ser Thr Gly
   325 330 335
   TGC GTT GTT ATC GTT GGT CGT GTT GTT CTG TCT GGT AAA CCG GCC ATT 1056
   Cys Val Val Ile Val Gly Arg Val Val Leu Ser Gly Lys Pro Ala Ile
   340 345 350
   ATC CCG GAC CGT GAA GTT CTG TAC CGT GAG TTC GAC GAA ATG GAA GAA 1104
   Ile Pro Asp Arg Glu Val Leu Tyr Arg Glu Phe Asp Glu Met Glu Glu
   355 360 365
   TGC TCT CAG CAC CTG CCG TAC ATC GAA CAG GGT ATG ATG CTG GCT GAA 1152
   Cys Ser Gln His Leu Pro Tyr Ile Glu Gln Gly Met Met Leu Ala Glu
   370 375 380
   CAG TTC AAA CAG AAA GCT CTG GGT CTG CTG CAG ACC GCT TCT CGT CAG 1200
   Gln Phe Lys Gln Lys Ala Leu Gly Leu Leu Gln Thr Ala Ser Arg Gln
   385 390 395 400
   GCT GAA GTT ATC GCT CCG GCT GTT CAG ACC AAC TGG CAG AAA CTC GAG 1248
   Ala Glu Val Ile Ala Pro Ala Val Gln Thr Asn Trp Gln Lys Leu Glu
   405 410 415
   ACC TTC TGG GCT AAA CAC ATG TGG AAC TTC ATC TCT GGT ATC CAG TAC 1296
   Thr Phe Trp Ala Lys His Met Trp Asn Phe Ile Ser Gly Ile Gln Tyr
   420 425 430
   CTG GCT GGT CTG TCT ACC CTG CCG GGT AAC CCG GCT ATC GCA AGC TTG 1344
   Leu Ala Gly Leu Ser Thr Leu Pro Gly Asn Pro Ala Ile Ala Ser Leu
   435 440 445
   ATG GCT TTC ACC GCT GCT GTT ACC TCT CCG CTG ACC ACC TCT CAG ACC 1392
   Met Ala Phe Thr Ala Ala Val Thr Ser Pro Leu Thr Thr Ser Gln Thr
   450 455 460
   CTG CTG TTC AAC ATT CTG GGT GGT TGG GTT GCT GCT CAG CTG GCT GCT 1440
   Leu Leu Phe Asn Ile Leu Gly Gly Trp Val Ala Ala Gln Leu Ala Ala
   465 470 475 480
   CCG GGT GCT GCT ACC GCT TTC GTT GGT GCT GGT CTG GCT GGT GCT GCT 1488
   Pro Gly Ala Ala Thr Ala Phe Val Gly Ala Gly Leu Ala Gly Ala Ala
   485 490 495
   ATC GGT TCT GTA GGC CTG GGT AAA GTT CTG ATC GAC ATT CTG GCT GGT 1536
   Ile Gly Ser Val Gly Leu Gly Lys Val Leu Ile Asp Ile Leu Ala Gly
   500 505 510
   TAC GGT GCT GGT GTT GCT GGA GCT CTG GTT GCT TTC AAA ATC ATG TCT 1584
   Tyr Gly Ala Gly Val Ala Gly Ala Leu Val Ala Phe Lys Ile Met Ser
   515 520 525
   GGT GAA GTT CCG TCT ACC GAA GAT CTG GTT AAC CTG CTG CCG GCT ATC 1632
   Gly Glu Val Pro Ser Thr Glu Asp Leu Val Asn Leu Leu Pro Ala Ile
   530 535 540
   CTG TCT CCG GGT GCT CTG GTT GTT GGT GTT GTT TGC GCT GCT ATC CTG 1680
   Leu Ser Pro Gly Ala Leu Val Val Gly Val Val Cys Ala Ala Ile Leu
   545 550 555 560
   CGT CGT CAC GTT GGC CCG GGT GAA GGT GCT GTT CAG TGG ATG AAC CGT 1728
   Arg Arg His Val Gly Pro Gly Glu Gly Ala Val Gln Trp Met Asn Arg
   565 570 575
   CTG ATC GCT TTC GCT TCT CGT GGT AAC CAC GTT TCT CCA TGG GAT CCT 1776
   Leu Ile Ala Phe Ala Ser Arg Gly Asn His Val Ser Pro Trp Asp Pro
   580 585 590
   CTA GAC TGC AGG CAT GCT AAG 1797
   Leu Asp Cys Arg His Ala Lys
   595

### (2) INFORMATION FOR SEQ ID NO: 25:

(i) SEQUENCE CHARACTERISTICS:
   (A) LENGTH: 599 amino acids
   (B) TYPE: amino acid
   (D) TOPOLOGY: linear
(ii) MOLECULE TYPE: protein
(xi) SEQUENCE DESCRIPTION: SEQ ID NO: 25:
   Met Ser Phe Val Val Ile Ile Pro Ala Arg Tyr Ala Ser Thr Arg Leu
   1 5 10 15
   Pro Gly Lys Pro Leu Val Asp Ile Asn Gly Lys Pro Met Ile Val His
   20 25 30
   Val Leu Glu Arg Ala Arg Glu Ser Gly Ala Glu Arg Ile Ile Val Ala
   35 40 45
   Thr Asp His Glu Asp Val Ala Arg Ala Val Glu Ala Ala Gly Gly Glu
   50 55 60
   Val Cys Met Thr Arg Ala Asp His Gln Ser Gly Thr Glu Arg Leu Ala
   65 70 75 80
   Glu Val Val Glu Lys Cys Ala Phe Ser Asp Asp Thr Val Ile Val Asn
   85 90 95
   Val Gln Gly Asp Glu Pro Met Ile Pro Ala Thr Ile Ile Arg Gln Val
   100 105 110
   Ala Asp Asn Leu Ala Gln Arg Gln Val Gly Met Ala Thr Leu Ala Val
   115 120 125
   Pro Ile His Asn Ala Glu Glu Ala Phe Asn Pro Asn Ala Val Lys Val
   130 135 140
   Val Leu Asp Ala Glu Gly Tyr Ala Leu Tyr Phe Ser Arg Ala Thr Ile
   145 150 155 160
   Pro Trp Asp Arg Asp Arg Phe Ala Glu Gly Leu Glu Thr Val Gly Asp
   165 170 175
   Asn Phe Leu Arg His Leu Gly Ile Tyr Gly Tyr Arg Ala Gly Phe Ile
   180 185 190
   Arg Arg Tyr Val Asn Trp Gln Pro Ser Pro Leu Glu His Ile Glu Met
   195 200 205
   Leu Glu Gln Leu Arg Val Leu Trp Tyr Gly Glu Lys Ile His Val Ala
   210 215 220
   Val Ala Gln Glu Val Pro Gly Thr Gly Val Asp Thr Pro Glu Asp Leu
   225 230 235 240
   Asp Pro Ser Thr Asn Ser Met Asp Ala His Phe Leu Ser Gln Thr Lys
   245 250 255
   Gln Ser Gly Glu Asn Leu Pro Tyr Leu Val Ala Tyr Gln Ala Thr Val
   260 265 270
   Cys Ala Arg Ala Gln Ala Pro Thr Pro Leu Leu Tyr Arg Leu Gly Ala
   275 280 285
   Val Gln Asn Glu Ile Thr Leu Thr His Pro Val Thr Lys Tyr Ile Met
   290 295 300
   Thr Cys Met Ser Ala Asp Leu Glu Val Val Thr Ser Thr Trp Val Leu
   305                 310                 315                 320
   Val Gly Gly Val Leu Ala Ala Leu Ala Ala Tyr Cys Leu Ser Thr Gly
               325                 330                 335
   Cys Val Val Ile Val Gly Arg Val Val Leu Ser Gly Lys Pro Ala Ile
           340                 345                 350
   Ile Pro Asp Arg Glu Val Leu Tyr Arg Glu Phe Asp Glu Met Glu Glu
       355                 360                 365
   Cys Ser Gln His Leu Pro Tyr Ile Glu Gln Gly Met Met Leu Ala Glu
       370                 375                 380
   Gln Phe Lys Gln Lys Ala Leu Gly Leu Leu Gln Thr Ala Ser Arg Gln
   385                 390                 395                 400
   Ala Glu Val Ile Ala Pro Ala Val Gln Thr Asn Trp Gln Lys Leu Glu
               405                 410                 415
   Thr Phe Trp Ala Lys His Met Trp Asn Phe Ile Ser Gly Ile Gln Tyr
           420                 425                 430
   Leu Ala Gly Leu Ser Thr Leu Pro Gly Asn Pro Ala Ile Ala Ser Leu
       435                 440                 445
   Met Ala Phe Thr Ala Ala Val Thr Ser Pro Leu Thr Thr Ser Gln Thr
       450                 455                 460
   Leu Leu Phe Asn Ile Leu Gly Gly Trp Val Ala Ala Gln Leu Ala Ala
   465                 470                 475                 480
   Pro Gly Ala Ala Thr Ala Phe Val Gly Ala Gly Leu Ala Gly Ala Ala
               485                 490                 495
   Ile Gly Ser Val Gly Leu Gly Lys Val Leu Ile Asp Ile Leu Ala Gly
           500                 505                 510
   Tyr Gly Ala Gly Val Ala Gly Ala Leu Val Ala Phe Lys Ile Met Ser
       515                 520                 525
   Gly Glu Val Pro Ser Thr Glu Asp Leu Val Asn Leu Leu Pro Ala Ile
       530                 535                 540
   Leu Ser Pro Gly Ala Leu Val Val Gly Val Val Cys Ala Ala Ile Leu
   545                 550                 555                 560
   Arg Arg His Val Gly Pro Gly Glu Gly Ala Val Gln Trp Met Asn Arg
               565                 570                 575
   Leu Ile Ala Phe Ala Ser Arg Gly Asn His Val Ser Pro Trp Asp Pro
           580                 585                 590
   Leu Asp Cys Arg His Ala Lys